# DEMANDE DE BREVET EUROPEEN

(11) **EP 3 037 537 A1**
(43) Date de publication de la demande: **29.06.2016**
(21) Numéro de dépôt: 16153703.0
(22) Date de dépôt: 08.11.2002
(51) Int. Cl.: C12N 15/113, A61K 31/713, C12Q 1/68, A61P 35/00, A61P 31/00

(54) **OLIGONUCLEOTIDES INHIBITEURS ET LEUR UTILISATION POUR REPRIMER SPECIFIQUEMENT UN GENE**

(30) Priorité: 09.11.2001 FR 0114549; 10.04.2002 FR 0204474
(62) Demande divisionnaire de: 08001984.7
(71) Demandeur: Centre National de la Recherche Scientifique, 75794 Paris Cedex 16 (FR)
(72) Inventeur: HAREL-BELLAN, Annick, 75005 Paris (FR); AIT-SI-ALI, Slimane, 94800 Villejuif (FR); CABON-GEORGET, Florence, 94400 Vitry sur Seine (FR); CHAUCHEREAU, Anne, 92260 Fontenay-aux-Roses (FR); DAUTRY, François, 75014 Paris (FR)
(74) Mandataire: Grosset-Fournier, Chantal Catherine

(57) **Abrégé**

L'invention a pour objet un oligonucléotide double brin caractérisé en ce qu'il est constitué de deux séquences oligonucléotidiques complémentaires formant un hybride comprenant chacune à l'une de leurs extrémités 3' ou 5' un à cinq nucléotides non appariés formant des bouts simples brins débordant de l'hybride, l'une desdites séquences oligonucléotidiques étant substantiellement complémentaire d'une séquence cible appartenant à une molécule d'ADN ou d'ARN à réprimer spécifiquement. L'invention se rapporte aussi à l'utilisation desdits oligonucléotides dans des compositions pharmaceutiques notamment pour le traitement des cancers.

LISTAGE DE SEQUENCES

<110>  CENTRE NATIONAL DE LA RECERCHE SCIENTIFIQUE

<120>  Oligonucleotides inhibiteurs et leur utilisation pour réprimer
spécifiquement un gène

<130>  24240PCT Nov 2002

<140>  PCT/FR02/03843

<141>  2002-11-08

<150>  FR01/14549

<151>  2001-11-09

<150>  FR02/04474

<151>  2002-04-10

<160>  77

<170>  PatentIn version 3.1


<210>  1
<211>  1182
<212>  DNA
<213>  Homo sapiens
<220>
<221>  misc_feature
<222>  (1)..(1182)
<223>  Séquence du gène p53


<400>  1
atggaggagc cgcagtcaga tcctagcgtc gagccccctc tgagtcagga aacattttca      60
gacctatgga aactacttcc tgaaaacaac gttctgtccc ccttgccgtc ccaagcaatg     120
gatgatttga tgctgtcccc ggacgatatt gaacaatggt tcactgaaga cccaggtcca     180
gatgaagctc ccagaatgcc agaggctgct ccccccgtgg cccctgcacc agcagctcct     240
acaccggcgg cccctgcacc agccccctcc tggcccctgt catcttctgt cccttcccag     300
aaaacctacc agggcagcta cggtttccgt ctgggcttct tgcattctgg gacagccaag     360
tctgtgactt gcacgtactc ccctgccctc aacaagatgt tttgccaact ggccaagacc     420
tgccctgtgc agctgtgggt tgattccaca cccccgcccg gcacccgcgt ccgcgccatg     480
gccatctaca agcagtcaca gcacatgacg gaggttgtga ggcgctgccc ccaccatgag     540
cgctgctcag atagcgatgg tctggcccct cctcagcatc ttatccgagt ggaaggaaat     600
ttgcgtgtgg agtatttgga tgacagaaac acttttcgac atagtgtggt ggtgccctat     660
gagccgcctg aggttggctc tgactgtacc accatccact acaactacat gtgtaacagt     720
tcctgcatgg gcggcatgaa ccggaggccc atcctcacca tcatcacact ggaagactcc     780
agtggtaatc tactgggacg gaacagcttt gaggtgcgtg tttgtgcctg tcctgggaga     840
gaccggcgca cagaggaaga gaatctccgc aagaaagggg agcctcacca cgagctgccc     900
ccagggagca ctaagcgagc actgcccaac aacaccagct cctctcccca gccaaagaag     960
aaaccactgg atggagaata tttcaccctt cagatccgtg ggcgtgagcg cttcgagatg    1020
ttccgagagc tgaatgaggc cttggaactc aaggatgccc aggctgggaa ggagccaggg    1080
gggagcaggg ctcactccag ccacctgaag tccaaaaagg gtcagtctac ctcccgccat    1140
aaaaaactca tgttcaagac agaagggcct gactcagact ga                       1182

<210>  2
<211>  7904
<212>  DNA
<213>  Homo sapiens
<220>

<221>  misc_feature
<222>  (1)..(7904)
<223>  Variant HPV16

<400>  2
actacaataa ttcatgtata aaactaaggg cgtaaccgaa atcggttgaa ccgaaaccgg      60
ttagtataaa agcagacatt ttatgcacca aaagagaact gcaatgtttc aggacccaca     120
ggagcgaccc agaaagttac cacagttatg cacagagctg caaacaacta tacatgatat     180
aatattagaa tgtgtgtact gcaagcaaca gttactgcga cgtgaggtat atgactttgc     240
ttttcgggat ttatgcatag tatatagaga tgggaatcca tatgctgtat gtgataaatg     300
tttaaagttt tattctaaaa ttagtgagta tagacattat tgttatagtt tgtatggaac     360
aacattagaa cagcaataca acaaaccgtt gtgtgatttg ttaattaggt gtattaactg     420
tcaaaagcca ctgtgtcctg aagaaaagca aagacatctg gacaaaaagc aaagattcca     480
taatataagg ggtcggtgga ccggtcgatg tatgtcttgt tgcagatcat caagaacacg     540
tagagaaacc cagctgtaat catgcatgga gatacaccta cattgcatga atatatgtta     600
gatttgcaac cagagacaac tgatctctac tgttatgagc aattaaatga cagctcagag     660
gaggaggatg aaatagatgg tccagctgga caagcagaac cggacagagc ccattacaat     720
attgtaacct tttgttgcaa gtgtgactct acgcttcggt tgtgcgtaca aagcacacac     780
gtagacattc gtactttgga agacctgtta atgggcacac taggaattgt gtgccccatc     840
tgttctcaga aaccataatc taccatggct gatcctgcag gtaccaatgg ggaagagggt     900
acgggatgta atggatggtt ttatgtagag gctgtagtgg aaaaaaaaac aggggatgct     960
atatcagatg acgagaacga aaatgacagt gatacaggtg aagatttggt agattttata    1020
gtaaatgata atgattattt aacacaggca gaaacagaga cagcacatgc gttgtttact    1080
gcacaggaag caaaacaaca tagagatgca gtacaggttc taaaacgaaa gtatttggta    1140
gtccacttag tgatattagt ggatgtgtag acaataatat tagtcctaga ttaaaagcta    1200
tatgtataga aaaacaaagt agagctgcaa aaaggagatt atttgaaagc gaagacagcg    1260
ggtatggcaa tactgaagtg gaaactcagc agatgttaca ggtagaaggg cgccatgaga    1320
ctgaaacacc atgtagtcag tatagtggtg gaagtggggg tggttgcagt cagtacagta    1380
gtggaagtgg gggagagggt gttagtgaaa gacacactat atgccaaaca ccacttacaa    1440
atattttaaa tgtactaaaa actagtaatg caaaggcagc aatgttagca aaatttaaag    1500
agttatacgg ggtgagtttt tcagaattag taagaccatt taaaagtaat aaatcaacgt    1560
gttgcgattg gtgtattgct gcatttggac ttacacccag tatagctgac agtataaaaa    1620
cactattaca acaatattgt ttatatttac acattcaaag tttagcatgt tcatggggaa    1680
tggttgtgtt actattagta agatataaat gtggaaaaaa tagagaaaca attgaaaaat    1740
tgctgtctaa actattatgt gtgtctccaa tgtgtatgat gatagagcct ccaaaattgc    1800
gtagtacagc agcagcatta tattggtata aaacaggtat atcaaatatt agtgaagtgt    1860
atggagacac gccagaatgg atacaaagac aaacagtatt acaacatagt tttaatgatt    1920
gtacatttga attatcacag atggtacaat gggcctacga taatgacata gtagacgata    1980
gtgaaattgc atataaatat gcacaattgg cagacactaa tagtaatgca agtgcctttc    2040
taaaaagtaa ttcacaggca aaaattgtaa aggattgtgc aacaatgtgt agacattata    2100
aacgagcaga aaaaaaacaa atgagtatga gtcaatggat aaaatataga tgtgataggg    2160
tagatgatgg aggtgattgg aagcaaattg ttatgttttt aaggtatcaa ggtgtagagt    2220
ttatgtcatt tttaactgca ttaaaaagat ttttgcaagg catacctaaa aaaaattgca    2280
tattactata tggtgcagct aacacaggta aatcattatt tggtatgagt ttaatgaaat    2340
ttctgcaagg gtctgtaata tgttttgtaa attctaaaag ccatttttgg ttacaaccat    2400
tagcagatgc caaaataggt atgttagatg atgctacagt gccctgttgg aactacatag    2460
atgacaattt aagaaatgca ttggatggaa atttagtttc tatggatgta aagcatagac    2520
cattggtaca actaaaatgc cctccattat taattacatc taacattaat gctggtacag    2580
attctaggtg gccttattta cataatagat tggtggtgtt tacatttcct aatgagtttc    2640
catttgacga aaacggaaat ccagtgtatg agcttaatga taagaactgg aaatcctttt    2700
tctcaaggac gtggtccaga ttaagtttgc acgaggacga ggacaaggaa aacgatggag    2760
actctttgcc aacgtttaaa tgtgtgtcag gacaaaatac taacacatta tgaaaatgat    2820
agtacagacc tacgtgacca tatagactat tggaaacaca tgcgcctaga atgtgctatt    2880
tattacaagg ccagagaaat gggatttaaa catattaacc accaagtggt gccaacactg    2940
gctgtatcaa agaataaagc attacaagca attgaactgc aactaacgtt agaaacaata    3000
tataactcac aatatagtaa tgaaaagtgg acattacaag acgttagcct tgaagtgtat    3060
ttaactgcac caacaggatg tataaaaaaa catggatata cagtggaagt gcagtttgat    3120
ggagacatat gcaatacaat gcattataca aactggacac atatatatat ttgtgaagaa    3180
gcatcagtaa ctgtggtaga gggtcaagtt gactattatg gtttatatta tgttcatgaa    3240
ggaatacgaa catattttgt gcagtttaaa gatgatgcag aaaaatatag taaaaataaa    3300
gtatgggaag ttcatgcggg tggtcaggta atattatgtc ctacatctgt gtttagcagc    3360
aacgaagtat cctctcctga aattattagg cagcacttgg ccaaccaccc cgccgcgacc    3420
cataccaaag ccgtcgcctt gggcaccgaa gaaacacaga cgactatcca gcgaccaaga    3480

tcagagccag acaccggaaa cccctgccac accactaagt tgttgcacag agactcagtg 3540
gacagtgctc caatcctcac tgcatttaac agctcacaca aaggacggat taactgtaat 3600
agtaacacta cacccatagt acatttaaaa ggtgatgcta atactttaaa atgtttaaga 3660
tatagattta aaaagcattg tacattgtat actgcagtgt cgtctacatg gcattggaca 3720
ggacataatg taaaacataa aagtgcaatt gttacactta catatgatag tgaatggcaa 3780
cgtgaccaat tttgtctcaa agttaaaata ccaaaaacta ttacagtgtc tactggattt 3840
atgtctatat gacaaatctt gatactgcat ccacaacatt actggcgtgc tttttgcttt 3900
gctttgtgtg cttttgtgtg tctgcctatt aatacgtccg ctgcttttgt ctgtgtctac 3960
atacacatca ttaataatat tggtattact attgtggata acagcagcct ctgcgtttag 4020
gtgttttatt gtatatatta tatttgttta tataccatta tttttaatac atacacatgc 4080
acgcttttta attacataat gtatatgtac ataatgtaat tgttacatat aattgttgta 4140
taccataact tactattttt tcttttttat tttcatatat aatttttttt tttgtttgtt 4200
tgtttgtttt ttaataaact gttattactt aacaatgcga cacaaacgtt ctgcaaaacg 4260
cacaaaacgt gcatcggcta cccaacttta taaaacatgc aaacaggcag gtacatgtcc 4320
acctgacatt atacctaagg ttgaaggcaa aactattgct gaacaaatat tacaatatgg 4380
aagtatgggt gtattttttg gtgggttagg aattggaaca gggtcgggta caggcggacg 4440
cactgggtat attccattgg gaacaaggcc tcccacagct acagatacac ttgctcctgt 4500
aagaccccct ttaacagtag atcctgtggg cccttctgat ccttctatag tttctttagt 4560
ggaagaaact agttttattg atgctggtgc accaacatct gtaccttcca ttcccccaga 4620
tgtatcagga tttagtatta ctacttcaac tgataccaca cctgctatat tagatattaa 4680
taatactgtt actactgtta ctacacataa taatcccact ttcactgacc catctgtatt 4740
gcagcctcca acacctgcag aaactggagg gcattttaca ctttcatcat ccactattag 4800
tacacataat tatgaagaaa ttcctatgga tacatttatt gttagcacaa accctaacac 4860
agtaactagt agcacaccca taccagggtc tcgcccagtg gcacgcctag gattatatag 4920
tcgcacaaca caacaggtta aagttgtaga ccctgctttt gtaaccactc ccactaaact 4980
tattacatat gataatcctg catatgaagg tatagatgtg gataatacat tatatttttc 5040
tagtaatgat aatagtatta atatagctcc agatcctgac tttttggata tagttgcttt 5100
acataggcca gcattaacct ctaggcgtac tggcattagg tacagtagaa ttggtaataa 5160
acaaacacta cgtactcgta gtggaaaatc tataggtgct aaggtacatt attattatga 5220
tttaagtact attgatcctg cagaagaaat agaattacaa actataacac cttctacata 5280
tactaccact tcacatgcag cctcacctac ttctattaat aatggattat atgatattta 5340
tgcagatgac tttattacag atacttctac aaccccggta ccatctgtac cctctacatc 5400
tttatcaggt tatattcctg caaatacaac aattcctttt ggtggtgcat acaatattcc 5460
tttagtatca ggtcctgata tacccattaa tataactgac caagctcctt cattaattcc 5520
tatagttcca gggtctccac aatatacaat tattgctgat gcaggtgact tttatttaca 5580
tcctagttat tacatgttac gaaaacgacg taaacgttta ccatattttt tttcagatgt 5640
ctctttggct gcctagtgag gccactgtct acttgcctcc tgtcccagta tctaaggttg 5700
taagcacgga tgaatatgtt gcacgcacaa acatatatta tcatgcagga acatccagac 5760
tacttgcagt tggacatccc tattttccta ttaaaaaacc taacaataac aaaatattag 5820
ttcctaaagt atcaggatta caatacaggg tatttagaat acatttacct gaccccaata 5880
agtttggttt tcctgacacc tcattttata atccagatac acagcggctg gtttgggcct 5940
gtgtaggtgt tgaggtaggt cgtggtcagc cattaggtgt gggcattagt ggccatcctt 6000
tattaaataa attggatgac acagaaaatg ctagtgctta tgcagcaaat gcaggtgtgg 6060
ataatagaga atgtatatct atggattaca aacaaacaca attgtgttta attggttgca 6120
aaccacctat aggggaacac tggggcaaag gatccccatg taccaatgtt gcagtaaatc 6180
caggtgattg tccaccatta gagttaataa acacagttat tcaggatggt gatatggttc 6240
atactggctt tggtgctatg gactttacta cattacaggc taacaaaagt gaagttccac 6300
tggatatttg tacatctatt tgcaaatatc cagattatat taaaatggtg tcagaaccat 6360
atggcgacag cttatttttt tatttacgaa gggaacaaat gtttgttaga catttattta 6420
atagggctgg tactgttggt gaaaatgtac cagacgattt atacattaaa ggctctgggt 6480
ctactgcaaa tttagccagt tcaaattatt ttcctacacc tagtggttct atggttacct 6540
ctgatgccca aatattcaat aaaccttatt ggttacaacg agcacagggc cacaataatg 6600
gcatttgttg gggtaaccaa ctatttgtta ctgttgttga tactacacgc agtacaaata 6660
tgtcattatg tgctgccata tctacttcag aaactacata taaaaatact aactttaagg 6720
agtacctacg acatggggag gaatatgatt tacagtttat ttttcaactg tgcaaaataa 6780
ccttaactgc agacgttatg acatacatac attctatgaa ttccactatt ttggaggact 6840
ggaattttgg tctacaacct cccccaggag gcacactaga agatacttat aggtttgtaa 6900
cccaggcaat tgcttgtcaa aaacatacac ctccagcacc taaagaagat gatcccctta 6960
aaaaatacac tttttgggaa gtaaatttaa aggaaaagtt ttctgcagac ctagatcagt 7020
ttcctttagg acgcaaattt ttactacaag caggattgaa ggccaaacca aaatttacat 7080
taggaaaacg aaaagctaca cccaccacct catctacctc tacaactgct aaacgcaaaa 7140
aacgtaagct gtaagtattg tatgtatgtt gaattagtgt tgtttgttgt gtatatgttt 7200
gtatgtgctt gtatgtgctt gtaaatatta agttgtatgt gtgtttgtat gtatggtata 7260

ataaacacgt gtgtatgtgt ttttaaatgc ttgtgtaact attgtgtcat gcaacataaa 7320
taaacttatt gtttcaacac ctactaattg tgttgtggtt attcattgta tataaactat 7380
atttgctaca tcctgttttt gttttatata tactatattt tgtagcgcca ggcccatttt 7440
gtagcttcaa ccgaattcgg ttgcatgctt tttggcacaa aatgtgtttt tttaaatagt 7500
tctatgtcag caactatggt ttaaacttgt acgtttcctg cttgccatgc gtgccaaatc 7560
cctgttttcc tgacctgcac tgcttgccaa ccattccatt gttttttaca ctgcactatg 7620
tgcaactact gaatcactat gtacattgtg tcatataaaa taaatcacta tgcgccaacg 7680
ccttacatac cgctgttagg cacatatttt tggcttgttt taactaacct aattgcatat 7740
ttggcataag gtttaaactt ctaaggccaa ctaaatgtca ccctagttca tacatgaact 7800
gtgtaaaggt tagtcataca ttgttcattt gtaaaactgc acatgggtgt gtgcaaaccg 7860
attttgggtt acacatttac aagcaactta tataataata ctaa 7904

<210> 3
<211> 22
<212> DNA
<213> Homo sapiens
<220>
<221> misc_feature
<222> (1)..(20)
<223> brin sens de PML-rare

<220>
<221> misc_feature
<222> (21)..(22)
<223> residus thymine ajoutés

<400> 3
caugucaugu gucacaucuc tt 22

<210> 4
<211> 22
<212> DNA
<213> Homo sapiens
<220>
<221> misc_feature
<222> (1)..(20)
<223> brin anti-sens de PML-rare

<220>
<221> misc_feature
<222> (21)..(22)
<223> residus thimine ajoutés

<400> 4
gagaugugac acaugacaug tt 22

<210> 5
<211> 22
<212> DNA
<213> Homo sapiens
<220>
<221> misc_feature
<222> (1)..(20)
<223> brin sens PLM-rare

<220>
<221> misc_feature
<222> (21)..(22)
<223> residus thimine ajoutés

<400> 5
ggggaggcag ccauugagac tt 22

<210>  6
<211>  22
<212>  DNA
<213>  Homo sapiens
<220>
<221>  misc_feature
<222>  (1)..(20)
<223>  brin anti-sens PML-rare

<220>
<221>  misc_feature
<222>  (21)..(22)
<223>  Résidus thimine ajoutés

<400>  6
gucucaaugg cugccucccc tt                                                22

<210>  7
<211>  23
<212>  DNA
<213>  Homo sapiens
<220>
<221>  misc_feature
<222>  (1)..(21)
<223>  séquence issue du VGEF humain

<220>
<221>  misc_feature
<222>  (22)..(23)
<223>  résidus thimine ajoutés

<400>  7
augugaaugc agaccaaaga att                                               23

<210>  8
<211>  23
<212>  DNA
<213>  Homo sapiens
<220>
<221>  misc_feature
<222>  (1)..(21)
<223>  séquence issue du VGEF humain

<220>
<221>  misc_feature
<222>  (22)..(23)
<223>  residus thimine ajoutés

<400>  8
uucuuugguc ugcauucaca utt                                               23

<210>  9
<211>  22
<212>  DNA
<213>  Homo sapiens
<220>
<221>  misc_feature
<222>  (1)..(20)
<223>  séquence issue du VEGF humain

<220>
<221>  misc_feature
<222>  (21)..(22)

<223>  résidus thimine ajoutés

<400>  9
caugucaugu gucacaucuc tt                                                22

<210>  10
<211>  22
<212>  DNA
<213>  Homo sapiens
<220>
<221>  misc_feature
<222>  (1)..(20)
<223>  séquence issue du VEGF humain

<220>
<221>  misc_feature
<222>  (21)..(22)
<223>  résidus thimine ajoutés

<400>  10
gagaugugac acaugacaug tt                                                22

<210>  11
<211>  23
<212>  DNA
<213>  Homo sapiens
<220>
<221>  misc_feature
<222>  (1)..(21)
<223>  séquence issue du HIF 1 alpha humain

<220>
<221>  misc_feature
<222>  (22)..(23)
<223>  résidus thimine ajoutés

<400>  11
caugugacca ugaggaaaug att                                               23

<210>  12
<211>  23
<212>  DNA
<213>  Homo sapiens
<220>
<221>  misc_feature
<222>  (1)..(21)
<223>  séquence issue du HIF 1 alpha humain

<220>
<221>  misc_feature
<222>  (22)..(23)
<223>  résidus Thimine ajoutés

<400>  12
ucauuuccuc auggucacau gtt                                               23

<210>  13
<211>  23
<212>  DNA
<213>  Homo sapiens
<220>
<221>  misc_feature
<222>  (1)..(21)

<223>  séquence issue du HIF 1 alpha humain

<220>
<221>  misc_feature
<222>  (22)..(23)
<223>  résidus thimine ajoutés

<400>  13
gauagcaaug acgaaugcgu att                                              23

<210>  14
<211>  23
<212>  DNA
<213>  Homo sapiens
<220>
<221>  misc_feature
<222>  (1)..(21)
<223>  Séquence issue du HIF 1 alpha humain

<220>
<221>  misc_feature
<222>  (22)..(23)
<223>  Séquence issue du HIF 1 alpha humain

<400>  14
uacgcauucg ucauugcuau ctt                                              23

<210>  15
<211>  23
<212>  DNA
<213>  Homo sapiens
<220>
<221>  misc_feature
<222>  (1)..(21)
<223>  séquence issue du récepteur aux androgènes humain

<220>
<221>  misc_feature
<222>  (22)..(23)
<223>  residus thimine ajoutés

<400>  15
gacucagcug ccccauccac gtt                                              23

<210>  16
<211>  23
<212>  DNA
<213>  Homo sapiens
<220>
<221>  misc_feature
<222>  (1)..(21)
<223>  séquence issue du HIF 1 alpha humain

<220>
<221>  misc_feature
<222>  (22)..(23)
<223>  résidus thimine ajoutés

<400>  16
cguggauggg gcagcugagu ctt                                              23

<210>  17
<211>  23

<212>  DNA
<213>  Homo sapiens
<220>
<221>  misc_feature
<222>  (1)..(21)
<223>  séquence issue du HIF 1 alpha humain

<220>
<221>  misc_feature
<222>  (22)..(23)
<223>  résidus thimine ajoutés

<400>  17
gauagcaaug acgaaugcgu att                                              23

<210>  18
<211>  23
<212>  DNA
<213>  Homo sapiens
<220>
<221>  misc_feature
<222>  (1)..(21)
<223>  séquence issue du HIF 1alpha humain

<220>
<221>  misc_feature
<222>  (22)..(23)
<223>  residus thimine ajoutés

<400>  18
uacgcauucg ucauugcuau ctt                                              23

<210>  19
<211>  21
<212>  DNA
<213>  Homo sapiens
<220>
<221>  misc_feature
<222>  (1)..(19)
<223>  Séquence issue du récepteur aux androgènes portant la mutation T8
       77A

<220>
<221>  misc_feature
<222>  (20)..(21)
<223>  residus thimine ajoutés

<400>  19
gcaucaguuc gcuuuugact t                                                21

<210>  20
<211>  21
<212>  DNA
<213>  Homo sapiens
<220>
<221>  misc_feature
<222>  (1)..(19)
<223>  séquence issue du récepteur aux androgènes portant la mutation T8
       77A

<220>
<221>  misc_feature
<222>  (20)..(21)

<223>  résidus thimine ajoutés

<400>  20
gucaaaagcg aacugaugct t                                                21

<210>  21
<211>  21
<212>  DNA
<213>  Homo sapiens
<220>
<221>  misc_feature
<222>  (1)..(19)
<223>  séquence issue du p53 humain sauvage (sens)

<220>
<221>  misc_feature
<222>  (20)..(21)
<223>  residus thimine ajoutés

<400>  21
gcaugaaccg gaggcccaut t                                                21

<210>  22
<211>  21
<212>  DNA
<213>  Homo sapiens
<220>
<221>  misc_feature
<222>  (1)..(19)
<223>  séquence issue du gène p53 humain sauvage (antisens)

<220>
<221>  misc_feature
<222>  (20)..(21)
<223>  résidus thimine ajoutés

<400>  22
augggccucc gguucaugct t                                                21

<210>  23
<211>  21
<212>  DNA
<213>  Homo sapiens
<220>
<221>  misc_feature
<222>  (1)..(19)
<223>  séquence issue du p53 humain muté portant la mutation MT1 (r248w)
       (sens)

<220>
<221>  misc_feature
<222>  (20)..(21)
<223>  residus thimine ajoutés

<400>  23
gcaugaacug gaggcccaut t                                                21

<210>  24
<211>  21
<212>  DNA
<213>  Homo sapiens
<220>
<221>  misc_feature

<222>  (1)..(19)
<223>  séquence issue du p53 humain muté portant la mutation MT1 (r248w)
        (antisens)

<220>
<221>  misc_feature
<222>  (20)..(21)
<223>  résidus thimine ajoutés

<400>  24
augggccucc aguucaugct t                                                21

<210>  25
<211>  21
<212>  DNA
<213>  Homo sapiens
<220>
<221>  misc_feature
<222>  (1)..(19)
<223>  séquence issue du p53 humain muté portant la mutation MT2 (r248w)
        (sens)

<220>
<221>  misc_feature
<222>  (20)..(21)
<223>  résidus thimine ajoutés

<400>  25
ucaugaacug gaggcccaut t                                                21

<210>  26
<211>  21
<212>  DNA
<213>  Homo sapiens
<220>
<221>  misc_feature
<222>  (1)..(19)
<223>  séquence issue du p53 humain muté portant la mutation MT2 (r248w)
        (antisens)

<220>
<221>  misc_feature
<222>  (20)..(21)
<223>  résidus thimine ajoutés

<400>  26
augggccucc aguucaugat t                                                21

<210>  27
<211>  21
<212>  DNA
<213>  Homo sapiens
<220>
<221>  misc_feature
<222>  (1)..(19)
<223>  séquence issue du E6 de HPV (sens)

<220>
<221>  misc_feature
<222>  (20)..(21)
<223>  réidus thimine ajoutés

<400>  27

ccacaguuau gcacagagct t                                                21

<210>  28
<211>  20
<212>  DNA
<213>  Homo sapiens
<220>
<221>  misc_feature
<222>  (1)..(18)
<223>  séquence issue du E6 de HPV (antisens)

<220>
<221>  misc_feature
<222>  (19)..(20)
<223>  résidus thimine ajoutés

<400>  28
gcucugugca uaacuuggtt                                                   20

<210>  29
<211>  22
<212>  DNA
<213>  Homo sapiens
<220>
<221>  misc_feature
<222>  (1)..(22)
<223>  séquence issue du gène codant la GFP  (brin sens)

<400>  29
gcaagctgac cctgaagttc at                                                22

<210>  30
<211>  22
<212>  DNA
<213>  Homo sapiens
<220>
<221>  misc_feature
<222>  (1)..(22)
<223>  séquence issue du gène codant la GFP  (brin anti-sens)

<400>  30
gaacuucagg gucagcuugc cg                                                22

<210>  31
<211>  22
<212>  DNA
<213>  Homo sapiens
<220>
<221>  misc_feature
<222>  (1)..(20)
<223>  séquence issue du gène codant la GFP  (brin sens)

<220>
<221>  misc_feature
<222>  (21)..(22)
<223>  résidus thimine ajoutés

<400>  31
caugucaugu gucacaucuc tt                                                22

<210>  32
<211>  22
<212>  DNA

<213>  Homo sapiens
<220>
<221>  misc_feature
<222>  (1)..(20)
<223>  séquence issue du gène codant la GFP  (brin antisens)

<220>
<221>  misc_feature
<222>  (21)..(22)
<223>  résidus thimine ajoutés

<400>  32
gagaugugac acaugacaug tt                                              22

<210>  33
<211>  21
<212>  DNA
<213>  Homo sapiens
<220>
<221>  misc_feature
<222>  (1)..(21)
<223>  séquence issue du récepteur aux androgènes humain  muté (brin sen
       s)

<400>  33
gcatcagttc gcttttgact t                                               21

<210>  34
<211>  21
<212>  DNA
<213>  Homo sapiens
<220>
<221>  misc_feature
<222>  (1)..(19)
<223>  séquence issue du récepteur aux androgènes humain  muté (brin sen
       s)

<220>
<221>  misc_feature
<222>  (20)..(21)
<223>  résidus thimine ajoutés

<400>  34
gcaucaguuc gcuuuugact t                                               21

<210>  35
<211>  21
<212>  DNA
<213>  Homo sapiens
<220>
<221>  misc_feature
<222>  (1)..(21)
<223>  séquence issue du récepteur aux androgènes humain  muté (brin sen
       s)

<400>  35
gtcaaaagcg aactgatgct t                                               21

<210>  36
<211>  21
<212>  DNA
<213>  Homo sapiens
<220>

<221>  misc_feature
<222>  (1)..(19)
<223>  séquence issue du récepteur aux androgènes humain  muté (brin sen
       s)

<220>
<221>  misc_feature
<222>  (20)..(21)
<223>  residus thimine ajoutés

<400>  36
gucaaaagcg aacugaugct t                                                21

<210>  37
<211>  21
<212>  DNA
<213>  Homo sapiens
<220>
<221>  misc_feature
<222>  (1)..(19)
<223>  séquence issue du récepteur aux androgènes humain  muté (brin sen
       s)

<220>
<221>  misc_feature
<222>  (20)..(21)
<223>  résidus thimine ajoutés

<400>  37
guucggucug cuuacacuat t                                                21

<210>  38
<211>  21
<212>  DNA
<213>  Homo sapiens
<220>
<221>  misc_feature
<222>  (1)..(19)
<223>  séquence issue du récepteur aux androgènes humain  muté (brin ant
       isens)

<220>
<221>  misc_feature
<222>  (20)..(21)
<223>  résidus thimine ajoutés

<400>  38
uaguguaagc agaccgaact t                                                21

<210>  39
<211>  21
<212>  DNA
<213>  Homo sapiens
<220>
<221>  misc_feature
<222>  (1)..(19)
<223>  séquence issue du gène p53 humain sauvage (brin sens)

<220>
<221>  misc_feature
<222>  (20)..(21)
<223>  residus thimine ajoutés

<400>  39
gcaugaaccg gaggcccaut t                                                21

<210>  40
<211>  21
<212>  DNA
<213>  Homo sapiens
<220>
<221>  misc_feature
<222>  (1)..(19)
<223>  séquence issue du gène p53 humain sauvage (brin  antisens)

<220>
<221>  misc_feature
<222>  (20)..(21)
<223>  résidus thimine ajoutés

<400>  40
augggccucc gguucaugct t                                                21

<210>  41
<211>  21
<212>  DNA
<213>  Homo sapiens
<220>
<221>  misc_feature
<222>  (1)..(19)
<223>  séquence issue du gène p53 humain muté (brin sens)

<220>
<221>  misc_feature
<222>  (20)..(21)
<223>  résidus thimine ajoutés

<400>  41
gcaugaaccg gaggcccaut t                                                21

<210>  42
<211>  21
<212>  DNA
<213>  Homo sapiens
<220>
<221>  misc_feature
<222>  (1)..(19)
<223>  séquence issue du gène p53 humain sauvage (brin antisens)

<220>
<221>  misc_feature
<222>  (20)..(21)
<223>  résidus thimine ajoutés

<400>  42
augggccucc gguucaugct t                                                21

<210>  43
<211>  21
<212>  DNA
<213>  Homo sapiens
<220>
<221>  misc_feature
<222>  (1)..(19)
<223>  séquence issue du gène p53 muté (brin sens)

<220>
<221>  misc_feature
<222>  (20)..(21)
<223>  résidus thimine ajoutés

<400>  43
gcaugaaccg gaggcccaut t                                                21

<210>  44
<211>  21
<212>  DNA
<213>  Homo sapiens
<220>
<221>  misc_feature
<222>  (1)..(19)
<223>  séquence issu du gène p53 humain muté (brin antisens)

<220>
<221>  misc_feature
<222>  (20)..(21)
<223>  résidus thimine ajoutés

<400>  44
augggccucc gguucaugct t                                                21

<210>  45
<211>  21
<212>  DNA
<213>  Homo sapiens
<220>
<221>  misc_feature
<222>  (1)..(19)
<223>  séquence issue du gène p53 muté (brin sens)

<220>
<221>  misc_feature
<222>  (20)..(21)
<223>  résidus thimine ajoutés

<400>  45
gcatgaaccg gaggcccatt t                                                21

<210>  46
<211>  21
<212>  DNA
<213>  Homo sapiens
<220>
<221>  misc_feature
<222>  (1)..(19)
<223>  séquence issue du gène p53 humain muté (brin antisens)

<220>
<221>  misc_feature
<222>  (20)..(21)
<223>  résidus thimine ajoutés

<400>  46
augggccucc gguucaugct t                                                21

<210>  47
<211>  21
<212>  DNA
<213>  Homo sapiens

<220>
<221>  misc_feature
<222>  (1)..(19)
<223>  séquence issue du gène p53 humain muté (brin sens)

<220>
<221>  misc_feature
<222>  (20)..(21)
<223>  résidus thimine ajoutés

<400>  47
gcaugaaccg gaggcccaut t                                                21

<210>  48
<211>  22
<212>  DNA
<213>  Homo sapiens
<220>
<221>  misc_feature
<222>  (1)..(20)
<223>  séquence issue du gène p53 humain muté (brin antisens)

<220>
<221>  misc_feature
<222>  (21)..(22)
<223>  résidus thimine ajoutés

<400>  48
atgggccutc cggttcatgc tt                                               22

<210>  49
<211>  21
<212>  DNA
<213>  Homo sapiens
<220>
<221>  misc_feature
<222>  (1)..(19)
<223>  séquence issue du gène p53 humain muté (brin sens)

<220>
<221>  misc_feature
<222>  (20)..(21)
<223>  résidus thimine ajoutés

<400>  49
gcaugaacug gaggcccaut t                                                21

<210>  50
<211>  21
<212>  DNA
<213>  Homo sapiens
<220>
<221>  misc_feature
<222>  (1)..(19)
<223>  séquence issue du gène p53 humain muté (brin antisens)

<220>
<221>  misc_feature
<222>  (20)..(21)
<223>  résidus thimine ajoutés

<400>  50
augggccucc aguucaugct t                                                21

<210>  51
<211>  21
<212>  DNA
<213>  Homo sapiens
<220>
<221>  misc_feature
<222>  (1)..(19)
<223>  séquence issue du gène p53 humain muté (brin sens)

<220>
<221>  misc_feature
<222>  (20)..(21)
<223>  résidus thimine ajoutés

<400>  51
gcaugaacug gaggcccaut t                                                21

<210>  52
<211>  21
<212>  DNA
<213>  Homo sapiens
<220>
<221>  misc_feature
<222>  (1)..(19)
<223>  séquence issue du gène p53 humain muté (brin antisens)

<220>
<221>  misc_feature
<222>  (20)..(21)
<223>  résidus thimine ajoutés

<400>  52
augggccucc aguucaugct t                                                21

<210>  53
<211>  21
<212>  DNA
<213>  Homo sapiens
<220>
<221>  misc_feature
<222>  (1)..(19)
<223>  séquence issue du gène p53 humain muté (brin isens)

<220>
<221>  misc_feature
<222>  (20)..(21)
<223>  résidus thimine ajoutés

<400>  53
gcaugaacug gaggcccaut t                                                21

<210>  54
<211>  21
<212>  DNA
<213>  Homo sapiens
<220>
<221>  misc_feature
<222>  (1)..(19)
<223>  séquence issue du gène p53 humain muté (brin antisens)

<220>
<221>  misc_feature

<222>  (20)..(21)
<223>  résidus thimine ajoutés

<400>  54
augggccucc aguucaugct t                                                21

<210>  55
<211>  21
<212>  DNA
<213>  Homo sapiens
<220>
<221>  misc_feature
<222>  (1)..(19)
<223>  séquence issue du gène p53 humain muté (brin sens)

<220>
<221>  misc_feature
<222>  (20)..(21)
<223>  résidus thimine ajoutés

<400>  55
gcatgaactg gaggcccatt t                                                21

<210>  56
<211>  21
<212>  DNA
<213>  Homo sapiens
<220>
<221>  misc_feature
<222>  (1)..(19)
<223>  séquence issue du gène p53 humain muté (brin antisens)

<220>
<221>  misc_feature
<222>  (20)..(21)
<223>  résidus thimine ajoutés

<400>  56
augggccucc aguucaugct t                                                21

<210>  57
<211>  21
<212>  DNA
<213>  Homo sapiens
<220>
<221>  misc_feature
<222>  (1)..(19)
<223>  séquence issue du gène p53 humain muté (brin sens)

<220>
<221>  misc_feature
<222>  (20)..(21)
<223>  résidus thimine ajoutés

<400>  57
gcatgaactg gaggcccatt t                                                21

<210>  58
<211>  21
<212>  DNA
<213>  Homo sapiens
<220>
<221>  misc_feature

<222>  (1)..(19)
<223>  séquence issue du gène p53 humain muté (brin antisens)

<220>
<221>  misc_feature
<222>  (20)..(21)
<223>  résidus thimine ajoutés

<400>  58
augggccucc aguucaugct t                                                21

<210>  59
<211>  3933
<212>  DNA
<213>  Homo sapiens
<220>
<221>  misc_feature
<222>  (1)..(3933)
<223>  Homo sapiens hypoxia-inducible factor 1 sous-unité alpha. (HIF-1
       alpha)

<400>  59
cacgaggcag cactctcttc gtcgcttcgg ccagtgtgtc gggctgggcc ctgacaagcc      60
acctgaggag aggctcggag ccgggcccgg accccggcga ttgccgcccg cttctctcta     120
gtctcacgag gggtttcccg cctcgcaccc ccacctctgg acttgccttt ccttctcttc     180
tccgcgtgtg gagggagcca gcgcttaggc cggagcgagc ctgggggccg cccgccgtga     240
agacatcgcg gggaccgatt caccatggag ggcgccggcg gcgcgaacga caagaaaaag     300
ataagttctg aacgtcgaaa agaaaagtct cgagatgcag ccagatctcg gcgaagtaaa     360
gaatctgaag ttttttatga gcttgctcat cagttgccac ttccacataa tgtgagttcg     420
catcttgata aggcctctgt gatgaggctt accatcagct atttgcgtgt gaggaaactt     480
ctggatgctg gtgatttgga tattgaagat gacatgaaag cacagatgaa ttgcttttat     540
ttgaaagcct tggatggttt tgttatggtt ctcacagatg atggtgacat gatttacatt     600
tctgataatg tgaacaaata catgggatta actcagtttg aactaactgg acacagtgtg     660
tttgatttta ctcatccatg tgaccatgag gaaatgagag aaatgcttac acacagaaat     720
ggccttgtga aaaagggtaa agaacaaaac acacagcgaa gcttttttct cagaatgaag     780
tgtaccctaa ctagccgagg aagaactatg aacataaagt ctgcaacatg gaaggtattg     840
cactgcacag gccacattca cgtatatgat accaacagta accaacctca gtgtgggtat     900
aagaaaccac ctatgacctg cttggtgctg atttgtgaac ccattcctca cccatcaaat    960
attgaaattc ctttagatag caagactttc ctcagtcgac acagcctgga tatgaaattt    1020
tcttattgtg atgaaagaat taccgaattg atgggatatg agccagaaga acttttaggc    1080
cgctcaattt atgaatatta tcatgctttg gactctgatc atctgaccaa aactcatcat    1140
gatatgttta ctaaaggaca agtcaccaca ggacagtaca ggatgcttgc caaaagaggt    1200
ggatatgtct gggttgaaac tcaagcaact gtcatatata acaccaagaa ttctcaacca    1260
cagtgcattg tatgtgtgaa ttacgttgtg agtggtatta ttcagcacga cttgattttc    1320
tcccttcaac aaacagaatg tgtccttaaa ccggttgaat cttcagatat gaaaatgact    1380
cagctattca ccaaagttga atcagaagat acaagtagcc tctttgacaa acttaagaag    1440
gaacctgatg ctttaacttt gctggcccca gccgctggag acacaatcat atctttagat    1500
tttggcagca acgacacaga aactgatgac cagcaacttg aggaagtacc attatataat    1560
gatgtaatgc tcccctcacc caacgaaaaa ttacagaata taaatttggc aatgtctcca    1620
ttacccaccg ctgaaacgcc aaagccactt cgaagtagtg ctgaccctgc actcaatcaa    1680
gaagttgcat taaaattaga accaaatcca gagtcactgg aactttcttt taccatgccc    1740
cagattcagg atcagacacc tagtccttcc gatggaagca ctagacaaag ttcacctgag    1800
cctaatagtc ccagtgaata ttgtttttat gtggatagtg atatggtcaa tgaattcaag    1860
ttggaattgg tagaaaaact ttttgctgaa gacacagaag caaagaaccc attttctact    1920
caggacacag atttagactt ggagatgtta gctccctata tcccaatgga tgatgacttc    1980
cagttacgtt ccttcgatca gttgtcacca ttagaaagca gttccgcaag ccctgaaagc    2040
gcaagtcctc aaagcacagt tacagtattc cagcagactc aaatacaaga acctactgct    2100
aatgccacca ctaccactgc caccactgat gaattaaaaa cagtgacaaa agaccgtatg    2160
gaagacatta aaatattgat tgcatctcca tctcctaccc acatacataa agaaactact    2220
agtgccacat catcaccata tagagatact caaagtcgga cagcctcacc aaacagagca    2280
ggaaaaggag tcatagaaca gacagaaaaa tctcatccaa gaagccctaa cgtgttatct    2340
gtcgctttga gtcaaagaac tacagttcct gaggaagaac taaatccaaa gatactagct    2400
ttgcagaatg ctcagagaaa gcgaaaaatg gaacatgatg gttcactttt tcaagcagta    2460

ggaattggaa cattattaca gcagccagac gatcatgcag ctactacatc actttcttgg 2520
aaacgtgtaa aaggatgcaa atctagtgaa cagaatggaa tggagcaaaa gacaattatt 2580
ttaataccct ctgatttagc atgtagactg ctggggcaat caatggatga aagtggatta 2640
ccacagctga ccagttatga ttgtgaagtt aatgctccta tacaaggcag cagaaaccta 2700
ctgcagggtg aagaattact cagagctttg gatcaagtta actgagcttt ttcttaattt 2760
cattcctttt tttggacact ggtggctcac tacctaaagc agtctattta tattttctac 2820
atctaatttt agaagcctgg ctacaatact gcacaaactt ggttagttca atttttgatc 2880
ccctttctac ttaatttaca ttaatgctct tttttagtat gttctttaat gctggatcac 2940
agacagctca ttttctcagt tttttggtat ttaaaccatt gcattgcagt agcatcattt 3000
taaaaaatgc acctttttat ttatttattt ttggctaggg agtttatccc tttttcgaat 3060
tatttttaag aagatgccaa tataattttt gtaagaaggc agtaaccttt catcatgatc 3120
ataggcagtt gaaaaatttt tacacctttt ttttcacatt ttacataaat aataatgctt 3180
tgccagcagt acgtggtagc cacaattgca caatatattt tcttaaaaaa taccagcagt 3240
tactcatgga atatattctg cgtttataaa actagttttt aagaagaaat tttttttggc 3300
ctatgaaatt gttaaacctg gaacatgaca ttgttaatca tataataatg attcttaaat 3360
gctgtatggt ttattattta aatgggtaaa gccatttaca taatatagaa agatatgcat 3420
atatctagaa ggtatgtggc atttatttgg ataaaattct caattcagag aaatcatctg 3480
atgtttctat agtcactttg ccagctcaaa agaaaacaat accctatgta gttgtggaag 3540
tttatgctaa tattgtgtaa ctgatattaa acctaaatgt tctgcctacc ctgttggtat 3600
aaagatattt tgagcagact gtaaacaaga aaaaaaaaat catgcattct tagcaaaatt 3660
gcctagtatg ttaatttgct caaaatacaa tgtttgattt tatgcacttt gtcgctatta 3720
acatcctttt tttcatgtag atttcaataa ttgagtaatt ttagaagcat tattttagga 3780
atatatagtt gtcacagtaa atatcttgtt ttttctatgt acattgtaca aatttttcat 3840
tccttttgct ctttgtggtt ggatctaaca ctaactgtat tgttttgtta catcaaataa 3900
acatcttctg tggaaaaaaa aaaaaaaaaa aaa 3933

<210> 60
<211> 3166
<212> DNA
<213> Homo sapiens
<220>
<221> misc_feature
<222> (1)..(3166)
<223> VEGF A humain

<400> 60
aagagctcca gagagaagtc gaggaagaga gagacggggt cagagagagc gcgcgggcgt 60
gcgagcagcg aaagcgacag gggcaaagtg agtgacctgc ttttgggggt gaccgccgga 120
gcgcggcgtg agccctcccc cttgggatcc cgcagctgac cagtcgcgct gacggacaga 180
cagacagaca ccgcccccag ccccagttac cacctcctcc ccggccggcg gcggacagtg 240
gacgcggcgg cgagccgcgg gcaggggccg gagcccgccc ccggaggcgg ggtggagggg 300
gtcggagctc gcggcgtcgc actgaaactt ttcgtccaac ttctgggctg ttctcgcttc 360
ggaggagccg tggtccgcgc gggggaagcc gagccgagcg gagccgcgag aagtgctagc 420
tcgggccggg aggagccgca gccggaggag ggggaggagg aagaagagaa ggaagaggag 480
agggggccgc agtggcgact cggcgctcgg aagccgggct catggacggg tgaggcggcg 540
gtgtgcgcag acagtgctcc agcgcgcgcg ctccccagcc ctggcccggc ctcgggccgg 600
gaggaagagt agctcgccga ggcgccgagg agagcgggcc gccccacagc ccgagccgga 660
gagggacgcg agccgcgcgc cccggtcggg cctccgaaac catgaacttt ctgctgtctt 720
gggtgcattg gagccttgcc ttgctgctct acctccacca tgccaagtgg tcccaggctg 780
cacccatggc agaaggagga gggcagaatc atcacgaagt ggtgaagttc atggatgtct 840
atcagcgcag ctactgccat ccaatcgaga ccctggtgga catcttccag gagtaccctg 900
atgagatcga gtacatcttc aagccatcct gtgtgcccct gatgcgatgc gggggctgct 960
ccaatgacga gggcctggag tgtgtgccca ctgaggagtc caacatcacc atgcagatta 1020
tgcggatcaa acctcaccaa ggccagcaca taggagagat gagcttccta cagcacaaca 1080
aatgtgaatg cagaccaaag aaagatagag caagacaaga aaatccctgt gggccttgct 1140
cagagcggag aaagcatttg tttgtacaag atccgcagac gtgtaaatgt tcctgcaaaa 1200
acacacactc gcgttgcaag gcgaggcagc ttgagttaaa cgaacgtact tgcagatgtg 1260
acaagccgag gcggtgagcc gggcaggagg aaggagcctc cctcagggtt tcgggaacca 1320
gatctctctc caggaaagac tgatacagaa cgatcgatac agaaaccacg ctgccgccac 1380
cacaccatca ccatcgacag aacagtcctt aatccagaaa cctgaaatga aggaagagga 1440
gactctgcgc agagcacttt gggtccggag ggcgagactc cggcggaagc attcccgggc 1500
gggtgaccca gcacggtccc tcttggaatt ggattcgcca ttttattttt cttgctgcta 1560
aatcaccgag cccggaagat tagagagttt tatttctggg attcctgtag acacacccac 1620

ccacatacat acatttatat atatatatat tatatatata taaaaataaa tatctctatt 1680
ttatatatat aaaatatata tattcttttt ttaaattaac agtgctaatg ttattggtgt 1740
cttcactgga tgtatttgac tgctgtggac ttgagttggg aggggaatgt tcccactcag 1800
atcctgacag ggaagaggag gagatgagag actctggcat gatctttttt ttgtcccact 1860
tggtggggcc agggtcctct cccctgccca agaatgtgca aggccagggc atgggggcaa 1920
atatgaccca gttttgggaa caccgacaaa cccagccctg gcgctgagcc tctctacccc 1980
aggtcagacg gacagaaaga caaatcacag gttccgggat gaggacaccg gctctgacca 2040
ggagtttggg gagcttcagg acattgctgt gctttgggga ttccctccac atgctgcacg 2100
cgcatctcgc ccccaggggc actgcctgga agattcagga gcctgggcgg ccttcgctta 2160
ctctcacctg cttctgagtt gcccaggagg ccactggcag atgtcccggc gaagagaaga 2220
gacacattgt tggaagaagc agcccatgac agcgcccctt cctgggactc gccctcatcc 2280
tcttcctgct ccccttcctg gggtgcagcc taaaaggacc tatgtcctca caccattgaa 2340
accactagtt ctgtcccccc aggaaacctg gttgtgtgtg tgtgagtggt tgaccttcct 2400
ccatcccctg gtccttccct tcccttcccg aggcacagag agacagggca ggatccacgt 2460
gcccattgtg gaggcagaga aaagagaaag tgttttatat acggtactta tttaatatcc 2520
ctttttaatt agaaattaga acagttaatt taattaaaga gtagggtttt ttttcagtat 2580
tcttggttaa tatttaattt caactattta tgagatgtat cttttgctct ctcttgctct 2640
cttatttgta ccggtttttg tatataaaat tcatgtttcc aatctctctc tccctgatcg 2700
gtgacagtca ctagcttatc ttgaacagat atttaatttt gctaacactc agctctgccc 2760
tccccgatcc cctggctccc cagcacacat tcctttgaaa gagggtttca atatacatct 2820
acatactata tatatattgg gcaacttgta tttgtgtgta tatatatata tatatgttta 2880
tgtatatatg tgatcctgaa aaaataaaca tcgctattct gttttttata tgttcaaacc 2940
aaacaagaaa aaatagagaa ttctacatac taaatctctc tcctttttta attttaatat 3000
ttgttatcat ttatttattg gtgctactgt ttatccgtaa taattgtggg gaaaagatat 3060
taacatcacg tctttgtctc tagtgcagtt tttcgagata ttccgtagta catatttatt 3120
tttaaacaac gacaaagaaa tacagatata tcttaaaaaa aaaaaa 3166

<210> 61
<211> 17
<212> DNA
<213> Homo sapiens
<220>
<221> misc_feature
<222> (1)..(17)
<223> séquence issue du gène p53 humain sauvage

<400> 61
gaggtgcgtg tttgtgc 17

<210> 62
<211> 19
<212> DNA
<213> Homo sapiens
<220>
<221> misc_feature
<222> (1)..(19)
<223> séquence issue du gène p53 humain sauvage

<400> 62
gcatgaaccg gaggcccat 19

<210> 63
<211> 19
<212> DNA
<213> Homo sapiens
<220>
<221> misc_feature
<222> (1)..(19)
<223> séquence issue du gène p53 humain sauvage

<400> 63
gcatgaaccg gaggcccat 19

<210>  64
<211>  19
<212>  DNA
<213>  Homo sapiens
<220>
<221>  misc_feature
<222>  (1)..(19)
<223>  séquence issue du gène p53 humain sauvage

<400>  64
gcatgaaccg gaggcccat                                                    19

<210>  65
<211>  19
<212>  DNA
<213>  Homo sapiens
<220>
<221>  misc_feature
<222>  (1)..(19)
<223>  séquence issue du gène p53 humain sauvage

<400>  65
ctgcatgggc ggcatgaac                                                    19

<210>  66
<211>  19
<212>  DNA
<213>  Homo sapiens
<220>
<221>  misc_feature
<222>  (1)..(19)
<223>  séquence issue du gène p53 humain sauvage

<400>  66
tgggagagac cggcgcaca                                                    19

<210>  67
<211>  19
<212>  DNA
<213>  Homo sapiens
<220>
<221>  misc_feature
<222>  (1)..(19)
<223>  séquence issue du gène p53 humain sauvage

<400>  67
tgtgaggcac tgcccccac                                                    19

<210>  68
<211>  20
<212>  DNA
<213>  Homo sapiens
<220>
<221>  misc_feature
<222>  (1)..(20)
<223>  séquence issue du gène p53 humain sauvage

<400>  68
taacagttcc tgcatgggcg                                                   20

<210>  69
<211>  17
<212>  DNA

<213>  Homo sapiens
<220>
<221>  misc_feature
<222>  (1)..(17)
<223>  séquence issue du gène p53 humain muté, portant la mutation r273h
      .

<400>  69
gaggtgcatg tttgtgc                                                    17

<210>  70
<211>  19
<212>  DNA
<213>  Homo sapiens
<220>
<221>  misc_feature
<222>  (1)..(19)
<223>  séquence issue du gène p53 humain muté, portant la mutation r248q

<400>  70
gcatgaacca gaggcccat                                                  19

<210>  71
<211>  18
<212>  DNA
<213>  Homo sapiens
<220>
<221>  misc_feature
<222>  (1)..(18)
<223>  séquence issue du gène p53 humain muté, portant la mutation r248w

<400>  71
gcatgaactg gaggccat                                                   18

<210>  72
<211>  19
<212>  DNA
<213>  Homo sapiens
<220>
<221>  misc_feature
<222>  (1)..(19)
<223>  séquence issue du gène p53 humain muté, portant la mutation r249s

<400>  72
gcatgaaccg gagtcccat                                                  19

<210>  73
<211>  19
<212>  DNA
<213>  Homo sapiens
<220>
<221>  misc_feature
<222>  (1)..(19)
<223>  séquence issue du gène p53 humain muté, portant la mutation g245s

<400>  73
ctgcatgggc agcatgaac                                                  19

<210>  74
<211>  19
<212>  DNA
<213>  Homo sapiens
<220>

<221>  misc_feature
<222>  (1)..(19)
<223>  séquence issue du gène p53 humain muté, portant la mutation r282w

<400>  74
tgggagagac tggcgcaca                                                    19

<210>  75
<211>  19
<212>  DNA
<213>  Homo sapiens
<220>
<221>  misc_feature
<222>  (1)..(19)
<223>  séquence issue du gène p53 humain muté, portant la mutation r175h

<400>  75
tgtgaggcgc tgcccccac                                                    19

<210>  76
<211>  20
<212>  DNA
<213>  Homo sapiens
<220>
<221>  misc_feature
<222>  (1)..(20)
<223>  séquence issue du gène p53 humain muté, portant la mutation c242s

<400>  76
taacagttcc tccatgggcg                                                   20

<210>  77
<211>  3231
<212>  DNA
<213>  Homo sapiens
<220>
<221>  misc_feature
<222>  (1)..(3231)
<223>  séquence codant pour le récepteur aux androgènes humain.

<400>  77
agctagctgc agcgactacc gcatcatcac agcctgttga actcttctga gcaagagaag      60
gggaggcggg gtaagggaag taggtggaag attcagccaa gctcaaggat ggaagtgcag     120
ttagggctgg gaagggtcta ccctcggccg ccgtccaaga cctaccgagg agctttccag     180
aatctgttcc agagcgtccg cgaagtgatc cagaacccgg gccccaggca cccagaggcc     240
gcgagcgcag cacctcccgg cgccagtttg ctgctgctgc agcagcagca gcagcagcag     300
cagcagcagc agcagcagca gcagcaagag actagcccca ggcagcagca gcagcagcag     360
ggtgaggatg gttctcccca agcccatcgt agaggcccca caggctacct ggtcctggat     420
gaggaacagc aaccttcaca gccgcagtcg gccctggagt gccaccccga gagaggttgc     480
gtcccagagc ctggagccgc cgtggccgcc agcaaggggc tgccgcagca gctgccagca     540
cctccggacg aggatgactc agctgcccca tccacgttgt ccctgctggc ccccactttc     600
cccggcttaa gcagctgctc cgctgacctt aaagacatcc tgagcgaggc cagcaccatg     660
caactccttc agcaacagca gcaggaagca gtatccgaag gcagcagcag cgggagagcg     720
agggaggcct cgggggctcc cacttcctcc aaggacaatt acttaggggg cacttcgacc     780
atttctgaca acgccaagga gttgtgtaag gcagtgtcgg tgtccatggg cctgggtgtg     840
gaggcgttgg agcatctgag tccaggggaa cagcttcggg gggattgcat gtacgcccca     900
cttttgggag ttccacccgc tgtgcgtccc actccttgtg ccccattggc cgaatgcaaa     960
ggttctctgc tagacgacag cgcaggcaag agcactgaag atactgctga gtattcccct    1020
ttcaagggag gttacaccaa agggctagaa ggcgagagcc taggctgctc tggcagcgct    1080
gcagcaggga gctccgggac acttgaactg ccgtctaccc tgtctctcta caagtccgga    1140
gcactggacg aggcagctgc gtaccagagt cgcgactact acaactttcc actggctctg    1200
gccggaccgc cgccccctcc gccgcctccc catccccacg ctcgcatcaa gctggagaac    1260
ccgctggact acggcagcgc ctgggcggct gcggcggcgc agtgccgcta tggggacctg    1320

gcgagcctgc atggcgcggg tgcagcggga cccggttctg ggtcaccctc agccgccgct 1380
tcctcatcct ggcacactct cttcacagcc gaagaaggcc agttgtatgg accgtgtggt 1440
ggtggtgggg gtggtggcgg cggcggcggc ggcggcggcg gcggcgaggc gggagctgta 1500
gccccctacg gctacactcg gccccctcag gggctggcgg gccaggaaag cgacttcacc 1560
gcacctgatg tgtggtaccc tggcggcatg gtgagcagag tgccctatcc cagtcccact 1620
tgtgtcaaaa gcgaaatggg cccctggatg gatagctact ccggacctta cggggacatg 1680
cgtttggaga ctgccaggga ccatgttttg cccattgact attactttcc accccagaag 1740
acctgcctga tctgtggaga tgaagcttct gggtgtcact atggagctct cacatgtgga 1800
agctgcaagg tcttcttcaa aagagccgct gaagggaaac agaagtacct gtgcgccagc 1860
agaaatgatt gcactattga taaattccga aggaaaaatt gtccatcttg tcgtcttcgg 1920
aaatgttatg aagcagggat gactctggga gcccggaagc tgaagaaact tggtaatctg 1980
aaactacagg aggaaggaga ggcttccagc accaccagcc ccactgagga gacaacccag 2040
aagctgacag tgtcacacat tgaaggctat gaatgtcagc ccatctttct gaatgtcctg 2100
gaagccattg agccaggtgt agtgtgtgct ggacacgaca acaaccagcc cgactccttt 2160
gcagccttgc tctctagcct caatgaactg ggagagagac agcttgtaca cgtggtcaag 2220
tgggccaagg ccttgcctgg cctccgcaac ttacacgtgg acgaccagat ggctgtcatt 2280
cagtactcct ggatggggct catggtgttt gccatgggct ggcgatcctt caccaatgtc 2340
aactccagga tgctctactt cgcccctgat ctggttttca atgagtaccg catgcacaag 2400
tcccggatgt acagccagtg tgtccgaatg aggcacctct ctcaagagtt tggatggctc 2460
caaatcaccc cccaggaatt cctgtgcatg aaagccatgc tactcttcag cattattcca 2520
gtggatgggc tgaaaaatca aaaattcttt gatgaacttc gaatgaacta catcaaggaa 2580
ctcgatcgta tcattgcatg caaaagaaaa aatcccacat cctgctcaag acgcttctac 2640
cagctcacca agctcctgga ctccgtgcag cctattgcga gagagctgca tcagttcact 2700
tttgacctgc taatcaagtc acacatggtg agcgtggact ttccggaaat gatggcagag 2760
atcatctctg tgcaagtgcc caagatcctt tctgggaaag tcaagcccat ctatttccac 2820
acccagtgaa gcattggaaa ccctatttcc ccaccccagc tcatgccccc tttcagatgt 2880
cttctgcctg ttataactct gcactactcc tctgcagtgc cttggggaat ttcctctatt 2940
gatgtacagt ctgtcatgaa catgttcctg aattctatct gctgggcttt ttttttctct 3000
ttctctcctt tctttttctt cttccctccc tatctaaccc tcccatggca ccttcagact 3060
ttgcttccca ttgtggctcc tatctgtgtt ttgaatggtg ttgtatgcct taaatctgtg 3120
atgatcctca tatggcccag tgtcaagttg tgcttgttta cacgcatctc tgtgccagcc 3180
acacaaaccg tttacttact taccgcaagg gaacttagag agctagaatt c 3231

## Description

La présente invention concerne le domaine de l'investigation et du traitement génétiques de pathologies humaines, notamment les cancers ou les maladies infectieuses. Plus particulièrement, l'invention vise à offrir des moyens pour déterminer la fonction d'un gène ou d'une famille de gènes impliqués dans un processus cellulaire, et pour réprimer un gène nocif responsable d'une pathologie chez l'homme ou l'animal. L'invention se rapporte aux agents actifs pour la mise en oeuvre de ces méthodes et les compositions les contenant.

On connaît dans l'art antérieur, des techniques d'oligonucléotides anti-sens permettant d'inhiber spécifiquement un gène dans les cellules de mammifères. Ces techniques sont basées sur l'introduction dans les cellules d'un court oligonucléotide d'ADN complémentaire du gène cible. Cet oligonucléotide induit la dégradation de l'ARN messager transcrit par le gène cible. Un autre mode d'action des anti-sens consiste à introduire dans la cellule un oligonucléotide d'ADN qui va former une triple hélice avec le gène cible. La formation de cette triple hélice réprime le gène soit en bloquant l'accès pour des protéines activatrices, soit dans des approches plus sophistiquées, en induisant la dégradation du gène. Aucune de ces approches ne semble s'appuyer sur un mécanisme cellulaire existant dans les cellules de mammifères, et elles se sont avérés peu efficaces. En effet, l'utilisation des anti-sens en clinique est réduite à quelques cas très rares, et il n'y a aucune utilisation possible des oligonucléotides formant triple hélice.

La méthode de l'invention est basée sur l'interférence ARN désigné aussi « RNA'inh » ou « RNAi » ou encore co-suppression, qui a été mise en évidence dans les plantes. Chez les plantes, il a été observé que l'introduction d'un long ARN double brin, correspondant à un gène, induit la répression spécifique et efficace du gène ciblé. Le mécanisme de cette interférence comporte la dégradation de l'ARN double brin en courts duplex d'oligonucléotides de 20 à 22 nucléotides.

Les Inventeurs ont maintenant montré que ce principe peut s'appliquer à des gènes de mammifères qui jouent un rôle important dans le contrôle du destin cellulaire.

L'approche « RNA'inh » plus généralement dénommée selon l'invention oligonucléotides inhibiteurs ou ARNi s'appuie sur un mécanisme cellulaire dont l'importance est soulignée par son grand degré de conservation puisque ce mécanisme est conservé à travers les règnes et les espèces et a été montré non seulement chez la plante, mais aussi chez le vers Caenorhabditis Elegans et la levure et les mammifères, homme et souris.

Les travaux de recherche réalisés dans le cadre de l'invention ont montré que cette approche est beaucoup plus efficace pour réprimer spécifiquement les gènes que les techniques envisagées dans l'art antérieur. En outre, elle réunit potentiellement les avantages des anti-sens et des anti-gènes. En effet, chez la plante, la co-suppression s'effectue au niveau post-transcriptionnel, sur l'ARN mature, mais aussi au niveau transcriptionnel, donc sur le gène lui-même. En effet, la répression se transmet de génération en génération et permettrait donc de réprimer un gène de façon prolongée voire définitive.

L'invention a donc pour objet un oligonucléotide double brin pour être utilisé dans un processus d'interférence ARN (RNAi) caractérisé en ce qu'il est constitué de deux séquences oligonucléotidiques complémentaires comprenant chacune à l'une de leurs extrémités 3' ou 5' un à cinq nucléotides non appariés formant des bouts simples brins débordant de l'hybride, l'une desdites séquences oligonucléotidiques étant substantiellement complémentaire d'une séquence cible appartenant à une molécule d'ADN ou d'ARN cible que l'on souhaite réprimer spécifiquement. Cet ADN ou ARN peut être de toute nature, il peut s'agir par exemple d'ARN messager ou ribosomique ou encore de préférence d'un gène.

Avantageusement, chacune des deux séquences oligonucléotidiques complémentaires comprend à la même extrémité 3' ou 5' un à cinq nucléotides non appariés formant des bouts simples brins débordant de l'hybride.

Avantageusement les deux séquences oligonucléotidiques ont la même taille.

Du fait de la loi d'appariement des bases, on désignera aussi indistinctement ci-après par oligonucléotide de l'invention, l'une ou l'autre des séquences de l'oligonucléotide double brin de l'invention qui est complémentaire d'une séquence cible appartenant à une molécule d'ADN ou d'ARN que l'on souhaite réprimer spécifiquement et qui peut donc être aussi simple ou double brin(s).

Les oligonucléotides de l'invention peuvent être de nature ribonucléotidique, désoxy ribonucléotidique ou mixte. On préfère toutefois que l'oligonucléotide complémentaire de la séquence cible, aussi désigné brin antisens, soit majoritairement de nature ribonucléotidique. Le brin sens peut être de nature ribonucléotidique désoxy ribonucléotidique ou mixte. Des exemples d'oligonucléotides de l'invention de type ARN/ARN ou ADN/ARN sont donnés dans la partie expérimentale ci-après.

En effet, les hybrides ARN/ARN sont plus stables que les hybrides ADN/ADN ou ADN/ARN et beaucoup plus stables que les acides nucléiques simples brins utilisés dans des stratégies anti-sens.

On entend aussi par oligonucléotide, un polynucléotide de 2 à 100, et plus généralement de 5 à 50, nucléotides de type ribo-, désoxyribo- ou mixte.

La partie de la séquence oligonucléotidique qui est hybridée et complémentaire de la séquence cible a de préférence une taille comprise entre 15 et 25 nucléotides et tout préférentiellement de 20 à 23 nucléotides.

Les oligonucléotides doubles brins de l'invention comprennent, de préférence à l'extrémité 3' de chaque brin, de 1 à 5 nucléotides de préférence de 2 à 3 et tout préférentiellement 2 nucléotides débordant de l'hybride. Ces nucléotides débordant de l'hybride peuvent être ou non complémentaire de la séquence cible. Ainsi, dans une forme de réalisation particulière de l'invention, les nucléotides débordant de l'hybride sont des nucléotides quelconque par exemple des Thymines.

On peut représenter un oligonucléotides double brin de l'invention de la façon suivante, où chaque tiret correspond à un nucléotide et où chaque brin comprend à son extrémité 3' deux thymines débordant de l'hydride :
5'--------TT3'
3'TT-------5'

La séquence des oligonucléotides de l'invention est substantiellement complémentaire d'une séquence cible appartenant à une molécule d'ADN ou d'ARN messager d'un gène que l'on souhaite réprimer spécifiquement. Bien que l'on préfère des oligonucléotides parfaitement complémentaires de la séquence cible, on entend par substantiellement complémentaire, le fait que la séquence oligonucléotidique peut comprendre quelques nucléotides mutés par rapport à la séquence cible dès lors que les propriétés de répression du gène visé ne sont pas altérées. Ainsi, une séquence oligonucléotidique de l'invention peut comprendre de 1 à 3 nucléotides mutés.

Ces nucléotides mutés peuvent donc être ceux débordant de l'hybride ou des nucléotides à l'intérieur de la séquence oligonucléotide.

Ainsi un oligonucléotide de l'invention peut être un hybride parfait ou comprendre un ou plusieurs mismatch au sein du double brin. On préfère toutefois que la partie de la séquence oligonucléotidique qui est hybridée soit parfaitement complémentaire de la séquence cible alors que les nucléotides débordant de l'hybride peuvent être quelconques et notamment des thymines. On entend ainsi également par parfaitement complémentaire le fait que l'oligonucléotide de l'invention soit complémentaire d'une séquence qui appartient à un ADN ou ARN d'un gène muté. Les oligonucléotides de l'invention peuvent permettre ainsi de discriminer entre la séquence du gène sauvage et du gène muté ce qui peut présenter un intérêt particulier tant dans l'analyse des gènes que dans les utilisations thérapeutiques des oligonucléotides de l'invention.

Les oligonucléotides de l'invention sont généralement constitués de bases nucléotidiques naturelles (A, T, G, C, U), mais peuvent aussi comprendre des nucléotides modifiés ou des nucléotides portant des groupements réactifs ou des agents de pontage ou agents intercalant pouvant réagir avec la séquence cible complémentaire à l'oligonucléotide.

Les oligonucléotides de l'invention peuvent être préparés par les méthodes conventionnelles de synthèse chimique ou biologique des oligonucléotides.

L'invention envisage aussi les oligonucléotides couplés à des substances favorisant ou permettant leur pénétration, le ciblage ou l'adressage dans les cellules, il peut s'agit de lipides, de protéines, polypeptides ou peptides ou de toute autre substance naturelle ou synthétique. En effet, les oligonucléotides de l'invention sont destinés à être internalisés dans les cellules et avantageusement dans certains cas, jusque dans le noyau des cellules, où ils vont interagir avec des molécules d'acide nucléiques portant la séquence cible de l'oligonucléotide. De même, il peut être intéressant de favoriser leur pénétration dans un tissu particulier comme une tumeur, l'os, etc.

Les oligonucléotides de l'invention sont utiles pour réprimer de manière très efficace et très spécifique un gène ou un ensemble de gènes et donc pour le traitement de nombreuses pathologies humaines. Ils constituent aussi un outil de recherche pour l'investigation et la compréhension de la fonction de gènes. L'invention à donc pour objet des compositions pharmaceutiques comprenant un oligonucléotide ou un ensemble de nucléotides différents et l'utilisation de ces oligonucléotides, seuls ou couplés à des substances de transport, comme médicament.

Les oligonucléotides de l'invention peuvent être mis en oeuvre dans des applications *ex vivo* par exemple lors de greffe. Ainsi, les oligonucléotides peuvent être soit transfectés dans des cellules, notamment tumorales, qui seront ensuite injectées soit injectés dans les tissus par exemple des tumeurs déjà développées, par exemple par voie locale, systémique ou aérosols etc, avec agents de vectorisation éventuellement nécessaires.

Les oligonucléotides seront utilisés à des concentrations suffisantes en fonction de l'application et de la forme d'administration utilisée avec des excipients pharmaceutiques appropriés. Suivant la nature des oligonucléotides (ADN/ARN ou ARN/ARN) des doses différentes pourront être utilisées pour obtenir l'effet biologique recherché.

Les oligonucléotides de l'invention sont également utiles comme outils de diagnostic permettant d'établir in vitro le profil génétique d'un patient à partir d'un échantillon cellulaire de celui-ci. La mise en oeuvre des oligonucléotides de l'invention dans un tel procédé d'analyse permet de connaître ou d'anticiper la réponse des cellules cancéreuses de ce patient et d'établir un traitement personnalisé ou encore d'ajuster le traitement d'un patient.

Les oligonucléotides de l'invention présentent plusieurs avantages par rapport aux agents chimiothérapeutiques classiques :
- Les hybrides ARN-ARN sont plus stables que les hybrides ADN-ADN ou ADN-ARN et beaucoup plus stables que les acides nucléiques simples brins utilisés dans des stratégies anti-sens.
- Ils constituent des composés naturels, aucune réaction immunologique ou d'intolérance médicamenteuse n'est a priori à craindre.
- Les expériences de transfections réalisées dans le cadre de l'invention montrent une bien meilleure pénétration des RNAi dans les cellules tumorales que celle obtenue avec des plasmides. Ce point est essentiel dans le cas de cellules tumorales qui sont généralement très difficiles à transfecter.
- Les expériences d'injection systémique de siRNA *in vivo* montrent une très bonne pénétration de ces molécules dans les tissus.
- Il est aisé de mélanger plusieurs RNAi entre eux afin de prendre pour cibles plusieurs gènes cellulaires en même temps.

Les oligonucléotides de l'invention et les compositions les contenant sont utiles pour le traitement ou la prévention des maladies infectieuses ou virales, en particulier le SIDA, les maladies infectieuses non conventionnelles, en particulier ESB et Kreutzfeld Jacob. Ils sont tout particulièrement indiqués pour traiter des maladies virales à l'origine de cancers. Le tableau ci-dessous rapporte des exemples de virus impliqués dans des pathologies cancéreuses chez l'homme.

**Tableau 1**

| Virus | Type de cancer humain associé |
|---|---|
| Virus de l'hépatite B (VHB) | Carcinome du foie |
| Virus d'Epstein-Barr (EBV) | Lymphome de Burkitt, cancer nasopharyngé, maladie de Hodgkin, lymphomes non hodgkiniena, cancer gastrique, cancer du sein. |
| Herpèsvirus humain 8 ou HHV-8/KSHV | Sarcome de Kaposi (SK), lymphomes primitif des séreuses (PEL), maladie de Castelman multifocale (MCD) |
| VPH | Col de l'utérus, tête, cou, peau, nasopharynx |
| Virus des lymphocytes T (HTLV) | Leucémie de type T |
| Virus de l'hépatite C (VHC) | Carcinome du foie |

Les oligonucléotides de l'invention et les compositions les contenant sont encore utiles pour le traitement ou la prévention des maladies liées à une hypervascularisation comme la dégénérescence maculaire liée à l'age, l'angiogénèse tumorale, les rétinopathies diabétiques, le psoriasis, l'arthrite rhumatoïde.

Les travaux de recherche réalisés dans le cadre de l'invention ont permis de montrer que ces oligonucléotides sont particulièrement adaptés pour réprimer des gènes nocifs impliqués dans la cancérisation et sont donc tout particulièrement utiles pour le traitement ou la prévention des cancers et plus généralement des maladies oncologiques.

Un traitement anti-cancéreux idéal doit entraîner la mort de la cellule tumorale tout en évitant les phénomènes de résistance. La mort cellulaire peut être obtenue par :
- Inhibition de la division cellulaire, blocage du cycle cellulaire,
- Induction de l'apoptose des cellules tumorales,
- Induction de la sénescence,
- Induction de la nécrose,
- Induction de la différenciation. Dans ce cas, les traitements conduisent la cellule à redevenir normale.

Ainsi, l'invention s'intéresse tout particulièrement à un oligonucléotide ou un ensemble d'oligonucléotides différents, comportant chacun une séquence oligonucléotidique complémentaire d'une séquence cible appartenant à une molécule d'ADN ou d'ARN messager d'un gène dont la répression induit l'apoptose, ou la sénescence, ou la nécrose, ou la différenciation des cellules tumorales ou empêche leur division ou plusieurs de ces phénomènes.

L'induction de l'apoptose des cellules tumorales est basée sur le fait que la fonction de nombreux gènes cellulaires (par exemple membres de la famille BCL2, BCL XL) est de protéger les cellules de l'apoptose. La perte d'expression de ces gènes induite par RNAi permet donc le passage en apoptose.

La mort cellulaire peut également être provoquée par la perte d'adhésion des cellules à la matrice (anoïkis). Cet effet peut être obtenue en perturbant la balance entre protéases et inhibiteurs de protéases dans les tumeurs et leur environnement stromal. Cette perturbation a par ailleurs pour effet de diminuer les capacités des cellules tumorales à envahir les tissus sains et à se métastaser. Les siRNA peuvent donc être utilisés pour empêcher la synthèse de protéines des familles des métallo protéases matricielles (MMP), des métallo protéases matricielles membranaires, de leurs inhibiteurs (TIMPs), ainsi que celle des activateurs des inhibiteurs des protéases comme par exemple PAI-1 et des protéases elles-mêmes comme par exemple l'urokinase.

L'induction de la sénescence repose sur le fait que les cellules normales ne peuvent se diviser qu'un nombre limité de fois. Ce nombre est programmé, environ 50 divisions par exemple pour des fibroblastes embryonnaires, et "mesuré" par la longueur des télomères qui se raccourcit au fur et à mesure des divisions cellulaires. En deçà d'une certaine taille, les télomères ne sont plus fonctionnels et la cellule, incapable de se diviser, entre en sénescence. Dans les cellules germinales cependant, cette longueur est maintenue constante par l'action d'une enzyme, la télomérase. La télomérase est ré-exprimée dans de nombreux cancers, ce qui permet aux cellules tumorales de se multiplier indéfiniment. Un RNAi bloquant l'expression de la télomérase serait sans conséquence sur les cellules somatiques normales et devrait conduire les cellules tumorales vers la sénescence.

Le blocage de la division cellulaire conduit également les cellules à la sénescence. Ce blocage peut être obtenu en inhibant des récepteurs cellulaires essentiels. Ces récepteurs peuvent appartenir suivant la nature de la cellule soit à la classe des récepteurs des facteurs de croissance (EGF, SST2, PDGF, FGF notamment), que ceux-ci soient ou non mutés, soit à celle des récepteurs nucléaires d'hormones (androgènes, oestrogènes, glucocorticoïdes notamment).

Les récepteurs d'hormones sont fréquemment mutés dans les cancers, et l'invention concerne dans ce cas l'utilisation d'oligonucléotides reconnaissant les formes mutées de ces récepteurs et qui n'inhibent pas la synthèse des formes sauvages. Ceci permet par exemple dans le cas des carcinomes prostatiques devenus résistants par mutation du récepteur des androgènes de traiter par voie systémique les patients avec des siRNA qui bloquent la synthèse du récepteur muté sans induire d'effets de castration liés à l'inhibition des formes sauvage du récepteur dans d'autres organes. Un exemple d'utilisation d'oligonucléotides reconnaissant des formes mutées du récepteur est présenté.

Le cycle cellulaire peut également être arrêté en inhibant la synthèse de protéines indispensables à son déroulement comme par exemple les cyclines, kinases dépendantes des cyclines, enzymes de réplication de l'ADN, facteurs de transcription tels que E2F.

L'induction de la nécrose résulte du besoin des cellules tumorales en oxygène et en nutriments. Initialement une tumeur assure son développement à partir des vaisseaux préexistants de l'hôte. Au-delà de 1 à 2 mm de diamètre, les cellules situées au centre de la tumeur se trouvent en hypoxie. Cette hypoxie, par l'intermédiaire d'une proline hydroxylase, entraîne la stabilisation du facteur de transcription Hifla, dont la séquence SEQ ID NO. 59 est donnée en annexe, qui, en se fixant sur des séquences HRE dans les promoteurs de ses gènes cibles, déclenche la réaction hypoxique. Cette réaction conduit à l'activation d'une centaine de gènes permettant d'activer notamment la voie de la glycolyse anaérobie, la réponse au stress et l'angiogénèse. Ce dernier mécanisme active en particulier le gène du VEGF, dont la séquence SEQ ID NO. 60 est donnée en annexe, principal facteur angiogénique tumoral.

Ainsi des oligonucléotides selon l'invention bloquant par exemple l'expression du facteur de transcription Hif1α ou par exemple celle du VEGF mettent les cellules tumorales dans l'incapacité à monter une réponse hypoxique ou angiogénique. L'angiogénèse est un mécanisme normalement réprimé chez l'adulte à l'exception du cycle menstruel (utérus ovaires). L'inhibition de ce mécanisme a donc peu de conséquences pour les tissus normaux.

En conséquence, l'invention se rapporte à un oligonucléotide dont l'une desdites séquences oligonucléotidiques est substantiellement complémentaire d'une séquence cible appartenant à une molécule d'ADN ou d'ARN messager du gène codant :
- le facteur de transcription Hif1α;
- une ou plusieurs des isoformes du VEGF A ou d'un membre de la famille de ce facteur de croissance.

Dans certains cancers, le phénotype tumoral résulte de, ou est maintenu par, l'expression d'une protéine normalement absente des cellules normales. Cette protéine peut résulter de l'expression actuelle ou ancienne d'un génome viral dans la cellule comme celui du virus du papillome, HPV, ou du virus de l'hépatite B. Cette protéine peut également résulter de la mutation (ponctuelle, délétion, insertion) d'un gène cellulaire normal. Dans ce cas, il est fréquent que la protéine mutée ainsi produite possède des propriétés transdominantes négatives par rapport à la protéine normale. La spécificité des siRNA permet d'inhiber la synthèse de la protéine mutante sans bloquer la synthèse des protéines sauvages. Deux exemples concernant des formes mutées de la protéine p53 et du récepteur des androgènes sont rapportés dans la partie expérimentale ci-après.

Les travaux de recherche réalisés dans le cadre de l'invention ont permis de montrer que ces oligonucléotides sont particulièrement adaptés pour réprimer des gènes nocifs impliqués dans la cancérisation et tout particulièrement à ceux conduisant à la formation de protéine de fusion dans les cellules cancéreuses, comme la protéine de fusion PML-RAR alfa.

En conséquence, l'invention se rapporte tout particulièrement à des oligonucléotides dont la séquence est complémentaire d'une séquence cible appartenant à un gène résultant d'une translocation chromosomique de façon à inhiber les effets de la protéine de fusion exprimée par ce gène. Ainsi, la séquence cible est celle correspondant à la séquence de la jonction de la protéine de fusion.

Le tableau 2 de l'annexe A à la fin de la présente description est une liste non exhaustive des protéines de fusion représentant des cibles thérapeutiques ou diagnostiques pour les oligonucléotides de l'invention.

Le fait de cibler avec un oligonucléotide de l'invention, la jonction entre deux gènes, par exemple les deux gènes *pml* et *rara,* permet d'aboutir à l'inhibition spécifique de la protéine de fusion sans affecter le rôle biologique des protéines naturelles qui peuvent être codées par le second allèle. Cette forme de mise en oeuvre de l'invention englobe donc toutes les protéines de fusion impliquées dans la cancérogénèse, particulièrement les leucémies. Les formes réciproques si elles existent, ainsi que tous les variants des protéines de fusion citées en annexe constituent également des cibles de l'invention. L'invention concerne donc tout particulièrement, l'utilisation des oligonucléotides comme défini ci-dessus pour la préparation d'une composition pharmaceutique destinée au traitement des maladies résultant de l'expression d'une protéine de fusion, tout particulièrement dans les cancers.

Les thérapies anti-cancéreuses actuelles prennent pour cible les cellules cancéreuses, par différentes approches, prises isolément ou combinées entre elles (chimiothérapie, chirurgie, radiothérapie, immunothérapie). Les échecs thérapeutiques sont massivement dus soit à des cellules n'ayant pas été atteintes par le traitement soit, et majoritairement, à des cellules ayant muté en réponse au traitement. Cette capacité de mutation est grandement facilitée par l'instabilité génétique des cellules tumorales. L'inhibition de la vascularisation tumorale, privant les cellules d'oxygène et de nutriments, a depuis quelques années ouvert de nouvelles perspectives thérapeutiques en cancérologie. Cette stratégie, complémentaire des précédentes, prend pour cible la cellule endothéliale normale de l'hôte, génétiquement stable, et donc théoriquement peu susceptible de muter. De nombreux essais cliniques visant à inhiber l'angiogénèse tumorale par différentes approches sont en cours dans le monde. Cependant, les premiers résultats rapportés semblent assez décevants.

Les Inventeurs ont démontré que des tumeurs sont capables de compenser les effets d'inhibiteurs de l'angiogénèse, en sélectionnant des sous-populations de cellules sécrétant de fortes concentrations de facteurs pro angiogéniques.

Les tumeurs ne sont pas constituées de cellules homogènes quant à leur expression génique. Ceci est attesté par de très nombreuses études dans lesquelles des immunomarquages ont été réalisés pour une grande variété d'antigènes dans les tumeurs. Macroscopiquement, une tumeur est fréquemment composée de régions hautement vascularisées côtoyant des zones de nécrose ou au contraire avasculaires.

Cette hétérogénéité tumorale favorise l'échappement des tumeurs aux traitements appliqués, quelle qu'en soit la nature. Plus la diversité de l'expression génique dans une tumeur est grande plus la probabilité qu'il existe au moins une cellule capable de résister à un agent anti-tumoral est en effet élevée. Il apparaît dès lors essentiel d'associer différentes stratégies afin de réduire tout d'abord l'hétérogénéité tumorale et d'éviter les phénomènes d'échappement.

L'invention s'intéresse tout particulièrement à des siRNAs inhibiteurs de l'expression de gènes responsables de l'inactivation de la p53 et à leur utilisation dans le traitement des cancers. La p53 est le produit d'un gène suppresseur de tumeurs ou anti-oncogène, muté dans plus de 50% des tumeurs chez l'homme. La p53 est ainsi considérée comme un « gardien du génome ». Elle est activée dans les cellules en cas de stress génotoxique et participe à divers processus dont l'induction du processus de mort programmée.

Dans 74% des cas de mutation monoallélique, l'inactivation de la p53 est due à une mutation ponctuelle aboutissant à l'expression d'une protéine de taille normale, mais mutée. On considère généralement que la forme mutée forme des hétéromères avec le produit de l'allèle sauvage sur lequel elle agit comme un « transdominant négatif » et bloque son activité. La forme mutante semble également avoir une activité oncogénique en elle-même. Ainsi, des formes mutées de la p53 sont capables d'activer le gène MDR, qui facilite la résistance des cellules cancéreuses aux chimiothérapies. De plus, l'expression de mutants de la p53 est associée à une plus forte angiogenèse tumorale, sans doute en raison du fait que les formes mutantes de la p53 ne sont plus capables de stimuler la transcription du gène de la thrombospondine, l'un des plus puissants répresseurs de l'angiogénèse, et activent le VEGF et le bFGF, deux puissants activateurs de l'angiogénèse. De plus, les cellules dans lesquelles une forme mutée de la p53 s'exprime perdent divers niveaux de régulation. En particulier elles ne sont plus capables d'entamer un processus de mort programmée, qui constitue l'un des processus majeurs de protection contre la tumorigenèse. La restauration d'une activité p53 sauvage entraîne, dans des cellules tumorales en culture, la restauration de cette réponse cellulaire. Ainsi, l'inhibition de l'expression des formes mutées de la p53 représente potentiellement un outil puissant en thérapie anti-cancéreuse.

Il n'y a, à l'heure actuelle, aucun moyen efficace de restaurer une activité p53 dans les cellules cancéreuses humaines. En ce qui concerne les cancers dans lesquels les deux allèles sont inactivés, des tentatives de restauration de l'activité p53 par thérapie génique sont envisagées. Ces approches sont compliquées par l'utilisation de vecteurs viraux et se montrent pour le moment peu efficaces.

Par ailleurs, il a été observé spécifiquement dans les cancers cervicaux liés à l'infection par le virus HPV des cellules du col de l'utérus, que la p53 peut-être inactivée par la surexpression d'une protéine virale. En effet, ce virus code pour une protéine, la protéine E6, qui inactive la p53. Dans ce type de cancers, c'est l'inhibition de la protéine E6 qui pourra restaurer une activité p53 sauvage.

L'invention vise à offrir de nouveaux moyens permettant d'activer la p53 en inhibant l'expression de gènes responsables de son inactivation. Les travaux de recherche réalisés dans le cadre de la présente invention ont permis de mettre en évidence qu'il était ainsi possible de réprimer de manière très efficace et très spécifique l'expression d'une forme mutante de la p53.

L'invention concerne des oligonucléotides présentant une séquence complémentaire d'une séquence polynucléotidique spécifique du gène de la p53 mutée. Il s'agit donc d'oligonucléotides dont la séquence porte une mutation par rapport à la séquence de la p53 sauvage. La séquence du gène sauvage de la p53 est indiquée dans la liste de séquences en annexe sous le numéro SEQ ID NO.1. Les différentes mutations pouvant intervenir sur la séquence de la p53 sont indiquées dans le tableau 3 de l'annexe B à la fin de la présente description.

Les mutations les plus fréquemment observées dans les pathologies cancéreuses sont reportées dans le tableau 4 ci-après.

**Tableau 4**

| Position | P53 sauvage | SEQ ID No. |
|---|---|---|
| R273H | GAGGTGCGTGTTTGTGC | SEQ ID No. 61 |
| R248Q | gcaTgaaccggaggcccaT | SEQ ID No. 62 |
| R248W | gcaTgaaccggaggcccaT | SEQ ID No. 63 |
| R249S | gcaTgaaccggaggcccaT | SEQ ID No. 64 |
| G245S | CTGCATGGGCGGCATGAAC | SEQ ID No. 65 |
| R282W | TGGGAGAGACCGGCGCACA | SEQ ID No. 66 |
| R175H | TGTGAGGCACTGCCCCCAC | SEQ ID No. 67 |
| C242S | TAACAGTTCCTGCATGGGCG | SEQ ID No. 68 |
| Position | P53 mutée | |
| R273H | GAGGTGCATGTTTGTGC | SEQ ID No. 69 |
| R248Q | gcaTgaacCAgaggcccaT | SEQ ID No. 70 |
| R248W | GCATGAACTGGAGGC CAT | SEQ ID No. 71 |
| R249S | gcaTgaaccggagTcccaT | SEQ ID No. 72 |
| G245S | CTGCATGGGCAGCATGAAC | SEQ ID No. 73 |
| R282W | TGGGAGAGACTGGCGCACA | SEQ ID No. 74 |
| R175H | TGTGAGGCGCTGCCCCCAC | SEQ ID No. 75 |
| C242S | TAACAGTTCCTCCATGGGCG | SEQ ID No. 76 |

Ainsi, des oligonucléotides selon l'invention sont complémentaires d'une séquence cible appartenant au gène de la p53 muté portant l'une au moins des mutations données dans le tableau 3 et tout particulièrement l'une au moins des mutations du tableau 4 ci-dessus.

Ces oligonucléotides sont capables de discriminer de manière efficace entre la forme sauvage et la forme mutée de la p53. En effet, la stratégie est de bloquer l'expression de la forme mutée pour réactiver la forme sauvage et induire dans les cellules un processus de mort programmée pour lequel la forme sauvage est indispensable, et/ou bloquer tout autre processus induit par la forme mutée de la p53. En outre, cette capacité de discrimination des oligonucléotides de l'invention permet de ne toucher que les cellules cancéreuses et d'épargner les cellules normales, qui n'expriment pas cette forme mutée de la p53.

L'invention a donc aussi pour objet le traitement ou la prévention des maladies induites par une inactivation de la protéine p53 et tout particulièrement les cancers résultant de l'expression de la protéine p53 mutée et les cancers résultant de l'expression de gènes inhibiteurs de la p53. L'invention a encore pour objet de prévenir l'apparition de cancers chez les sujets exprimant une forme mutée de la p53, comme dans le cas du syndrome de Li Fraumeni.

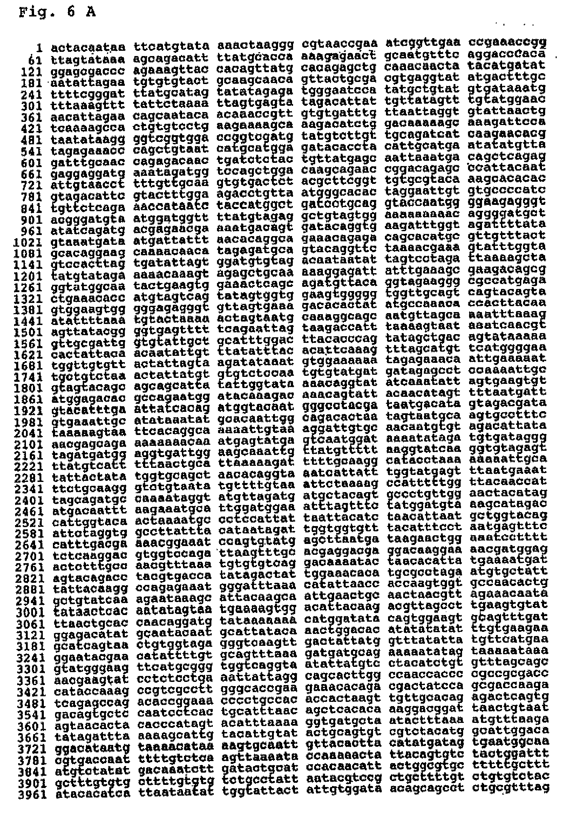

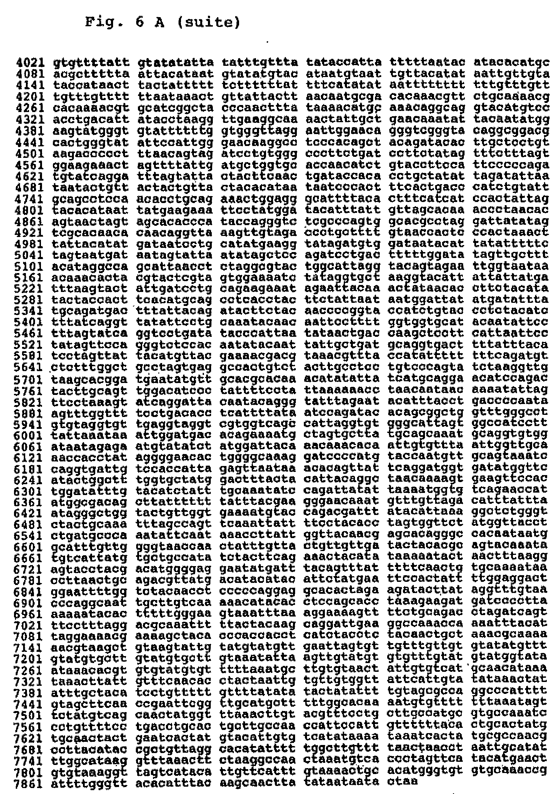

La P53 peut être inactivée à travers plusieurs mécanismes distincts. En particulier, dans la majorité des cancers cervicaux, la P53 est inactivée par une protéine codée par le virus du papillome humain, la protéine E6. E6 entraîne l'ubiquitinylation de la P53 ce qui conduit à sa dégradation par le protéasome. Dans ce cas, l'expression de la P53 peut être restaurée par l'inhibition de l'expression de la protéine E6. L'invention concerne également des oligonucléotides présentant une séquence complémentaire d'une séquence polynucléotidique spécifique du gène de la protéine E6 de HPV. La séquence du gène de la protéine E6 de HPV est donnée à la figure 6A ainsi que dans la liste de séquences en annexe sous le numéro SEQ ID NO 2.

Comme indiqué précédemment, une stratégie selon l'invention a pour but de bloquer à l'aide de RNAi l'expression du récepteur des androgènes dans les carcinomes. La séquence du récepteur des androgènes est donnée dans la liste de séquence en annexe sous le numéro SEQ ID NO. 77. Pour traiter les carcinomes avant qu'ils ne soient devenus résistants ou ceux qui le sont devenus par amplification du récepteur sans mutation, des siRNA homologues d'une région pour laquelle aucune mutation n'a été décrite dans les banques de données des mutations du récepteur des androgènes (notés siRNA AR) ont été utilisés. Pour traiter spécifiquement les carcinomes prostatiques devenus androgéno résistants par mutation, un séquençage de l'ARNm codant pour le récepteur sera effectué dans les cellules du patient afin de concevoir une séquence spécifique de la mutation, permettant de traiter le patient sans conséquence pour les cellules normales. Un exemple est présenté par l'utilisation de siRNA reconnaissant spécifiquement la mutation du récepteur des androgènes présente dans la lignée cellulaire LNCaP (siRNA LNCaP).

En conséquence, l'invention se rapporte à des oligonucléotides substantiellement complémentaires d'une séquence cible appartenant à une molécule d'ADN ou d'ARN messager codant le récepteur aux androgènes muté ou non muté. Il s'agit par exemple du récepteur aux androgènes portant l'une au moins des mutations données dans le tableau 5 de l'annexe C. Ces oligonucléotides de l'invention spécifiques du récepteur aux androgènes sont utiles pour traiter ou prévenir les maladies androgéno-dépendantes, comme par exemple le cancer de la prostate.

D'autres avantages et caractéristiques de l'invention apparaîtront des exemples qui suivent concernant :
- Exemple 1 : Inhibition de la protéine PML-RARα associée à la leucémie aiguë promyélocytaire (APL).
- Exemple 2 : Inhibition de l'angiogénèse tumorale induite par le VEGF.
- Exemple 3 : Inhibition de la réponse hypoxique induite par HIF1 α.
- Exemple 4 : Inhibition des formes sauvages ou mutantes des récepteurs des androgènes dans les cellules de carcinome prostatique.
- Exemple 5 : Inhibition des formes sauvages ou mutantes de la protéine p53.
- Exemple 6 : Inhibition de la protéine virale E6.
- Exemple 7 : Utilisation des hybrides ADN/ARN pour inhiber l'expression de différentes protéines.
- Exemple 8 : Administration *in vivo* de siRNA par différentes voies.

Il est fait référence dans les exemples aux figures dans lesquelles :

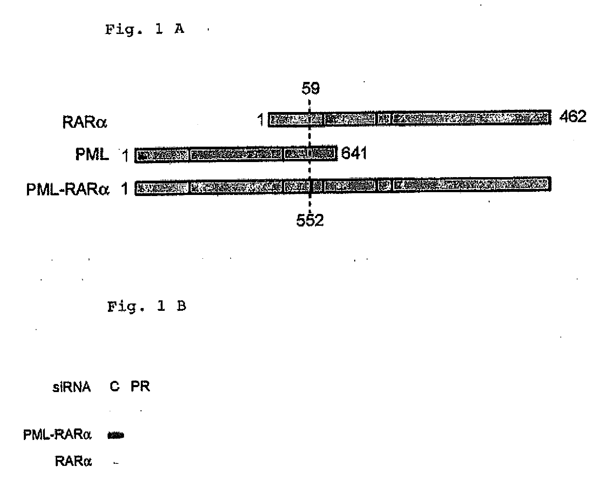

- La figure 1A est une représentation schématique des protéines RARα, PML et de la protéine de fusion associée, PML-RARα. La figure 1B représente les résultats de transfections avec un siRNA dirigé contre PML-RARα.

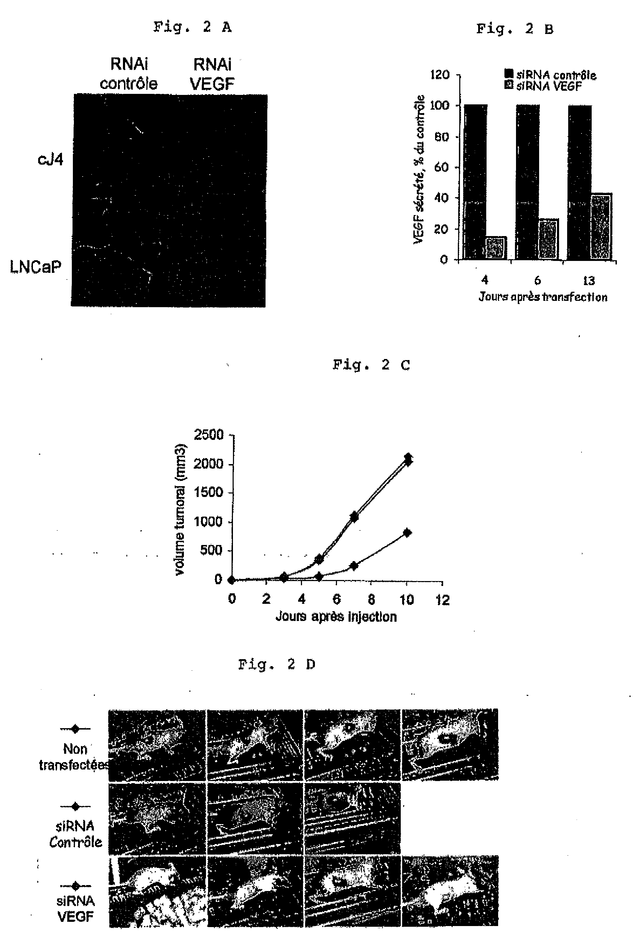

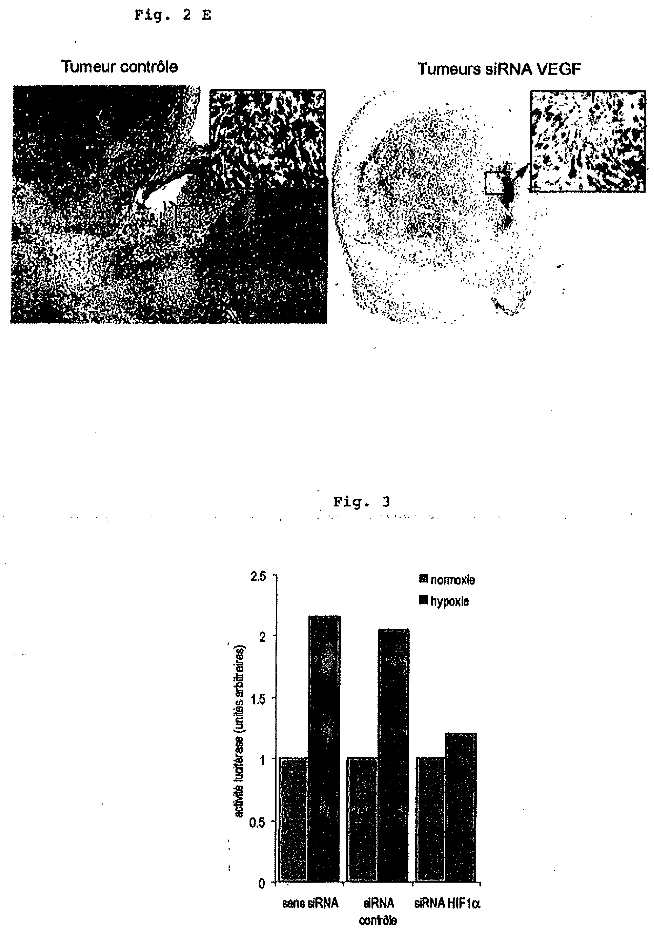

- La figure 2 concerne l'inhibition de l'expression du VEGF par des siRNA dirigés contre cette protéine et les conséquences de cette inhibition. La figure 2A représente l'immunodétection du VEGF dans des cellules cJ4 ou LNCaP transfectées par le siRNA contrôle ou un siRNA dirigé contre le VEGF. La figure 2B représente la quantification par ELISA du VEGF dans le milieu conditionné des cellules cj4 transfectées par le siRNA contrôle ou le siRNA VEGF en fonction du temps après la transfection. La figure 2C représente la courbe de croissance chez des souris nudes de tumeurs provenant de l'injection sous cutanée de 10⁶ cellules cJ4 non transfectées, transfectées par le siRNA contrôle ou le siRNA VEGF. La figure 2D représente l'aspect des tumeurs au jour 7 après injection des cellules. La figure 2E représente l'immunodétection du VEGF dans des tumeurs provenant de l'injection de cellules cj4 transfectées avec le siRNA contrôle ou le siRNA VEGF après 12 jours de développement in vivo.
- La figure 3 concerne l'effet de l'inhibition par un siRNA spécifique de l'expression d'un facteur de transcription, HIF1alpha, sur la réponse transcriptionnelle à l'hypoxie. La figure représente la mesure de l'activité d'un reporter VEGF luciférase en réponse à l'hypoxie dans des cellules cJ4 non transfectées, transfectées par le siRNA contrôle ou par le siRNA dirigé contre HIF1alpha.

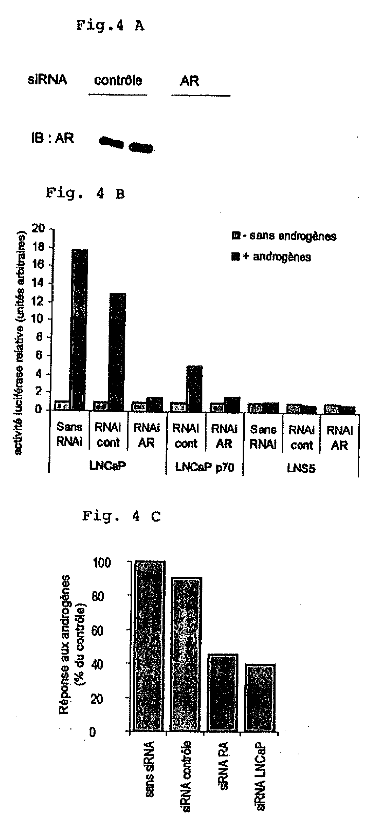

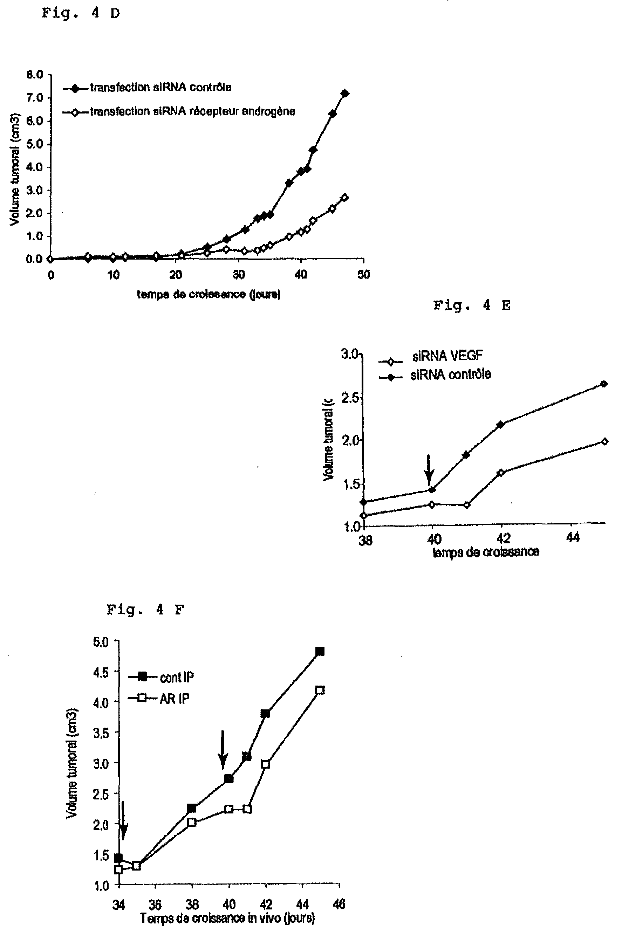

- La figure 4 concerne l'inhibition par des siRNA spécifiques de l'expression du récepteur des androgènes dans des cellules et les conséquences fonctionnelles de ces inhibitions. La figure 4A représente la détection par immunoblot de l'expression du récepteur des androgènes 48h après transfection des cellules LNCaP par un siRNA contrôle ou un siRNA dirigé contre le récepteur des androgènes (AR). La figure 4B représente la mesure de l'activité d'un reporter 4xARE luciferase au R1881 dans divers clones de la lignée LNCaP non transfectée, ou transfectée par le siRNA contrôle ou le siRNA AR. La figure 4C représente la comparaison de la réponse au R1881 de cellules LNCaP non transfectées (100%), et des cellules LNCaP transfectées par un siRNA contrôle, un siRNA dirigé contre le récepteur des androgènes (AR) ou un siRNA reconnaissant spécifiquement une mutation ponctuelle présente dans le récepteur des androgènes de la lignée LNCaP. La figure 4D représente la croissance chez des souris nudes de tumeurs résultant de l'injection sous cutanée de cellules LNCaP transfectées par un siRNA contrôle ou par un siRNA dirigé contre le récepteur des androgènes. La figure 4E représente la croissance de tumeurs LNCaP chez des souris ayant reçu au 40e jour après implantation des cellules une injection intraveineuse dans la veine de la queue de 2*µ*g de siRNA dirigés contre le VEGF ou de siRNA contrôle. La figure 4F représente la croissance de tumeurs LNCaP chez des souris ayant reçu au 34e et au 40e jour après implantation des cellules tumorales une injection intrapéritonéale de 2*µ*g de siRNA dirigé contre le récepteur des androgènes ou de siRNA contrôle.

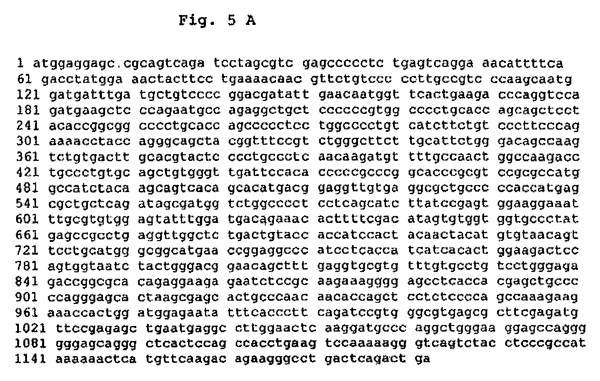

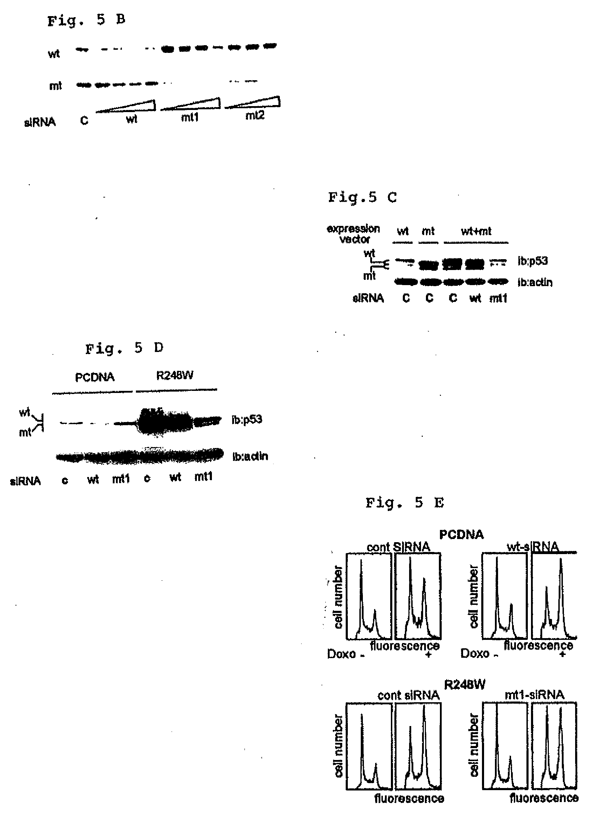

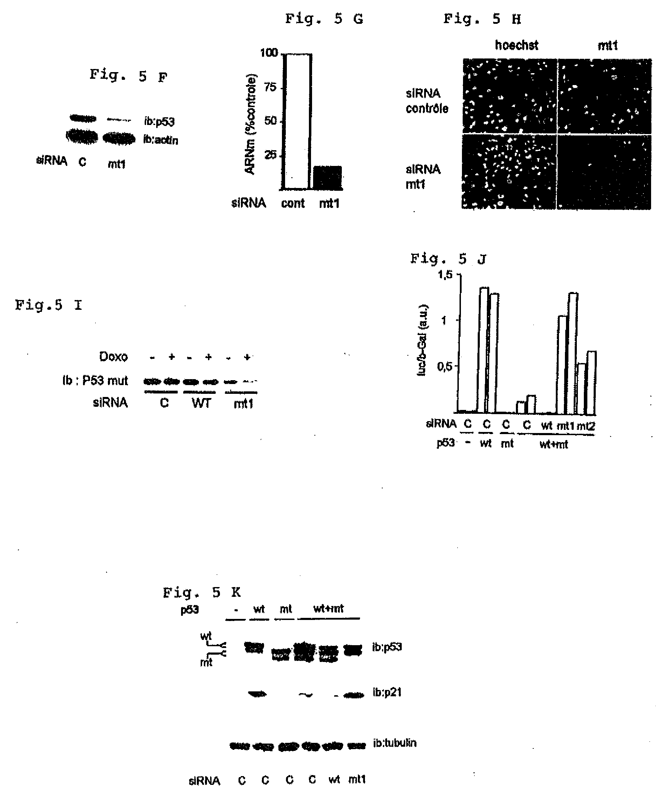

- La figure 5 concerne l'inhibition de l'expression de formes sauvages ou mutantes de la p53 par des siRNA et des conséquences fonctionnelles de ces inhibitions. La figure 5A représente la séquence de protéine p53 humaine. La figure 5B représente l'inhibition spécifique et dépendante de la dose par des siRNA de l'expression de formes sauvages ou mutantes de la p53 transfectées dans des cellules ne l'exprimant pas initialement. La figure 5C représente l'inhibition spécifique par des siRNA de l'expression simultanée ou non de formes sauvages ou mutantes de la p53 transfectées dans des cellules ne l'exprimant pas initialement. La figure 5D représente l'inhibition de l'expression de la p53 endogène sauvage ou d'une forme mutante transfectée de p53 par des siRNA. La figure 5E représente l'effet de l'inhibition de la p53 par des siRNA sur la résistance à un stress génotoxique. Les figures 5F, G, H et I montrent l'inhibition de l'expression d'un forme mutante de la p53 dans les cellules d'un patient atteint du syndrome de Li Fraumeni sur le niveau de l'ARNm (5G), et l'expression de la protéine par immunoblot (GF) ou en immunofluorescence indirecte (5H) et les conséquences sur la résistance de ces cellules à un stress génotoxique. La figure 5J montre l'inhibition par les siRNA spécifiques de la transcription dépendante de la p53 dans des cellules exprimant par transfection des formes sauvages ou mutantes de la p53. La figure 5K montre l'inhibition de l'expression d'un des gènes cibles de la p53, la p21, protéine inhibitrice de la prolifération cellulaire, par la coexpression de formes mutantes de la p53 et la restauration de cette expression par traitement des cellules avec un siRNA inhibant la synthèse de la forme mutante de la p53.

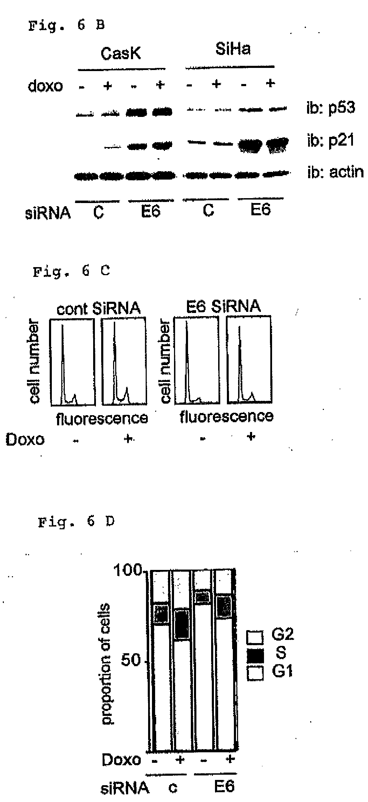

- La figure 6 concerne l'inhibition de l'expression de la protéine E6 du virus du papillome humain HPV par des siRNA spécifiques et les conséquences de cette inhibition. La figure 6A représente la séquence de la protéine HPV. La figure 6B représente l'effet de l'inhibition par des siRNA spécifiques de l'expression de la protéine E6 de HPV dans des cellules qui expriment ce virus, sur l'expression de la p53 et de la p21. Les figures 6C et 6D représentent l'effet de l'inhibition de l'expression de la protéine E6 de HPV sur le cycle cellulaire.

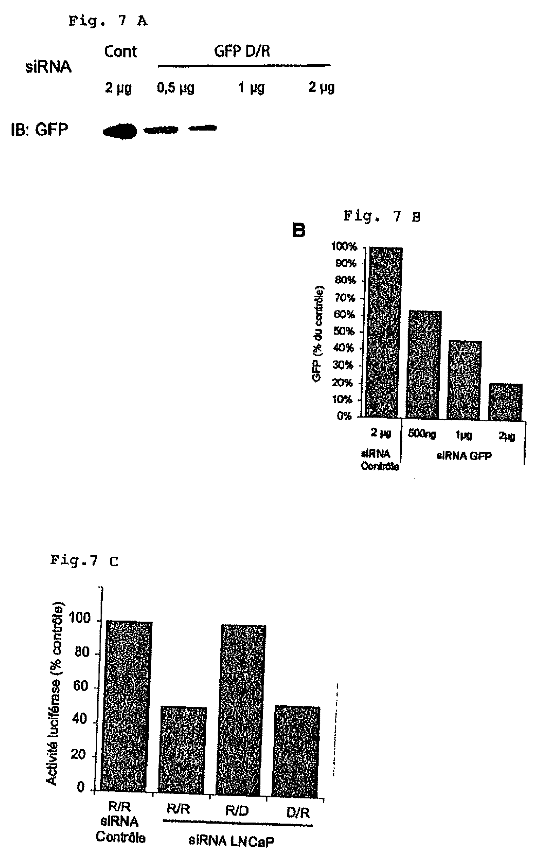

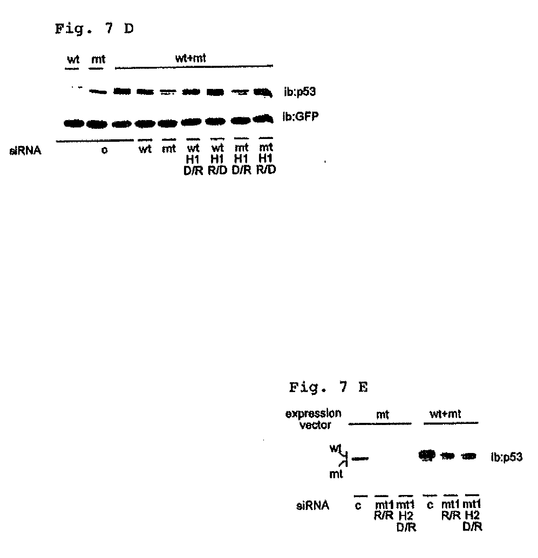

- La figure 7 concerne l'utilisation de siRNA hybrides, comportant des bases ADN et des bases ARN. Les figures 7A et 7B représentent l'effet de siRNA hybrides ADN/ARN sur l'expression de la GFP exprimée par transfection dans des cellules. La figure 7C compare l'effet de siRNA ARN/ARN, ADN/ARN ou ARN/ADN à dose constante sur l'inhibition de la transcription induite par le récepteur des androgènes. Les figures 7D et 7E représentent les effets d'une substitution de bases ARN par des bases ADN dans la séquence de siRNA inhibant la synthèse de la p53.

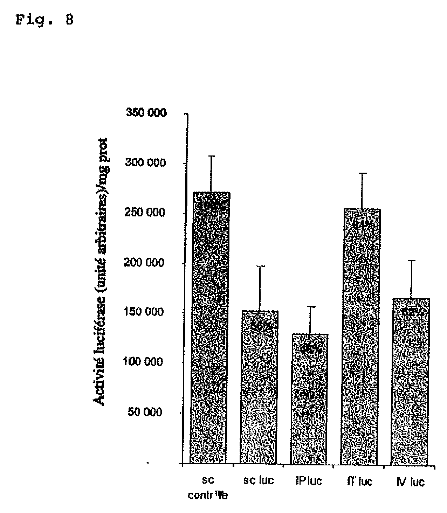

- La figure 8 concerne l'inhibition de la luciférase dans des tumeurs exprimant cette enzyme par injection de siRNA par voie sous cutanée, ou intra-tumorale ou intra-péritonéale ou intra-veineuse

### EXEMPLE 1 : INHIBITION DE LA PROTEINE PML-RARα ASSOCIEE A LA LEUCEMIE AIGUË PROMYELOCYTAIRE (APL).

### I - Introduction.

La leucémie aiguë promyélocytaire (APL) est due à la translocation t(15 ;17) sur le chromosome 15. Chez les patients atteints, le récepteur de l'acide rétinoïque RARα (RARα) est fusionné à la protéine PML (*promyelocytic leukemia protein*) générant ainsi la protéine de fusion PML-RARα. Jusqu'à ce jour, cinq protéines de fusion mettant en jeu le RARα ont été identifiées. Tous ces types de leucémies impliquent le récepteur RARα et sont cliniquement similaires, ce qui suggère que la rupture de la voie de transduction de l'acide rétinoïque est cruciale dans la pathogenèse des leucémies APL.

La protéine de fusion PML-RARα a gardé les domaines de liaison à l'ADN et à l'acide rétinoïque du RARα. Il a été montré que la protéine de fusion PML-RARα réprime l'expression des gènes cibles de l'acide rétinoïque et provoque ainsi le blocage de la différenciation des cellules promyélocytaires. Seule l'administration de doses pharmacologiques d'acide rétinoïque permet la levée de la répression transcriptionnelle exercée par PML-RARα et la reprise de la différenciation cellulaire. En outre, la portion protéique PML de la protéine de fusion pourrait également intervenir dans le mécanisme du blocage de la voie de transduction par l'acide rétinoïque. Dans la mesure où PML fonctionne comme un inhibiteur de croissance et un agent apoptotique et qu'elle est nécessaire pour l'expression de certains gènes induit par l'acide rétinoïque, l'effet dominant négatif de PML-RARα sur PML pourrait permettre aux cellules d'acquérir une capacité de croissance, une résistance à l'apoptose et un arrêt de la différenciation.

Des études de biologie cellulaire sur PML ont montré que cette protéine possède une localisation particulière dans le noyau, dans des structures particulières appelées corps nucléaires. Il semble que le rôle de ces structures soit en relation directe avec le rôle antioncogène de PML. Dans les cellules malignes APL, la protéine PML-RARα provoque, par hétérodimérisation avec PML, la délocalisation de PML des corps nucléaires vers des structures microponctuées pouvant correspondre à des sites d'ancrage de PML-RARα sur la chromatine. Cette délocalisation pourrait bloquer la fonction pro-apoptotique de PML et son rôle dans la différenciation myéloïde. Plusieurs équipes ont montré que le traitement combiné à l'acide rétinoïque et à l'AS₂O₃ sur des lignées cellulaires qui expriment la protéine de fusion PML-RARα permet la dégradation des protéines de fusion en même temps qu'une relocalisation de PML sur les corps nucléaires. Cette réorganisation des corps nucléaires restaure les fonctions de PML et contribue à la reprise de la différenciation.

Finalement, la protéine chimère PML=RARα aurait donc un double effet dominant négatif, sur RARα et sur PML, en permettant à la fois aux cellules d'échapper à l'apoptose et de bloquer la différenciation des promyélocytes ainsi transformés.

Plus de 98% des patients atteints par la leucémie APL présentent la translocation t(15 ;17)(q22 ;q21) qui conduit à la formation de gènes fusionnés PML-RARα et RARα-PML. Il existe deux sous-types de protéines de fusion PML-RARα, les fusions S (short) et L (Long). La forme longue de la protéine de fusion PML-RARα correspondant à une protéine de 955 acides aminés représente la forme majoritairement exprimée et a donc été prise comme modèle dans cette étude (Annexes A, B et C). Cette protéine comporte les acides aminés 1 à 552 de la protéine PML fusionnés avec les acides aminés 59 à 462 du récepteur α de l'acide rétinoïque (RARα).

### II - Préparation et administration des oligonucléotides.

Des oligonucléotides RNA complémentaires correspondant à la séquence de la jonction du gène de la protéine de fusion, soit 10 nucléotides du gène PML et 10 nucléotides du gène RARα ont été synthétisés avec adjonction de deux déoxythymidines en 3' (Figure 1). Ces oligonucléotides ont été hybridés et l'obtention de l'oligonucléotide double-brin a été vérifiée sur gel d'acrylamide.

Les séquences des siRNA PML-RAR et contrôle utilisés (5'-3') sont données ci-dessous.

### Contrôle :

FW :
   [CAUGUCAUGUGUCACAUCUC]ARN[TT]ADN (SEQ ID NO.3)
REV :
   [GAGAUGUGACACAUGACAUG]ARN[TT]ADN (SEQ ID NO.4)
PR :
   Sens :
      [GGGGAGGCAGCCAUUGAGAC]ARN[TT]ADN (SEQ ID NO.5)
   Antisens :
      [GUCUCAAUGGCUGCCUCCCC]ARN[TT]ADN (SEQ ID NO.6)

### III - Résultats.

Des fibrobastes NIH3T3 ont été cotransfectés avec de la lipofectamine par un vecteur d'expression de la protéine PML-RARα (100ng) et par 500ng de siRNA contrôle (C) ou dirigés contre PML-RARα (PR). 48 h après transfection, un western blot (Figure 1B) a été réalisé sur des extraits cellulaires totaux en utilisant un anticorps qui reconnaît la protéine RARα, entière ou sous forme de protéine de fusion.

La figure 1B montre que la transfection du siRNA PR inhibe très fortement l'expression de la protéine de fusion PML-RARα par rapport aux cellules transfectées avec le siRNA contrôle (C), sans modifier l'expression de la protéine RARα.

### EXEMPLE 2 : INHIBITION DE L'ANGIOGENESE TUMORALE PAR LE VEGF.

### I - Introduction.

Le VEGF (vascular endothelial growth factor) est l'un des plus puissants facteurs angiogéniques identifiés. Ces facteurs sont surexprimés dans de nombreuses situations d'hypervascularisation pathologiques et notamment dans le développement tumoral. L'inhibition de cette angiogénèse permet de bloquer la croissance tumorale. Notre procédé a pour but d'inhiber l'angiogénèse tumorale en bloquant l'expression de l'un de ces facteurs angiogéniques et dans cet exemple celui du VEGF par les cellules tumorales.

### II - Préparation et administration des oligonucléotides.

Deux oligonucléotides RNA, complémentaires d'une région de la séquence codante du VEGF humain, conservée chez le rat et la souris ont été synthétisés. Deux déoxynucléotides (TT) ont été ajoutés en 3'
- Séquence des RNAi VEGF :
   5' [AUGUGAAUGCAGACCAAAGAA]RNA-TT[DNA] (SEQ ID NO. 7)
   5' [UUCUUUGGUCUGCAUUCACAU]RNA-TT[DNA] (SEQ ID NO. 8)
- Séquence des RNAi contrôle :
   5' [CAUGUCAUGUGUCACAUCUC]RNA-TT[DNA] (SEQ ID NO. 9)
   5' [GAGAUGUGACACAUGACAUg]RNA-TT[DNA] (SEQ ID NO. 10)

Ces oligonucléotides ou des oligonucléotides contrôle, dont la séquence ne présente aucune homologie avec les séquences répertoriées dans les bases de données, ont été hybridés et transfectés en utilisant le kit polyfect (Qiagen) dans des cellules d'un fibrosarcome de rat (cJ4) ou dans les cellules humaines du carcinome prostatique LNCaP.

### III - Résultats.

48h après transfection, une immunofluorescence indirecte a été réalisée pour détecter l'expression de la protéine dans les cellules. La figure 2A montre une inhibition massive de l'expression du VEGF.

Pour quantifier cet effet, un dosage du VEGF dans des cellules cJ4 transfectées en parallèle avec le RNAi contrôle ou avec le RNAi VEGF a été effectué par ELISA (quantikine, R&D). Les cellules ont été incubées 48h avant le dosage dans un milieu contenant 1% de sérum. Le dosage a été réalisé 4 jours et 6 jours après la transfection. Dans ces conditions, la figure 2B montre une inhibition de la sécrétion de VEGF de 85% à 4 jours et de 75% à 6 jours et de 60% à 13 jours dans les cellules transfectées avec le RNAi VEGF comparé à celles transfectées avec le RNAi contrôle (figure 2B).

L'effet de l'inhibition de l'expression de VEGF par les cellules tumorales a été testé *in vivo* : 3 jours après transfection, trois groupes de 4 souris nudes femelles de 4 semaines ont été injectés en sous cutané à raison de un million de cellules par souris : Le premier groupe a été injecté avec des cellules non transfectées, le second avec des cellules transfectées par le RNAi contrôle, le troisième par des cellules transfectées avec le RNAi VEGF. Aucune sélection des cellules transfectées n'a été effectuée avant l'injection.

La croissance tumorale a été suivie en mesurant le volume des tumeurs à intervalles réguliers (figure 2C).

Les figures 2C et 2D ne montrent aucune différence significative entre les tailles des tumeurs des groupes A et B. Une très forte réduction du volume tumoral est observée dans le groupe C. L'aspect des tumeurs, beaucoup plus blanches dans le groupe C (Fig 2D) traduit une diminution marquée de la vascularisation tumorale. Après sacrifice des animaux, au jour 12 après l'injection, les tumeurs ont été disséquées, fixées et une immunodétection du VEGF réalisée sur des coupes de ces tumeurs. On observe une très forte réduction de l'expression du VEGF dans les tumeurs du groupe C en comparaison avec celles du groupe B (Figure 2E).

Dans une autre expérience, des tumeurs ont été induites chez des souris nudes males par injection de cellules du carcinome prostatique LNCaP. 40 jours après injection, le volume des tumeurs ayant atteint 1 à 1, 5cm³, les souris ont été divisées en deux groupes. Le premier groupe (4 souris) a reçu une injection intraveineuse dans la veine de la queue de 2 microgrammes de siRNA contrôle dans 100*µ*l de PBS. Le second groupe a reçu une dose équivalente de siRNA VEGF dans les mêmes conditions. On observe que le siRNA VEGF, mais pas le siRNA contrôle, induit un arrêt transitoire de la croissance tumorale. (figure 4D).

### EXEMPLE 3 : INHIBITION DE LA REPONSE HYPOXIQUE. I - Introduction.

Certaines tumeurs sont capables de se développer dans des conditions de forte anoxie. On observe très fréquemment dans les tumeurs des régions très peu vascularisées. Cette faible sensibilité à l'hypoxie a deux conséquences : d'une part un traitement anti-angiogénique a peu de chances d'être efficace sur ces tumeurs ou ces sous populations tumorales. D'autre part, cette faible vascularisation rend difficile la délivrance de molécules thérapeutiques. Le facteur de transcription Hif1α régule l'activité de plus de 100 gènes permettant la réponse hypoxique. L'inhibition de ce facteur de transcription dans les tumeurs hypoxiques a pour but de bloquer leur croissance.

### II - Préparation des oligonucléotides.

- RNAi Hifla
   5' [CAUGUGACCAUGAGGAAAUGA]RNA-TT[DNA] (SEQ ID NO. 11)
   5' [UCAUUUCCUCAUGGUCACAUG]RNA-TT[DNA] (SEQ ID NO. 12)
- RNAi contrôle
   5' [GAUAGCAAUGACGAAUGCGUA]RNA-TT[DNA] (SEQ ID NO. 13)
   5' [UACGCAUUCGUCAUUGCUAUC]RNA-TT[DNA] (SEQ ID NO. 14)

III - Résultats.

Le promoteur du VEGF contient un élément de réponse au facteur de transcription Hifla. Pour tester *in vitro* l'effet d'un RNAi dirigé contre Hif1α, nous avons transfecté des cellules cJ4 avec un vecteur reporter VEGF-luciférase, seul ou en association avec un RNAi Hif1α ou contrôle.

24h après transfection, les cellules ont été incubées pendant 18h en milieu sans sérum, additionné ou non de chlorure de Cobalt 100*µ*M afin de produire des conditions hypoxiques puis l'activité luciférase a été mesurée.

La figure 3 montre une inhibition complète de l'induction de la réponse du promoteur VEGF à l'hypoxie a été observée lorsque les cellules sont transfectées avec le RNAi Hif1α mais pas avec le RNAi contrôle.

### EXEMPLE 4 : inhibition des formes sauvages ou mutantes des récepteurs des androgènes dans les carcinomes prostatiques.

### I - Introduction.

Les carcinomes prostatiques sont la deuxième cause de mortalité par cancer pour les hommes dans les pays industrialisés. En France, ils sont la cause de plus de 9500 morts par an. Les cellules épithéliales prostatiques sont dépendantes des androgènes pour leur croissance. Les carcinomes prostatiques sont initialement androgéno-dépendants. Une castration chimique permet dans un premier temps de bloquer la croissance du carcinome. Cependant dans tous les cas, ces carcinomes deviennent androgéno-indépendants et leur pronostic est alors très pessimiste. Cette androgéno-indépendance est due suivant les individus le plus souvent à une mutation du récepteur (lui conférant par exemple une réponse aux oestrogènes ou aux glucocorticoïdes) ou à une amplification du récepteur.

### II - Préparation des oligonucléotides.

Deux oligonucléotides ARN, complémentaires d'une région de la séquence codante du récepteur androgène (AR) non muté humain, ont été synthétisés. Deux déoxynucléotides (TT) ont été ajoutés en 3'. Dans d'autres expériences, des siRNA, dénommés LNCaP, reconnaissant spécifiquement la mutation du récepteur des androgènes (T877A) dans les cellules de carcinome prostatique humain LNCaP ont été utilisés.
- AR :
   5' [GACUCAGCUGCCCCAUCCACG]ARN-TT[ADN] (SEQ ID NO.15)
   5' [CGUGGAUGGGGCAGCUGAGUC]ARN-TT[ADN] (SEQ ID NO.16)
- Contrôle :
   5' [GAUAGCAAUGACGAAUGCGUA]ARN-TT[ADN] (SEQ ID NO.17)
   5' [UACGCAUUCGUCAUUGCUAUC]ARN-TT[ADN] (SEQ ID NO.18)
- LNCap :
   5' [GCAUCAGUUCGCUUUUGAC]ARN -TT[ADN] (SEQ ID NO.19)
   5' [GUCAAAAGCGAACUGAUGC]ARN -TT[ADN] (SEQ ID NO.20)

Plusieurs sous-clones de la lignée de carcinome prostatique humaine LNCaP ont été utilisés dans cette étude. La lignée originale, LNCaP, est androgéno-dépendante. Les cellules LN70, obtenues par passages répétés de la lignée LNCaP in vitro, ont une diminution de leur réponse aux androgènes. Le clone LNS5, obtenu après passage des cellules chez l'animal, est androgéno-résistant.

### III - Résultats.

Des cellules LNCaP ont été transfectées in *vitro* avec des siRNA AR ou des siRNA contrôle en utilisant l'agent de transfection polyfect (qiagen). 48h après transfection, les cellules ont été détachées de leur support. La moitié des cellules a été utilisée pour réaliser une détection par western blot du récepteur des androgènes, l'autre moitié a été remise en culture. Le récepteur des androgènes (bande à 110 kDa) n'est plus détectable par western dans les cellules transfectées par le siRNA AR (Figure 4A). Les cellules transfectées par le siRNA remises en culture se sont avérées incapables de poursuivre leur croissance, contrairement aux cellules transfectées par le siRNA contrôle.

Le niveau de réponse aux androgènes a été mesuré en transfectant différents clones cellulaires de la lignée LNCaP avec un vecteur rapporteur plaçant la séquence codante de la luciférase en aval d'un promoteur minimal flanqué de 4 répétitions de l'élément de réponse aux androgènes (4xARE). Après transfection, les cellules ont été incubées pendant 18h en absence de sérum et en présence ou en absence d'un analogue métaboliquement stable de la dihydro testostérone, le R1881 (NEN). Le rapport des activités luciférases dans ces deux conditions permet de mesurer le niveau de réponse aux androgènes du vecteur reporter.

Nous avons mesuré l'effet de la cotransfection dans ces cellules du RNAi contrôle ou du RNAi AR sur la réponse aux androgènes des différents clones de la lignée LNCaP.

La figure 4B montre une inhibition complète de la réponse aux androgènes dans les deux clones sensibles aux androgènes : LNCaP et LNCaP p70. Cette méthode ne permet pas de mesurer la réponse du clone LNS5, androgéno-résistant, au traitement par le RNAi AR.

Le récepteur des androgènes présent dans la lignée LNCaP est porteur d'une mutation. Nous avons utilisé deux siRNA différents pour inhiber sa synthèse, le siRNA AR précédemment utilisé et le siRNA LNCaP reconnaissant spécifiquement la mutation LNCaP. La réponse aux androgènes a été mesurée comme dans l'expérience 4B (Figure 4C).

Pour étudier l'effet de l'inhibition de l'expression du récepteur des androgènes sur la croissance tumorale in vivo des cellules de carcinome prostatique, des cellules du carcinome LNCaP, transfectées par un siRNA contrôle (groupe A) ou AR (groupe B) ont été injectées en sous cutané à des souris nudes males. La croissance tumorale a été suivie à intervalles réguliers. On observe que les tumeurs des animaux du groupe B ont démarré plus tardivement que celles du groupe A et que le volume des tumeurs du groupe B au 48e jour est nettement plus petit que celui des tumeurs du groupe A (Figure 4D).

Dans une autre expérience, des cellules LNCaP ont été injectées chez des souris nudes males. Lorsque, au 34e jour, les tumeurs ont atteint un volume compris entre 1,2 et 1,5 cm³, les souris ont reçu par voie intrapéritonéale une injection de 2*µ*g de siRNA contrôle ou AR dans 100*µ*l de PBS. Cette injection a été répétée au 40e jour. On observe que l'administration de siRNA AR entraîne un ralentissement de la croissance tumorale (Figure 4E).

### EXEMPLE 5 : Inhibition des formes sauvages ou mutantes de la protéine p53.

### I - Préparation des oligonucléotides.

Les trois siRNAs dont la séquence est indiquée ci-dessous ont été préparés, l'un dirigé contre la forme sauvage de la p53, et l'autre dirigé contre la forme mutée exprimée chez un patient et ayant donné lieu à l'établissement d'une lignée.

Cette mutation correspond à l'une des trois observées le plus fréquemment dans les tumeurs humaines.
- p53 sauvage :
   Sense: [**G**CAUGAACCGGAGGCCCAU]ARN[TT]ADN (SEQ ID NO.21)
   Anti: [AUGGGCCUCCGGUUCAUG**C**]ARN[TT]ADN (SEQ ID NO.22)
- p53 MT1 (r248w) :
   Sense: [GCAUGAAC*U*GGAGGCCCAU]ARN[TT]ADN (SEQ ID NO.23)
   Anti: [AUGGGGCUCC*A*GUUCAUGC]ARN[TT]ADN (SEQ ID NO.24)
- p53 MT2 (r248w) :
   Sense: [*U*CAUGAAC*U*GGAGGCCCAU]ARN[TT]ADN (SEQ ID NO.25)
   Anti: [AUGGGCCUCC*A*GUUCAUG*A*]ARN[TT]ADN (SEQ ID NO.26)

Les nucléotides soulignés dans la p53 sauvage sont ceux qui sont mutés dans la forme mutante et qui sont en italique dans les séquences des formes mutées de la p53 mutée (p53 MT1 et MT2). Les bases en gras ci-dessus sont des mutations qui ont été introduites pour augmenter la spécificité.

II - Résultats.

Comme montré sur la figure 5B, les cellules H1299-NCI, qui n'expriment pas la p53, ont été transfectées (en utilisant la lipofectamine) par des vecteurs d'expression (400 ng) de la p53 sauvage (WT) ou mutée (mt). Les siRNAs (en dose croissante : 0, 125 ng, 250 ng, 500 ng et 1000 ng) dirigés contre la forme sauvage (WT), la forme mutée (MT1 et MT2), ou un siRNA irrelevant (C) ont été transfectés en même temps. Les cellules ont été collectées 24 heures après, et analysées par Western blot avec un anticorps dirigé contre la p53.

Comme montré sur la figure 5C, les cellules H1299-NCI, qui n'expriment pas la p53, ont été transfectées (en utilisant la lipofectamine) par des vecteurs d'expression (400 ng) de la p53 sauvage (WT), mutée (mt) ou un mélange des deux (WT+MT), comme indiqué. Les siRNAs (400 ng) dirigés contre la forme sauvage (WT), la forme mutée (MT1), ou un siRNA irrelevant (C) ont été transfectés en même temps. Les cellules ont été collectées 24 heures après, et analysées par Western blot (ib : immunoblot) avec un anticorps dirigé contre la p53 (DO1, Santa Cruz), ou l'actine cellulaire (Sigma) pour contrôler la quantité de protéines utilisée dans le test.

Comme montré sur la figure 5D, des cellules U2OS (ostéosarcome humain exprimant une forme sauvage de la p53) ont été transfectées de façon stable soit par un vecteur exprimant une forme mutante de la P53 (R248W) soit par le vecteur vide correspondant (pCDNA3). Ces lignées ont été transfectées par les siRNA indiqués et l'expression des protéines indiquées a été détectée par western blot.

Dans tous les cas, le siRNA dirigé contre la forme mutée de la protéine inhibe la forme mutée et le siRNA dirigé contre la forme sauvage inhibe la forme sauvage. De plus, il n'y a pas de « réaction croisée », puisque le siRNA dirigé contre la forme sauvage n'a pas d'effet sur la forme mutée et réciproquement. Il faut noter que l'expression du mutant stabilise la protéine sauvage lorsqu'elle est co-exprimée. En conséquence, l'inhibition du mutant ramène, par cet effet indirect, la forme sauvage à son niveau de base, sans qu'il n'y ait inhibition de l'expression de la protéine.

Comme montré sur la figure 5E, les cellules utilisées dans la figure 5D ont été transfectées par les siRNA indiqués. Les cellules ont ensuite été soumises à un stress génotoxique par traitement à la doxorubicine (200 ng/ml) pendant 24h.La Figure 5E montre l'analyse du cycle cellulaire de ces cellules par incorporation de Iodure de propidium et analyse au FACS. Les cellules non transfectées avec la forme mutante, et donc n'exprimant que la forme sauvage (cellules PCDNA) montrent un fort pourcentage d'arrêt en G1 en présence de dauxorubicine. Le traitement de ces cellules par le siRNA sauvage, en diminuant la p53 sauvage, réduit cet arrêt en G1. Les cellules qui expriment la forme mutée et sauvage (R248W) s'arrêtent peu en G1 en présence de dauxorubicine, montrant que la forme mutée inhibe l'activité de la forme sauvage. Quand ces cellules sont traitées avec le siRNA mt1, elles récupèrent une capacité normale (à comparer avec les contrôles PCDNA non traitées) de s'arrêter en G1, montrant la restauration de l'activité p53 sauvage dans ces cellules.

Comme montré sur les figures 5F, G, H, les cellules MDA 087 (provenant d'un patient atteint d'un syndrome de Li Fraumeni et exprimant le mutant R248W) ont été transfectées avec un siRNA dirigé contre la forme mutante (MT1) de la p53, ou encore avec un siRNA irrelevant (C) (1,6 *µ*g). L'expression de la P53 a été détectée dans ces cellules par western blot (Figure 5F), les ARN messagers ont été mesurés par PCR quantitative (Light cycler, Roche) (Figure 5G) ou immunofluorescence (Figure 5H).

Les cellules MDA 087 ont été transfectées avec un siRNA reconnaissant la forme sauvage (WT) ou la forme mutée de la p53 (mtl) ou encore par un siRNA contrôle puis soumises à un stress génotoxique par traitement à la doxorubicine (200 ng/ml) pendant 24h. L'expression de la forme mutante de la p53 a été détectée par western blot dans les cellules. On observe que les cellules ayant reçu le siRNA mt1 ne sont pas capables de stabiliser la p53 en réponse à la dauxorubicine (Figure 5I).

La figure 5J montre l'effet des siRNA mt1 et mt2 dans des cellules qui expriment les formes sauvages et mutées de la p53. Les cellules H1299-NCI, qui n'expriment pas la p53, ont été transfectées (en utilisant la lipofectamine) par un vecteur reporter comportant le gène de la luciférase sous contrôle d'un élément de réponse à la p53 et des vecteurs d'expression (400 ng) de la p53 sauvage (WT), mutée (MT) ou un mélange des deux (WT+MT), comme indiqué. Les siRNAs (400 ng) dirigés contre la forme sauvage (WT), la forme mutée (mt1, mt2), ou un siRNA irrelevant (C) ont été transfectés en même temps. Les cellules ont été collectées 24 heures après et analysées pour l'expression de la luciférase. La p53 sauvage seule active le vecteur reporter, et la co-expression de la forme mutante inhibe cette activité. La co-transfection du siRNA sauvage inhibe l'expression de la protéine sauvage et donc l'activation résiduelle du gène reporter. La co-transfection des siRNAs mt1 ou mt2, au contraire, restaure cette activation en bloquant sélectivement l'expression de la forme mutée et en empêchant l'effet de transdominant négatif qu'elle exerce sur la forme p53 sauvage.

La figure 5K montre un résultat similaire sur l'expression d'une des cibles de la p53, la protéine inhibitrice de la prolifération cellulaire p21, dans des cellules traitées comme dans la figure 5F. L'expression de la p21, détectée par western blot est activée par la p53 sauvage et inhibée lorsque le mutant est co-exprimé. Cette inhibition est levée en présence du siRNA mt1.

### EXEMPLE 6 : Inhibition de la protéine virale E6.

### I - Préparation des oligonucléotides.

Un siRNA dirigé contre la protéine E6 de HPV a également été préparé. Il répond à la séquence suivante :
- HPV-16-S2
   Sens: 5' [CCACAGUUAUGCACAGAGC]ARN[TT]ADN (SEQ ID NO.27)
   Anti: 5' [GCUCUGUGCAUAACUUGG]ARN[TT]]ADN (SEQ ID NO.28)

### II - Résultats.

Comme montré sur la figure 6B, des cellules CasKi et SiHA, exprimant toutes deux la protéine E6 de HPV ont été transfectées avec les siRNA indiqués, traitées ou non comme indiqué par la doxorubicine et analysées par western blot en utilisant les anticorps indiqués. Le traitement des cellules par le siRNA E6 induit une augmentation de l'expression de P53. Cette expression de p53 se traduit par une augmentation de l'expression de la protéine p21.

Comme montré sur la figure 6C, le cycle cellulaire de cellules SiHA traitées comme dans la figure 6B a été analysé par FACS. La figure représente une expérience caractéristique. On observe une augmentation de cellules en phase G1 (Figure 6D) dans les cellules traitées par le siRNA E6, augmentation qui est également observée dans ces cellules lorsqu'elles sont traitées par la doxorubicine.

### EXEMPLE 7 : Effet des oligonucléotides ARN/ARN et des hybrides ADN/ARN.

### I - Introduction.

L'invention envisage l'utilisation d'oligonucléotides hybrides ADN/ARN comme alternative aux oligonucléotides ARN/ARN pour inhiber spécifiquement l'expression d'un gène. Dans le cas des hybrides ADN/ARN, le brin sens est préférentiellement de nature ADN et le brin anti-sens de nature ARN. Les autres aspects ayant trait notamment à la taille des oligonucléotides, à la nature des extrémités 3' et au mode de synthèse sont les mêmes que pour les oligonucléotides ARN/ARN. Les applications de ces hybrides ADN/ARN sont identiques à celles précédemment décrites pour les siRNA ARN/ARN, notamment en ce qui concerne les applications thérapeutiques, à visées diagnostic ou de validation de gènes. Les doses d'oligonucléotides employées pour obtenir les mêmes effets avec les hybrides ADN/ARN et les ARN/ARN peuvent cependant être différentes

### II - préparation des oligonucléotides.

Le brin sens est celui dont la séquence est identique à celle de l'ARN messager. Le brin antisens est le brin complémentaire du brin sens. Par convention, dans un duplexe, la nature des brins est indiquée dans l'ordre sens/antisens. Ainsi par exemple, un hybride ADN/ARN, noté D/R est un oligonucléotide dont le brin sens est de nature ADN et le brin antisens, est de nature ARN et de séquence complémentaire de l'ARN messager.

Dans les expériences décrites, les oligonucléotides dont la séquence est indiquée ci-dessous ont été utilisés.
- Pour la GFP :
   GFP :
   Sens : [GCAAGCTGACCCTGAAGTTCAT]ADN (SEQ ID NO.29)
   Antisens : [GAACUC7CAGGGUCAGCUUGCCG]ARN (SEQ ID NO.30)
   Contrôle GFP :
   Sens : [CAUGUCAUGUGUCACAUCUC]ARN[TT]ADN (SEQ ID NO.31)
   Antisens : [GAGAUGUGACACAUGACAUG]ARN[TT]ADN (SEQ ID NO.32)
- Pour le LNCaP : Les bases soulignées ci-dessous correspondant à la mutation du récepteur des androgènes exprimée dans les cellules du carcinome prostatique humain (LNCap).
   LNCaP :
   Sens :
      [GCATCAGTTCGCTTTTGACTT]ADN (SEQ ID NO.33)
      [GCAUCAGUUCGCUUUUGAC]ARN-TT[ADN] (SEQ ID NO.34)
   Antisens :
      [GTCAAAAGCGAACTGATGCTT]ADN (SEQ ID NO.35)
      [GUCAAAAGCGAAGUGAUGC]ARN-TT[ADN] (SEQ ID NO.36)
   Contrôle LNCaP :
   Sens :
      [GUUCGGUCUGCUUACACUA]ARN-TT[ADN] (SEQ ID NO.37)
   Antisens :
      [UAGUGUAAGCAGACCGAAC]ARN-TT[ADN] (SEQ ID NO.38)
   - Pour 1a P53 :
      Les brins ADN des hybrides notés H1 comportent des bases ARN (**U**, soulignées).

La mutation présente dans les oligonucléotides MT1 est indiquée en italique.
WT :
Sens: 5'[GCAUGAACCGGAGGCCCAU]ARN[TT]ADN (SEQ ID NO.39)
Anti: 5`[AUGGGCCUCCGGUUCAUGC]ARN[TT]ADN (SEQ ID NO.40)
wt H1 D/R :
Sens: 5'[GCA**U**GAACCGGAGGCCCA**U**TT]ADN (SEQ ID NO.41)
Anti: 5'[AUGGGCCUCCGGUUCAUGC]ARN[TT]ADN (SEQ ID NO.42)
wt H1 R/D :
Sens: 5'[GCAUGAACCGGAGGCCCAU]ARN[TT]ADN (SEQ ID NO.43)
Anti: 5'[A**U**GGGCC**U**CCGG**UU**CA**U**GCTT]ADN (SEQ ID NO.44)
wt H2 D/R :
Sens: 5'[GCATGAACCGGAGGCCCATTT]ADN (SEQ ID NO.45)
Anti: 5'[AUGGGCCUCCGGUUCAUGC]ARN[TT]ADN (SEQ ID NO.46)
wt H2 R/D :
Sens: 5'[GCAUGAACCGGAGGCCCAU]ARN[TT]ADN (SEQ ID NO.47)
Anti: 5'ATGGGCCUTCCGGTTCATGCTT]ADN (SEQ ID NO.48)
Mt1 (r248w) ** :
Sens: 5'[GCAUGAAC***U***GGAGGCCCAU]ARN[TT]ADN (SEQ ID NO.49)
Anti: 5'[AUGGGCCUCC***A***GUUCAUGC]ARN[TT]ADN (SEQ ID NO.50)
Mt1 H1 D/R :
Sens : 5'[GCA**U**GAAC***U***GGAGGCCCA**U**TT]ADN (SEQ ID NO.51)
Anti : 5'[AUGGGCCUCC***A***GUUCAUGC]ARN[TT]ADN (SEQ ID NO.52)
Mt1 H1 R/D :
Sens: 5'[GCAUGAAC***U***GGAGGCCCAU]ARN[TT]ADN (SEQ ID NO.53)
Anti: 5'A**U**GGGCC**U**CCAG**UU**CA**U**GCTT]ADN (SEQ ID NO.54)
Mt1 H2 D/R :
Sens: 5'[GCATGAAC***T***GAGGCCCATTT] ADN (SEQ ID NO.55)
Anti: 5'[AUGGGCCUCC***A***GUUCAUGC]ARN[TT]ADN (SEQ ID NO.56)
Mt1 H2 R/D :
Sens: 5'[GCATGAAC***T***GGAGGCCCAT]ARN[TT]ADN (SEQ ID NO.57)
Anti: 5'[AUGGGCCUCC***A***GUUCAUGCTT]ADN (SEQ ID NO.58)

### II - Résultats.

### 1) Inhibition de la GFP (Green Fluorescent Protein) par les hybrides ADN/ARN.

Les siRNAs contrôle (R/R) ou GFP (D/R) en doses croissantes ont été introduits par transfection en utilisant le kit Polyfect dans les myoblastes de souris C2C12, en même temps qu'un vecteur d'expression de la GFP. Le niveau de GFP a été suivi par Western Blot (Figure 7A) et par mesure directe de la fluorescence émise par la GFP à l'aide d'un fluorimètre (Figure 7B). On observe une forte inhibition (jusqu'à 80%) de l'expression de la GFP par les siRNA hybrides ADN/ARN.

### 2) Inhibition du récepteur des androgènes par les hybrides ADN/ARN.

Les cellules LNCaP ont été transfectées avec un vecteur reporteur mettant la luciférase sous le contrôle d'un promoteur contenant 4 éléments de réponse au récepteur des androgènes. 24h plus tard, Les siRNA R/R, D/R ou R/D indiqués sur la figure ont été transfectés par l'agent de transfection Transit-tKO (Mirus) à raison de 250ng de chaque double brin pour 80000 cellules. Les cellules ont été incubées en milieu complet contenant des androgènes et l'activité luciférase, normalisée par rapport à la quantité de protéines de chaque échantillon, a été mesurée 24h plus tard (Figure 7C). Les hybrides R/D n'ont pas montré d'activité inhibitrice dans cette expérience. Les hybrides LNCaP D/R inhibent aussi efficacement que les siRNA R/R le récepteur des androgènes.

### 3) Inhibition de la p53 par les hybrides ADN/ARN.

La figure 7D montre que les hybrides H1 D/R sont aussi efficaces que les R/R pour inhiber l'expression des gènes. Les cellules H1299-NCI, qui n'expriment pas la p53, ont été transfectées (en utilisant la lipofectamine) par des vecteurs d'expression (400 ng) de la p53 sauvage (WT), mutée (MT) ou un mélange des deux (WT+MT), comme indiqué. Un vecteur CMV-GFP a été transfecté également comme contrôle interne. Les siRNAs (400 ng) dirigés contre la forme sauvage (WT), la forme mutée (MT), ou un siRNA irrelevant (CTRL) ont été transfectées en même temps. Les cellules ont été collectées 24 heures après, et analysées par Western blot avec un anticorps dirigé contre la p53 (DO1, Santa Cruz), ou la GFP (Santa-Cruz) pour contrôler l'efficacité de transfection. Note : l'expression de la forme mutée de la protéine stabilise la forme sauvage.

La figure 7E montre que les hybrides H2 D/R sont aussi efficaces que les R/R pour inhiber l'expression des gènes. Les cellules H1299-NCI, qui n'expriment pas la p53, ont été transfectées (en utilisant la lipofectamine) par des vecteurs d'expression (400 ng) de la p53 sauvage (WT), mutée (MT) ou un mélange des deux (WT+MT), comme indiqué. Les siRNAs (400 ng) dirigés contre la forme sauvage (WT), la forme mutée (MT), ou un siRNA irrelevant (C) ont été transfectées en même temps. Les cellules ont été collectées 24 heures après, et analysées par Western blot avec un anticorps dirigé contre la p53 (DO1, Santa Cruz).

### EXEMPLE 8 Administration in vivo de siRNA par différentes voies.

Des cellules tumorales exprimant la luciférase de façon stable ont été injectées en sous cutané à des souris nudes (1 million de cellules dans le flanc droit). Au 8e jour de la croissance tumorale, les tumeurs ayant un volume moyen de 200 mm3 ont été injectées soit avec des siRNA contrôles (séquence mélangée de HIF1 alpha, voir exemple 3) soit avec un siRNA dirigé contre la luciférase. Les siRNA contrôles (3 *µ*g/souris) ont été injectés dans un volume de 50*µ*l de PBS par voie sous cutanée dans le flanc de l'animal.

Les siRNA Luciférase ont été injectés à raison de 3*µ*g/souris (3 animaux dans chaque groupe) dans 50 *µ*l de PBS par voie sous cutanée (sc), ou par voie intrapéritonéale (ip) ou par voie intra-veineuse(iv) (veine de la queue) ou par voie intratumorale (it). Dans ce dernier cas, les siRNA luciferase (3*µ*g/souris) ont été dilués dans 20*µ*l de PBS seulement.

Trois jours après l'injection des siRNA, les animaux ont été sacrifiés, les tumeurs ont été prélevées, homogénéisées à l'aide d'un broyeur polytron. Sur les homogénats, un dosage de protéines et une mesure de l'activité luciférase dans un luminimètre ont été réalisés.

Les résultats représentés à la figure 8 montrent l'activité luciférase rapportée à la quantité de protéine.

**Tableau 2 Annexe A**

| **Maladie** | **Protéine de fusion** | **Translocation chromosomique** | **références** |
|---|---|---|---|
| APL (acute promyelocytic | pML-RARalpha | t(15;17)(q22;q21) | De The et al. *Cell* 1991, **66**:675 |
| leukaemia) | PLZF-RARalpha | t(11;17)(q23;q21) | Chen Z et al. EMBO J 1993, 12:1161 |
| | NPM-RARalpha | t(5;17)(q32;q12) | Redner RL et al. Blood 1996, 87:882 |
| | NuMA-RARalpha-- | t(5;17)(q13,q21) | Wells RA et al. Leukemia 1996, 10:735 |
| | STAT5beta/RARalpha | | Arnould C et al. Hum. Mol. Genet. 1999, 8:1741 |
| ALL (acute lymphoblastic | TEL-AMLI | t(12;21) (p13;q22) | |
| leukaemia) | BCR/ABL | t(9;22)(q34;q11) | |
| | MLL/AF4 | t(4;11))(q21;q23) | Domer PH et al. Proc Natl Acad Sci USA 1993, 90:7884-8 |
| | ALL-translocation | t(12;21)(q12;q22) | |
| | CALM/AF10 | t(10;11)(p12-p13;q14-q21) | Dreyling MH et al. Proc Natl Acad Sci U S A 1996, |
| | ALL1/AF4 | t(4;11) | 93:4804 |
| | E2A/HLF | t(17;19)(q22:p13) | Janssen JW et al. Blood 1994, 84:3835 |
| | | | Hunger SP et al. Genes Dev 1992, 6:1608 |
| AML (acute myeloid leukemia) | TLS/FUS-ERG | t(16;21)(p11;q22) AML(M7) | Ichikawa H et al. Cancer Res 1994, 54:2865 |
| | MLL-AF10 | t(10;11)(p12-p13;q23) | Borkhardt A et al. Leukemia 1995, 9:1796 |
| | MLL-ABI1 | t(10;11) | Shibuya et al. Genes Chromosomes Cancer 2001, 32:1 |
| | HLXB9-ETV6 | t(7;12)(q36;p13) | Beverloo et al. Cancer Res 2001, 61:5374 |
| | MLL-ELL | t(11,19)(q23;p13.1) | Rubnitz JE et al. Blood 1996, 87:4804 |
| | CBFbeta/MYH11 | inv[16] | Tobal K et al. Br J Haematol 1995, 91:104 |
| | AML1-MTG8 | t(8;21) | Miyoshi et al. EMBO J 1993, 12:2715 |
| | TEL-TRKC | t(12;15)(p13;q25) | Eguch et al. Blood, 1999, 93:1355 |
| | AML1/ETO | t(8;21) | Kusec R et al. Leukemia, 1994, 8:735 |
| | CALM/AF10 | t(10;11)(p12-p13;q14-q21) | Dreyling MH et al. Proc Natl Acad Sci U S A 1996, |
| | ETV6-BTL | t(4;12)(q11-q12;p13). | 93:4804 |
| | CBFbeta-SMMHC | inv(16)(p13;q22) | Cools et al. Blood 1999, 94:1820 |
| | FUS/ERG | t(16;21)(p11;q22) | Wijmenga C et al. Proc Natl Acad Sci U S A 1996, 93:1630 |
| | DEK/CAN | t(6;9) (p23; q34). | Panagopoulos I et al. Genes Chromosomes Cancer, 1994, |
| | MLL-AF9 | t(9;11)(p22;q23) | 11:256 |
| | MLL-ENL | (11q23) | on Lindern M et al. Mol Cell Biol, 1992, 12:1687 |
| | MLL-AF4 | t(4;11)(q21;q23) | Super HJ et al. Blood, 1995, 85:855 |
| | MLL-AF6 | t(6;11)(q27;23) | Schreiner SA et al. Leukemia 1999, 13:1525 |
| | MLL-AF17 | t(11;17)(q23;q21) | Domer PH et al. Proc Natl Acad Sci U S A 1993, 90:7884 |
| | MLL-AFX | t(X;11)(q13;q23). | Tanabe S et al. Genes Chromosomes Cancer 1996,15:206 |
| | MLL-AF1p | | Prasad R et al. Proc Natl Acad Sci USA 1994, 91:8107 |
| | MLL-AF1q | t(1;11)(q21;q23) | Borkhardt A et al. Oncogene 1997, 14:195 |
| | MLL self | | So CW et al. Leukemia 2000,14:594 |
| | MLL-CBP | t(11;16)(q23;p13) | Busson-Le Coniat M et al. Leukemia 1999, 13:302 |
| | AML1-ETO | t(8;21) | So CW et al. Cancer Res 1997, 57:117 Taki T et al. Blood 1997, 89:3945 Erickson P et al. Blood 1992, 80:1825 |
| MDS/AML (myelodysplasia/acute myeloid leukemia) | NPM-MUF1 | t(3;5)(q25.1;q34) | Yoneda-Kato N et al. Oncogene 1996, 12:265 |
| CML (chronic myelogenous leukemia) | Bcr-Abl/p210 | | Bcn-Neriah Y et al. Science 1986,233:212 |
| | AML1-MDS1-EVI1 (AME) | t(3;21)(q26;q22) | Fears S et al. Proc Natl Acad Sci U S A 1996, 93:1642 |
| BpALL (cell acute lymphoblastic leukemia) | TEL-AML1 | t(12;21) (p13;q22) | Golub TR et al. Proc Natl Acad Sci U S A 1995, 92:4917 |
| MPD (myeloproliferative disease) | TEL-JAK2 | t(9;12)(p24;q13) | Lacronique et al. Science 1997, 278:1309 |
| | TEL-PDGFbetaR | t(5;12)(q33;p13) | Jousset C et al. EMBO J, 1997, 16:69 |
| | TEL-TRKC | t(12;15)(p13;q25) | Eguch et al. Blood, 1999, 93:1355 |
| CMML (chronic myelomonocytic leukemia) | involving PDGFbetaR | t(5;17)(q33;p13) | Magnusson et al. Blood 2001 98:2518 |
| | HIP1/PDGFbetaR | t(5;7)(q33;q11.2) | Ross TS et al. Blood 1998, 91:4419 |
| | TEL/PDGFbetaR | t(5;12)(q33;p13) | Tomasson MH et al. Blood 1999, 93:1707 |
| MALT (gastric mucosa-associated lymphoid tissue lymphoma) | API2-MALT1 | t(11;18)(q21;q21) | Motegi M et al. Am J Pathol 2000, 156:807 |
| ALCL (anaplastic large cell lymphoma) | NPM-ALK | t(2;5)(p23;q35) | Waggott W et al. Br J Haematol 1995, 89-905 |
| | SU-DHL-1 | t(2;5) | Siminovitch KA et al. Blood 1986, 67:391 |
| | ATIC-ALK | inv(2)(p23q35) | Colleoni GW et al. Am J Pathol 2000, 156:781 |
| | ALK-related translocation | t(2;17)(p23;q25) | Maes et al. Am J Pathol 2001, 158:2185 |
| MPD (myeloproliferative disease) | NUP98-HOXA9 | t(7;11)(p15;p15) | Nakamura T et al. Nat Genet 1996, 12:154 |
| APP (amyloid precursor protein) in sporadic Alzheimer's disease (AD) or Down's syndrome | APP+1 (38-kDa) | | Hersberger et al. J Neurochem 2001 76(5):1308-I4 |
| primary pleural monophasic synovial sarcomas (SS) | SYT-SSX1 | t(X;18)(p11.2;q11.2) | Crew AJ et al. EMBO J 1995,14:2333 |
| | SYT-SSX2 | t(X;18)(p11.2;q11.2) | Crew AJ et al. EMBO J 1995,14:2333 |
| Dermatafibrosarcoma protuberans (DP) | COL1A1/PDGFB rearrangement | t(17;22) | O'Brien KP et al. Genes Chromosomes Cancer 1998, 23:187 |
| ARMS (pediatric alveolar rhabdomyosarcoma) | EWS-FLII | | Athale et al. J Pediatr Hematol Oncol 2061, 23:99 |
| | EWS-ERG | t(11;22)(q24;q12) | Sorensen PH et al. Nat Genet 1994, 6:146 |
| ESFT (Ewing sarcoma family of tumors) | PAX3-FKHR | t(2;13) (q35;q14) | Fredericks WJ et al. Mol Cell Biol 1995,15:1522 |
| | PAX7-FKHR | t(1;13)(p36;q14) | Barr FG et al. Hum Mol Genet 1996, 5:15 |
| DSRCT (desmoplastic small round cell tumors) | EWS-WTI | t(11:22)(p13:q12) | Benjamin et al. Med Pediatr Oncol 1996 27(5):434-9 |
| | EWS/FI-1 | t(11-22) (q24;q12) | Fidelia-Lambert et al. Hum Pathol 1999, 30:78 |
| MM (multiple myeloma) | IGH-MMSET | t(4;14)(p16.3; q32) | Malgeri et al. Cancer Res 2000 60:4058 |
| MPD (stem cell myeloproliferative disorder) | FGFR1-CEP110 | t(8;9)(p12;q33) | Guasch et al. Blood 2000 95:1788 |
| Ewing sarcoma (ES)-peripheral primitive neuroectodermal tumor (pPNET) | EWS-FEV | t(2;22)(q13;q22,t(3;18) (p21;q23) | Llombart-Bosch et al. Diagn Mol Pathol 2000, 9:137 |
| | EWS-FLI1 | t(11;22;14)(q24;q12;q11) | Bonin G et al. Cancer Res 1993, 53:3655 |
| | EWS-ERG | t(21;22)(q22;q12) | Sorensen PH et al. Nat Genet. 1994, 6:146 |
| | ETV6/CBFA2 | t(12;21)(p12;q22) | Fears S et al, Genes Chromosomes Cancer 1996, 17:127 |
| MLS (myxoid liposarcomas) | FUS/CHOP | t(12;16)(q13;p11) | Rabbitts TH et al. Nat Genet 1993, 4:175 |
| | EWS/CHOP | t(12;22;20)(q13;q12;q11) | Zinszner H et al. Genes Dev 1994, 8:2513 |

**Tableau 3 Annexe B**

| Codon | Event | Codon | Event | Codon | Event | Codon | Event | Codon | Event |
|---|---|---|---|---|---|---|---|---|---|
| 248 | G->A | 129 | C->A | 189 | C->G | 217 | Stop at 219 | 202 | Ins |
| 248 | C->T | 281 | A->G | 290 | G->T | 239 | Stop at 259 | 247 | Ins |
| 282 | C->T | 293 | Fr. | 136 | Stop at 169 | 187 | G->C | 171 | Ins |
| 175 | G->A | 157 | DEL | 201 | Stop at 208 | 273 | Stop at 343 | 203 | Ins |
| 196 | C->T | 161 | C->A | 275 | Stop at 344 | 182 | C->T | 290 | Stop at 303 |
| 213 | G->A | 195 | A->T | 132 | Stop at 148 | 263 | Stop at 344 | 233 | gdel |
| 234 | T->C | 197 | G->C | 176 | Stop at 180 | 307 | Stop at 344 | 210 | Stop at 244 |
| 237 | T->G | 342 | Fr. | 191 | del | 261 | Stop at 344 | 201 | G->A |
| 244 | G->T | 135 | G->C | 218 | G->A | 285 | Stop at 344 | 92 | Ins |
| 256 | A->G | 145 | T->A | 234 | T->A | 159 | G->A | 44 | Fr. |
| 259 | A->G | 276 | G->C | 136 | C->A | 168 | C->T | 109 | ins |
| | | | | | G->C/G- | | | | G->A/G- |
| 260 | T->G | 173 | G->T | 245 | >A | 230 | C->T | 279 | >A |
| 245 | G->T | 270 | T->G | 126 | Stop at 148 | 228 | A->C | 168 | Stop at 170 |
| 278 | C->T | 158 | G->C | 259 | G->C | 230 | C->A | 153 | Stop at 178 |
| 134 | T->A | 152 | Fr. | 171 | G->C | 287 | Stop at 300 | 247 | C->A |
| 194 | C->T | 132 | G->T | 197 | T->A | 269 | Stop at 343 | 272 | Stop at 305 |
| 273 | G->A | 288 | A->C | 236 | T->G | 227 | Stop at 227 | 137 | Stop at 169 |
| 309 | C->T | 247 | A->T | 239 | C->A | 231 | Stop at 238 | 148 | Stop at 180 |
| 274 | T->A | 273 | G->C | 288 | A->T | 275 | G->C | 157 | Stop at 180 |
| 156 | G->C | 283 | G->C | 161 | Fr. | 142 | T->C | 191 | Stop at 208 |
| 245 | G->A | 109 | Fr. | 164 | Fr. | 312 | C->G | 243 | Stop at 260 |
| 193 | A->G | 174 | G->C | 142 | Stop at 148 | 282 | C->G/G->C | 251 | C->A |
| 229 | T->A | 300 | C->G | 240 | A->C | 235 | Stop at 244 | 242 | C->A |
| 237 | G->A | 205 | A->G | 137 | T->C | 156 | Stop at 179 | 244 | C->A |
| 277 | G->T | 224 | G->T | 100 | G->A | 207 | A->G | 239 | C->G |
| 194 | T->G | 168 | A->T | 106 | C->G | 179 | Stop at 246 | 142 | T->A |
| 242 | G->C | 167 | Fr. | 215 | A->G | 210 | Stop at 214 | 248 | C->A |
| 246 | G->C | 136 | C->G | 246 | Fr. | 315 | T->C | 177 | Stop at246 |
| 68 | G->T | 164 | A->C | 117 | G->A | 229 | Stop at 229 | 296 | Fr. |
| 147 | T->A | 179 | C->G | 271 | Stop at 344 | 167 | A->C | 303 | C->T |
| 151 | C->A | 187 | G->T | 324 | T->G | 256 | Stop at 343 | 140 | C->A |
| 209 | A->T | 201 | Stop at 246 | 346 | Fr. | 176 | Stop at 176 | 268 | C->T |
| 213 | C->T | 213 | C->A | 174 | Stop at 246 | 309 | Stop at 336 | 254 | A->C |
| 214 | A->G | 238 | G->T | 170 | Stop at 177 | 270 | Stop at 344 | | G->A |
| | | | T->G/C- | | | | | | |
| 248 | G->T | 113 | >T | 234 | T->G | 129 | G->A | 139 | Stop at 148 |
| 266 | G->T | 143 | G->T | 354 | A->G | 46 | Stop at 50 | 251 | A->G |
| 273 | C->T | 160 | G->T | 259 | Stop at 344 | 160 | T->G | 221 | Stop at 224 |
| 273 | G->T | 198 | G->T | 319 | Stop at 344 | 56 | A->T | 237 | A->T/G->T |
| 282 | C->G | 203 | G->T | 332 | Ins | 74 | Stop at 144 | 234 | Stop at 234 |
| 334 | G->T | 238 | T->A | 340 | Ins | 118 | A->G | 215 | T->A |
| 342 | C->T | 272 | G->C | 177 | del | 257 | del | 191 | T->A |
| 132 | A->C | 276 | Ins | 179 | T->A | 192 | G->T | 290 | C->A |
| 249 | G->C | 277 | T->G | 190 | Stop at 246 | 294 | G->T/G->C | 60 | A->T |
| 280 | G->A | 302 | G->T | 254 | Ins | 240 | del | 93 | C->A |
| 285 | G->A | 131 | C->G | 194 | C->A | 306 | G->T | 143 | T->C/G->C |
| | | | | | | | C->G/G- | | |
| 241 | C->T | 168 | A->G | 172 | T->A | 175 | >A | 319 | G->T |
| | | | | | G->T/T- | | | | |
| 249 | G->T | 258 | G->T | 173 | >G | 246 | Stop at 261 | | Stop at 122 |
| 158 | G->A | 278 | C->A | 261 | T->C | 279 | Stop at 305 | 190 | T->G |
| 163 | T->C | 285 | G->C | 266 | A->G | 146 | T->G/G->T | 192 | C->G |
| 176 | G->A | 287 | G->A | 199 | Fr. | 154 | Stop at 169 | 126 | T->G/A->G |
| 206 | Fr. | 294 | Fr. | 236 | C->G | 132 | del | 273 | Stop at 305 |
| 234 | A->G | 236 | Fr. | 168 | C->G | 175 | Stop at 175 | 266 | Stop at 344 |
| 238 | G->A | 301 | Ins | 201 | G->C | 152 | Stop at 165 | 64 | Stop at 122 |
| | A->G/T- | | | | | | | | |
| 254 | >A | 228 | A->G | 203 | Stop at 208 | 260 | Stop at 262 | 103 | C->A |
| | | | | | C->A/C- | | | | |
| 287 | G->T | 175 | Fr. | 250 | >G | 194 | Stop at 246 | 343 | A->G |
| 143 | Fr. | 282 | G->T | 283 | G->T | 170 | C->A | 317 | Stop at 344 |
| 205 | A->T | 152 | Stop at 180 | 256 | C->A | 213 | C->G | 125 | Stop at 148 |
| 262 | Fr. | 177 | C->G | 245 | C->T | 213 | A->C | 239 | A->G/C->T |
| 171 | G->T | 216 | T->A | 342 | G->C | 232 | A->G | 119 | Fr. |
| 126 | C->G | 232 | T->G | 243 | G->A | 294 | G->C | 162 | T->G/C->G |
| 138 | Fr. | 275 | Stop at 305 | 296 | A->G | 240 | T->A | 12 | C->A/C->G |
| 223 | C->T | 216 | G->T | 68 | G->C | 223 | C->G | 247 | C->G |
| 274 | G->T | 137 | Fr. | 102 | C->T | 171 | A->C | 190 | T->A |
| 218 | Fr. | 251 | T->G | 104 | G->C | 328 | T->G | 240 | T->C |
| 246 | A->G | 252 | Ins | 117 | G->C | 150 | C->T | 315 | C->T |
| 250 | Fr. | 254 | T->A | 175 | Stop at 246 | 252 | C->A | 313 | C->T |
| 143 | T->C | 49 | G->C | 138 | Stop at 169 | 256 | Stop at 342 | 42 | G->T |
| 173 | G->A | 53 | G->T | 215 | T->G | 200 | Stop at 246 | 73 | G->T |
| 242 | G->T | 60 | C->T | 247 | Stop at 262 | 239 | del | 231 | C->A |
| 190 | Fr. | 202 | G->T | 104 | C->T | 215 | A->T | 172 | Stop at 173 |
| 246 | T->C | 204 | A->G | 297 | A->C | 147 | Stop at 169 | 211 | Stop at 214 |
| 157 | G->T | 265 | T->A | 252 | T->C | 276 | G->A | 150 | Stop at 180 |
| 239 | Fr. | 135 | T->C | 276 | C->T | 210 | A->C | 145 | C->A |
| 240 | A->T | 147 | G->A | 349 | A->C | 182 | T->C | 335 | C->G |
| | | | | | G->A/T- | | | | G->A/A- |
| 238 | T->C | 153 | C->T | 173 | >G/G->T | 161 | G->A/C->T | 285 | >G |
| 35 | Stop at 42 | 170 | G->T | 225 | G->C | 83 | C->A | 85 | Stop at 122 |
| 47 | C->T | 260 | C->T | 250 | del | 304 | Stop at 344 | 98 | C->T |
| 89 | Fr. | 255 | Stop at 263 | 224 | A->G | 225 | ins | 113 | C->T |
| 102 | Fr. | 139 | G->C | 166 | T->A | 314 | Stop at 344 | 87 | Stop at 148 |
| 141 | C->G | 234 | A->C | 156 | C->A | 301 | A->G | 97 | C->T |
| 144 | C->T | 152 | C->A | 291 | A->G | 224 | G->A | 217 | Stop at 246 |
| 146 | G->A | 170 | C->T | 305 | A->G | 112 | C->G | 226 | G->T |
| 158 | G->T | 175 | G->C | 306 | A->T | 163 | C->A | 278 | T->A |
| 161 | G->A | 240 | A->G | 296 | C->T | 299 | T->A | 145 | C->T |
| 164 | G->T | 259 | G->T | 267 | del | 251 | del | 133 | Stop at 148 |
| 165 | Ins | 87 | Fr. | 151 | Stop at 169 | 162 | Stop at 180 | 136 | A->C |
| 176 | C->G | 142 | Fr. | 228 | Stop at 238 | 44 | G->T | 239 | A->T/C->A |
| | | | | | | | | | G->A/C- |
| 191 | Fr. | 175 | C->G | 165 | Stop at 180 | 177 | C->A/C->T | 245 | >A |
| 215 | G->T | 126 | A->G | 176 | C->A | 236 | A->C | 252 | T->A |
| | | | | | | | | | G->A/C- |
| 217 | G->T | 128 | T->G | 192 | A->G | 243 | T->C | 244 | >A |
| 220 | A->G | 128 | C->T | 167 | Ins | 137 | G->A | 299 | Stop at 305 |
| 224 | G->C | 134 | T->C | 166 | T->C | 218 | G->C | 305 | A->T/G->A |
| 242 | C->G | 172 | Fr. | 120 | A->G | 277 | G->C | 310 | A->C |
| 259 | A->T | 237 | T->A | 150 | C->A | 54 | Fr. | 322 | C->G |
| 267 | G->C | 193 | A->C | 155 | A->C | 40 | Ins | 323 | Stop at 344 |
| 291 | A->T | 213 | Fr. | 203 | Stop at 246 | 156 | Stop at 166 | 315 | C->G |
| 298 | G->T | 246 | G->A | 221 | A->G | 168 | C->G/A->T | 308 | G->C |
| 182 | C->A | 235 | Fr. | 50 | Stop at 109 | 249 | G->T/G->T | 323 | T->G |
| | Stop at | | | | | | | | |
| 233 | 239 | 329 | Fr. | 191 | Stop at 243 | 158 | C->A | 201 | T->A |
| 173 | T->C | 155 | A->G | 205 | T->C | 209 | G->T | 190 | T->C |
| 251 | A->C | 7 | G->C | 210 | Stop at 246 | 184 | Stop at 207 | 278 | T->C |
| 219 | Fr. | 56 | G->T | 110 | C->G | 146 | G->T | 305 | G->C |
| 280 | A->T | 104 | Fr. | 166 | C->A | 250 | C->A | 176 | del |
| | | | G->A/G- | | | | | | |
| 126 | T->A | 245 | >A | 269 | G->T | 74 | Stop at 122 | 217 | T->G |
| 132 | G->C | 317 | C->T | 155 | Stop at 179 225 | 225 | del | 174 | A->C |
| 181 | C->T | 125 | G->A | 155 | Stop at 169 | 253 | C->A | 289 | C->G |
| 184 | G->T | 214 | Fr. | 156 | Stop at 169 | 269 | INS | 234 | C->G |
| 220 | T->C | 248 | G->C | 162 | C->T | 184 | T->C | 232 | A->C |
| 266 | G->A | 307 | Ins | 196 | A->G | 304 | T->G | 317 | A->T |
| 279 | G->A | 152 | G->T | 213 | Stop at 245 | 204 | A->C | 132 | Fr. |
| 305 | Ins | 178 | C->G | 214 | C->T | 66 | Stop at 145 | 299 | Fr. |
| 220 | A->C | 253 | C->T | 269 | C->T | 259 | Stop at 263 | 158 | C->G/G->T |
| 284 | A->C | 270 | T->C | 287 | A->G | 263 | Stop at 271 | 142 | Stop at 169 |
| 280 | G->C | 281 | C->A | 313 | C->G | 280 | Stop at 344 | 203 | Stop at 207 |
| | Stop at | | | | | | | | |
| 172 | 231 | 216 | Fr. | 108 | Stop at 144 237 | 237 | Stop at 246 | 248 | G->C/G->C |
| | Stop at | | | | | | | | |
| 174 | 176 | 131 | Fr. | 321 | A->G | 289 | C->A | 256 | A->C |
| 224 | Ins | 141 | Ins | 244 | C->T | 315 | Stop at 344 | 262 | Stop at 343 |
| | Stop at | | | | | | | | |
| 251 | 344 | 140 | Fr. | 198 | Stop at 246 | 312 | C->T | 301 | Stop at 343 |
| 261 | del | 163 | T->A | 135 | Ins | 145 | C->G | 335 | G->A |
| 181 | G->A | 178 | A->C | 187 | Stop at 246 | 169 | G->T | 179 | Fr. |
| 265 | C->T | 186 | G->T | 264 | del | 184 | G->A | 341 | Stop at 344 |
| 272 | T->C | 208 | A->T | 52 | C->T | 364 | G->A | 103 | C->G |
| 136 | C->T | 255 | Fr. | 141 | G->C | 144 | del | 159 | Stop at 179 |
| 281 | G->T | 307 | G->A | 167 | A->G | 146 | Stop at 169 | 189 | Stop at 246 |
| 316 | C->T | 130 | T->G | 84 | C->T | 190 | C->A | 274 | Stop at 304 |
| 130 | C->G | 356 | G->T | 122 | Stop at 169 | 249 | A->C | 149 | Fr. |
| 234 | C->A | 43 | T->C | 140 | A->T | 214 | T->A | 183 | Stop at 183 |
| 368 | Fr. | 159 | G->C | 153 | Ins | 204 | G->A | 227 | Stop at 245 |
| | | | | | | | G->A/C- | | |
| 301 | Fr. | 280 | lns | 173 | Fr. | 242 | >G | 292 | Stop at 343 |
| 148 | Fr. | 327 | Fr. | 186 | Fr. | 208 | Stop at 241 | 178 | A->G |
| 176 | G->T | 87 | C->A | 152 | C->G | 158 | Stop at 180 | 251 | Stop at 343 |
| 152 | C->T | 156 | G->T | 171 | A->G | 217 | Stop at 221 | 252 | Stop at 263 |
| 248 | C->G | 158 | C->G | 180 | G->T | 262 | Stop at 344 | 64 | Fr. |
| 255 | T->G | 161 | G->T | 202 | G->A | 239 | Stop at 246 | 89 | Stop at 122 |
| 271 | Fr. | 173 | Stop at 180 | 227 | T->G | 205 | Stop at 246 | 108 | Stop at 122 |
| 274 | Fr. | 199 | G->T | 298 | G->A | 214 | T->C | 110 | Ins |
| 225 | G->A | 144 | Fr. | 303 | G->A | 297 | ins | 124 | Stop at 124 |
| 176 | T->A | 233 | Fr. | 261 | Ins | 268 | Fr. | 285 | del |
| 135 | Fr. | 275 | T->G | 276 | C->G | 256 | A->T | 342 | del |
| 135 | C->G | 162 | T->G | 305 | Fr. | 223 | C->A | 313 | A->T |
| 151 | C->T | 178 | Fr. | 117 | Stop at 122 26 | 26 | Stop at 43 | 217 | T->A |
| 159 | C->T | 256 | Fr. | 155 | Stop at 177 | 186 | A->T | 167 | Stop at 169 |
| 179 | A->G | 225 | Fr. | 277 | T->A | 214 | Stop at 246 | 278 | C->T/T->C |
| 306 | C->T | 148 | T->A | 298 | A->C | 245 | C->A | 290 | Stop at 304 |
| 174 | G->A | 187 | G->A | 141 | C->T | 287 | G->C | 173 | Stop at 173 |
| | | | C->A/C- | | | | | | |
| 208 | Fr. | 250 | >A | 115 | T->C | 96 | C->T | 259 | C->T |
| 126 | Fr. | 254 | T->G | 119 | G->A | 164 | Stop at 166 | 288 | T->A |
| 173 | del | 257 | T->C | 120 | Fr. | 255 | Ins | 207 | T->A |
| 192 | C->T | 275 | T->C | 127 | T->A | 275 | del | 197 | Stop at 208 |
| | | | G->T/G- | | | | | | |
| 209 | Fr. | 216 | >T | 133 | Fr. | 284 | ins | 214 | A->T |
| 216 | T->G | 149 | T->C | 144 | A->T | 161 | G->C | 127 | C->G |
| 258 | G->A | 240 | G->T | 187 | T->C | 246 | A->T/G->T | 337 | G->C |
| 282 | G->C | 65 | A->T | 205 | T->A | 199 | G->C | 102 | Stop at 122 |
| 308 | Fr. | 125 | C->T | 209 | A->G | 195 | Stop at 246 | 187 | Stop at 202 |
| 332 | Fr. | 166 | C->T | 237 | A->T | 275 | Fr. | 100 | A->G |
| 173 | T->G | 242 | C->T | 337 | G->T | 283 | Stop at 305 | 140 | Stop at 143 |
| 249 | Fr. | 263 | A->C | 342 | G->A | 233 | ins | 176 | Stop at 179 |
| 275 | G->A | 139 | G->T | 377 | C->A | 127 | Stop at 169 | 235 | Ins |
| 294 | G->T | 165 | A->T | 93 | C->T | 138 | G->A | 250 | Stop at 262 |
| 316 | Fr. | 241 | T->G | 202 | C->T | 208 | A->T/C->T | 284 | Stop at 305 |
| 159 | C->A | 255 | T->A | 199 | Stop at 246 | 106 | del | 132 | G->A |
| 118 | Ins | 265 | Fr. | 252 | C->T | 245 | G->C/G->T | 129 | C->T |
| 277 | G->A | 279 | Fr. | 254 | C->T | 212 | Stop at 246 | 210 | C->T |
| 244 | G->C | 241 | Fr. | 262 | G->A | 133 | G->A | 232 | C->T |
| 264 | Fr. | 151 | C->G | 263 | A->G | 124 | T->C | 257 | C->T |
| | C->T/C- | | | | | | | | |
| 278 | >T | 156 | Fr. | 274 | Stop at 344 | 51 | Stop at 122 | 164 | Stop at 169 |
| 177 | C->T | 170 | A->T | 293 | Stop at 344 | 170 | G->A | 249 | del |
| 179 | C->T | 204 | G->T | 156 | C->G | 150 | del | 187 | Fr. |
| 281 | C->T | 249 | A->G | 157 | Stop at 169 | 85 | Stop at 143 | 210 | Fr. |
| 141 | G->A | 280 | G->T | 92 | C->T | 195 | T->A | 207 | T->C |
| | Stop at | | | | | | | | |
| 283 | 344 | 281 | A->C | 201 | G->T | 314 | Stop at 338 | 226 | G->C |
| | Stop at | | | | | | | | |
| 136 | 148 | 94 | T->A | 202 | Stop at 246 | 307 | Stop at 340 | 168 | C->G/C->G |
| 286 | G->A | 153 | C->A | 222 | C->T | 67 | Stop at 122 | 185 | A->G |
| 109 | C->A | 172 | T->C | 223 | Stop at 246 | 255 | Stop at 344 | 198 | Stop at 208 |
| 164 | A->G | 173 | T->A | 264 | Stop at 344 | 163 | del | 208 | G->C |
| | | | | | C->A/G- | | | | |
| 238 | G->C | 296 | C->G | 273 | >A | 191 | Stop at 246 | 331 | A->C |
| 110 | G->T | 284 | A->G | 316 | C->A | 255 | Stop at 257 | 320 | Stop at 336 |
| | | | | | | | | | A->C/G- |
| 113 | T->G | 135 | G->T | 271 | Ins | 262 | Stop at 263 | 331 | >A |
| 162 | C->G | 31 | G->A | 129 | Fr. | 264 | C->A | 338 | T->A |
| 183 | C->G | 72 | Fr. | 192 | Ins | 348 | G->T | 280 | Fr. |
| | Stop at | | | | | | | | |
| 287 | 344 | 91 | G->A | 307 | G->T | 232 | Stop at 246 | 290 | Fr. |
| 152 | G->A | 110 | Fr. | 220 | T->A | 170 | ins | 297 | Fr. |
| 138 | C->T | 154 | Fr. | 285 | A->G | 114 | T->A | 297 | C->G |
| 278 | C->G | 158 | Fr. | 226 | G->A | 343 | G->T | 136 | Stop at 164 |
| 236 | T->C | 167 | C->T | 137 | Ins | 26 | Stop at 36 | 149 | Stop at 180 |
| 237 | A->G | 178 | Stop at 180 | 259 | Ins | 137 | G->T | 221 | Stop at 246 |
| 289 | T->A | 195 | Stop at 208 | 234 | Fr. | 145 | G->C | 228 | ins |
| 237 | G->T | 197 | G->A | 135 | del | 146 | T->C | 243 | Stop at 340 |
| 136 | Ins | 199 | G->A | 102 | Stop at 116 | 286 | A->T | 292 | Stop at 304 |
| | Stop at | | | | G->A/A- | | | | |
| 99 | 147 | 227 | Ins | 324 | >G | 296 | A->T | 328 | del |
| | Stop at | | G->T/G- | | | | | | |
| 134 | 169 | 248 | >T | 27 | Fr. | 164 | G->A | 338 | Stop at 346 |
| | | | T->C/G- | | | | | | |
| 242 | T->C | 265 | >T | 162 | del | 148 | T->G | 243 | A->C |
| 193 | C->T | 272 | T->A | 277 | Ins | 274 | del | 348 | T->A |
| 188 | Fr. | 274 | T->G | 135 | T->G | 211 | Stop at215 | 304 | C->T |
| | Stop at | | | | | | | | |
| 152 | 169 | 349 | Fr. | 69 | C->G | 239 | A->T | 228 | G->T |
| 57 | Fr. | 203 | Fr. | 242 | T->G | 313 | ins | 370 | A->C |
| 281 | C->G | 205 | T->G | 157 | G->A | 327 | T->G | 149 | C->T/C->T |
| | Stop at | | | | | | | | |
| 260 | 263 | 205 | A->C | 198 | G->C | 211 | C->A | 158 | C->T/G->A |
| 132 | A->T | 246 | A->T | 157 | T->G | 246 | Stop at 246 | 240 | G->A |
| | Stop at | | | | | | | | |
| 249 | 263 | 282 | Stop at 305 | 279 | G->C | 163 | C->T | 258 | Stop at 263 |
| 167 | G->T | 133 | A->C | 134 | Ins | 252 | del | 317 | C->A |
| 17 | A->T | 162 | A->T | 239 | Stop at 263 | 129 | del | 262 | T->A |
| 24 | A->T | 174 | A->T | 168 | Stop at 169 | 215 | G->C | 263 | A->T |
| 175 | C->T | 253 | C->G | 134 | Fr. | 253 | A->C | 163 | T->G |
| 358 | G->A | 131 | A->G | 253 | A->T | 274 | Ins | 312 | Fr. |
| 175 | Ins | 137 | T->A | 254 | A->T | 154 | C->A | 301 | C->A |
| | | | | | | | | | G->A/G- |
| 115 | C->T | 141 | Fr. | 247 | A->G | 183 | C->T | 226 | >A |
| 103 | Fr. | 157 | T->A | 235 | A->T | 225 | T->A | 200 | A->C/A->C |
| 237 | Fr. | 157 | Fr. | 176 | Stop at 243 | 149 | ins | 207 | G->C |
| 250 | C->T | 176 | Ins | 163 | Stop at 169 | 171 | Fr. | 226 | Stop at 227 |
| 365 | A->G | 240 | Fr. | 248 | Stop at 344 | 287 | A->T | 266 | |
| 271 | G->A | 274 | T->C | 289 | Stop at 304 | 133 | G->T | 113 | del |
| 320 | G->C | 46 | Fr. | 163 | Fr. | 137 | C->A | 226 | Fr. |
| 349 | G->T | 112 | Fr. | 207 | Fr. | 148 | G->T | 94 | C->A |
| 126 | del | 295 | C->T | 251 | A->T | 246 | A->C | 127 | Fr. |
| 36 | G->A | 193 | T->A | 112 | Stop at 120 | 251 | T->C | 133 | Stop at 145 |
| 76 | Fr. | 221 | G->T | 120 | Stop at 122 | 273 | T->C | 153 | Stop at 180 |
| 241 | C->G | 227 | Fr. | 231 | C->T | 297 | C->A | 75 | C->T |
| 281 | G->C | 241 | C->A | 212 | Stop at 214 | 192 | G->A | 116 | Stop at 122 |
| 244 | G->A | 281 | G->A | 179 | A->C | 244 | C->G | 184 | del |
| 218 | T->G | 316 | Ins | 174 | G->T | 221 | Stop at 222 | 106 | Stop at 122 |
| | Stop at | | | | | | | | |
| 256 | 344 | 344 | Fr. | 232 | del | 243 | T->A | 69 | Stop at 147 |
| | A->C/G- | | | | | | | | |
| 280 | >C | 145 | T->G | 173 | Stop at 195 | 308 | C->G | 298 | Stop at 344 |
| 258 | A->G | 145 | T->C | 273 | Stop at 344 | 189 | C->A | 182 | ins |
| 270 | T->A | 194 | T->C | 143 | T->A | 239 | | 133 | del |
| 176 | T->G | 162 | A->G | 161 | C->T | 142 | C->G | 163 | Stop at 168 |
| | Stop at | | | | | | | | |
| 171 | 231 | 315 | Ins | 72 | Stop at 120 | 295 | C->A | 174 | del |
| | Stop at | | | | | | | | |
| 251 | 263 | 203 | T->A | 265 | del | 156 | Stop at 168 | 330 | T->G |
| 337 | C->T | 273 | Ins | 214 | Stop at 214 | 213 | Stop at 245 | 125 | C->G |
| 266 | G->C | 62 | G->T | 107 | Stop at 147 | 243 | Stop at 244 | 258 | Stop at 344 |
| 203 | G->C | 71 | Fr. | 317 | Ins | 289 | C->T | 330 | Stop at 335 |
| | Stop at | | | | | | | | |
| 241 | 252 | 128 | Fr. | 165 | C->A | 211 | del | 113 | T->C |
| 193 | A->T | 203 | T->C | 99 | Stop at 122 | 220 | Stop at 244 | 265 | T->G |
| 255 | A->G | 254 | C->G | 36 | C->T | 229 | T->G | 126 | T->C |
| 194 | C->G | 282 | Fr. | 245 | Ins | 253 | del | 214 | Stop at 218 |
| | Stop at | | | | | | | | |
| 342 | 342 | 258 | G->C | 76 | C->T | 302 | Stop at 303 | 284 | C->T |
| 55 | Ins | 217 | Fr. | 160 | T->A | 208 | A->G | 96 | ins |
| 257 | C->G | 139 | A->C | 165 | A->C | 212 | Stop at 244 | 62 | Stop at 121 |
| 282 | ins | 215 | A->C | 269 | Fr. | 129 | Stop at 145 | 285 | A->C |
| | | | | | G->T/C- | | | | |
| 245 | G->C | 243 | Ins | 245 | >A | 190 | del | 358 | G->T |
| 209 | ins | 295 | Fr. | 208 | G->A | 216 | Stop at 221 | 122 | G->A |
| 239 | ins | 285 | A->T | 236 | C->T | 275 | Stop at 304 | 69 | Stop at 122 |
| 179 | T->G | 170 | Stop at 179 | 294 | G->A | 150 | ins | 155 | C->T/C->G |
| | | | | | | | | | G->A/C- |
| 314 | C->T | 208 | Stop at 246 | 251 | Fr. | 188 | C->G | 245 | >G |
| 155 | C->T | 209 | Stop at 214 | 215 | Ins | 220 | Ins | 181 | C->G |
| | Stop at | | | | | | | | |
| 249 | 344 | 240 | Stop at 263 | 154 | C->T | 292 | A->C | 185 | Ins |
| 116 | C->G | 141 | G->T | 293 | G->C | 305 | G->T | 52 | Stop at 56 |
| 163 | A->G | 151 | Fr. | 161 | C->G | 48 | A->T/C->T | 112 | Stop at 122 |
| 173 | G->C | 182 | Stop at 246 | 56 | G->A | 154 | C->G | 165 | Stop at 169 |
| 255 | C->T | 140 | A->G | 139 | G->A | 150 | Fr. | 323 | del |
| 255 | C->G | 142 | C->T | 222 | G->C | 329 | C->A | 67 | C->G |
| 218 | T->C | 169 | T->A | 302 | Stop at 344 | 80 | C->T | 148 | A->T |
| | Stop at | | | | | | | | |
| 301 | 344 | 170 | A->G | 144 | Stop at 169 | 243 | T->G | 230 | Stop at 246 |
| 271 | A->T | 271 | A->G | 166 | T->G | 104 | Stop at 148 | 95 | Stop at 148 |
| 286 | A->G | 331 | C->T | 149 | T->A | 117 | Stop at 148 | 276 | Stop at 286 |
| 294 | A->G | 194 | Stop at 245 | 204 | del | 138 | C->A | 249 | Stop at 342 |
| | | | T->G/T- | | | | | | |
| 264 | C->T | 113 | >G | 127 | C->A | 248 | C->T/G->C | 208 | del |
| 235 | A->G | 165 | C->T | 289 | T->C | 167 | G->A | 163 | Stop at 163 |
| 249 | A->T | 176 | Stop at 246 | 261 | A->G | 214 | C->G | 213 | A->G |
| 216 | G->A | 207 | T->G | 269 | A->G | 272 | Fr. | 282 | Stop at 304 |
| 215 | G->A | 297 | C->T | 128 | Stop at 169 | 186 | A->G | 295 | Stop at 344 |
| 272 | G->A | 141 | T->G | 159 | Stop at 169 | 147 | Ins | 160 | del |
| | Stop at | | | | | | | | |
| 267 | 344 | 181 | G->C | 204 | Ins | 261 | T->G | 233 | A->T |
| 242 | G->A | 229 | Fr. | 242 | Stop at 242 | 240 | T->G | 186 | T->C |
| 195 | T->C | 276 | Fr. | 237 | del | 288 | Fr. | 243 | T->C/G->A |
| 172 | G->T | 149 | Stop at 169 | 284 | Stop at 304 | 286 | A->G/A->T | 142 | C->A |
| 239 | A->G | 193 | C->G | 331 | G->T | 126 | Stop at 169 | 144 | G->T |
| 262 | G->T | 293 | Stop at 304 | 130 | T->A | 182 | Fr. | 231 | Fr. |
| 255 | T->C | 147 | Fr. | 39 | C->T | 298 | Fr. | 254 | Fr. |
| 286 | A->C | 286 | G->T | 352 | G->C | 220 | T->G | 266 | ins |
| 283 | G->A | 287 | Stop at 303 | 209 | Stop at 246 | 269 | G->A | 258 | A->C |
| 190 | C->T | 293 | G->A | 90 | C->T | 232 | Fr. | 239 | Stop at 261 |
| 154 | G->A | 295 | T->C | 111 | G->A | 131 | del | 262 | G->C/G->C |
| | Stop at | | | | | | | | |
| 272 | 344 | 215 | Fr. | 119 | C->T | 261 | Fr. | 296 | Stop at 334 |
| 143 | G->A | 333 | Fr. | 141 | T->C | 111 | T->A | 284 | del |
| 271 | A->C | 28 | A->C | 202 | T->C | 285 | Fr. | 150 | A->C |
| 133 | T->A | 67 | C->T | 326 | A->G | 266 | G->A/A->T | 225 | G->T |
| 174 | Fr. | 288 | A->G | 36 | G->T | 162 | Stop at 169 | 247 | Fr. |
| 132 | A->G | 276 | del | 68 | A->G | 208 | Ins | 322 | C->T |
| 252 | Fr. | 292 | Fr. | 117 | G->T | 250 | Ins | 85 | Stop at 117 |
| 330 | T->A | 189 | C->T | 145 | G->T | 130 | Stop at 169 | 86 | ins |
| | | | | | G->A/T- | | | | |
| 179 | C->A | 210 | A->G | 215 | >A | 289 | Fr. | 189 | Fr. |
| 309 | C->G | 217 | T->C | 325 | G->A | 198 | Fr. | 315 | Fr. |
| 212 | ins | 135 | Stop at 169 | 112 | G->A | 302 | Fr. | 169 | Stop at 180 |
| 175 | G->T | 165 | A->G | 308 | G->A | 137 | C->T | 245 | G->T/G->T |
| | | | | | | | | | C->A/G- |
| 153 | C->G | 234 | del | 63 | C->T | 191 | Ins | 175 | >C/C->G |
| | C->G/G- | | | | | | | | |
| 145 | >T | 218 | del | 104 | A->T | 186 | G->A | 71 | C->T |
| 277 | Fr. | 100 | C->T | 212 | T->C | 237 | ins | 72 | Stop at 148 |
| 275 | G->T | 169 | G->A | 217 | G->A | 230 | A->G | 98 | T->A |
| 110 | C->T | 158 | Stop at 179 | 328 | T->C | 184 | A->G | 287 | Stop at 304 |
| 232 | T->A | 143 | Stop at 169 | 45 | C->T | 157 | ins | 162 | A->G/C->T |
| | C->A/C- | | | | | | | | |
| 151 | >T | 200 | Ins | 299 | G->C | 95 | T->A/T->G | 130 | T->C |
| 218 | G->T | 185 | Stop at 246 | 111 | T->C | 314 | Fr. | 215 | Stop at 243 |
| 139 | A->G | 11 | G->A | 127 | T->C | 306 | A->G | 204 | Stop at 207 |
| 250 | C->G | 217 | G->C | 162 | Ins | 45 | C->A | 315 | Stop at 336 |
| 280 | A->C | 72 | Stop at 122 | 360 | G->T | 100 | Fr. | 33 | Stop at 43 |
| 127 | C->T | 105 | G->T | 257 | Ins | 162 | Fr. | 41 | Stop at 43 |
| 176 | G->C | 221 | G->A | 341 | T->G | 319 | Fr. | 80 | Stop at 120 |
| 274 | G->C | 253 | A->G | 242 | Stop at 246 | 113 | Fr. | 96 | Stop at 147 |
| 246 | T->G | 300 | Stop at 344 | 262 | del | 126 | C->A | 207 | Stop at 212 |
| | Stop at | | | | | | | | |
| 229 | 238 | 250 | Stop at 342 | 257 | T->G | 196 | Stop at 246 | 215 | Stop at 245 |
| | | | | | | | G->A/C. | | |
| 247 | A->C | 135 | T->A | 229 | T->C | 175 | >G | 224 | Stop at 246 |
| | | | C->T/C- | | | | | | |
| 290 | G->A | 159 | >T | 196 | G->A | 182 | T->G | 260 | del |
| | Stop at | | | | | | | | |
| 219 | 246 | 249 | G->A | 200 | A->G | 190 | C->G | 276 | Stop at 339 |
| | Stop at | | | | | | | | |
| 88 | 122 | 198 | A->G | 278 | Stop at 344 | 141 | Stop at 148 | 290 | Stop at 339 |
| 254 | T->C | 238 | T->G | 144 | G->C | 166 | Stop at 180 | 300 | Stop at 343 |
| 283 | C->G | 243 | A->T | 158 | Stop at 169 | 345 | Stop at 369 | 51 | Stop at 121 |
| 299 | G->A | 259 | G->A | 252 | Stop at 344 | 192 | C->A | 301 | Stop at 303 |
| 346 | G->A | 268 | A->G | 241 | Stop at 261 | 65 | Fr. | 236 | A->C/C->G |
| | | | | | C->T/G- | | | | |
| 116 | T->C | 287 | Fr. | 282 | >A | 185 | G->T | 83 | C->T |
| 150 | A->G | 302 | G->A | 276 | G->T | 181 | C->A | 237 | T->C |
| 95 | T->C | 189 | G->C | 196 | C->A | 190 | Stop at 208 | 156 | ins |
| 54 | T->A | 212 | Fr. | 193 | T->C | 155 | C->G | 128 | C->G |
| 256 | C->T | 51 | G->T | 160 | A->C | 242 | G->T/C->T | 243 | T->G/G->C |
| 309 | C->A | 160 | G->C | 243 | A->G | 269 | A->T | 133 | A->G |
| 109 | T->C | 207 | Ins | 206 | Stop at 246 | 283 | Fr. | 125 | C->A |
| 265 | T->C | 147 | T->G | 194 | T->A | 189 | G->A | 62 | A->G |
| | Stop at | | | | | | G->A/G- | | |
| 139 | 169 | 177 | Fr. | 212 | T->A | 244 | >C | 54 | C->T |
| 154 | G->T | 121 | Fr. | 169 | A->G | 138 | Stop at 148 | 84 | C->G |
| 179 | A->T | 147 | T->C | 183 | T->C | 188 | Stop at 208 | 202 | G->C/T->G |
| 255 | del | 160 | A->G | 77 | C->G | 246 | del | 319 | A->C |
| | Stop at | | | | | | | | |
| 342 | 344 | 230 | Fr. | 188 | Ins | 180 | G->C | 138 | C->G |
| | | | | | G->T/C- | | | | |
| 11 | G->C | 237 | A->C | 158 | >T | 175 | del | 229 | T->A/G->A |
| | Stop at | | | | | | | | |
| 121 | 122 | 47 | Stop at 121 | 194 | Stop at 207 | 290 | Stop at 301 | 101 | Stop at 122 |
| 34 | ins | 78 | Fr. | 253 | Ins | 271 | del | 278 | Stop at 304 |
| 53 | G->A | 81 | Stop at 122 | 360 | Stop at 369 | 156 | Stop at 180 | 339 | Fr. |
| 144 | A->C | 108 | Stop at 146 | 191 | C->G | 69 | C->A | 303 | G->T |
| 280 | A->G | 110 | G->C | 141 | T->A | 112 | C->A | 247 | Stop at 344 |
| 326 | G->T | 156 | C->T | 303 | A->T | 193 | C->A | 299 | Ins |
| | Stap at | | | | | | | | |
| 332 | 344 | 217 | del | 49 | Ins | 222 | Fr. | 293 | del |
| 256 | Ins | 242 | T->A | 62 | Stop at 141 | 228 | Stop at 245 | 247 | Stop at 343 |
| 283 | C->T | 245 | Stop at 340 | 103 | del | 145 | del | 5 | C->T |
| 232 | T->C | 251 | Ins | 105 | del | 148 | Stop at 167 | 123 | C->T |
| 184 | Fr. | 91 | G->T | 121 | del | 140 | Stop at 168 | 126 | C->T |
| 273 | C->G | 136 | A->G | 124 | Ins | 171 | Stop at 180 | 320 | G->A |
| 133 | T->C | 146 | G->C | 124 | Stop at 167 | 304 | Fr. | 356 | G->A |
| 272 | G->T | 164 | A->T | 338 | Stop at 343 | 159 | ins | 379 | G->A |
| 293 | G->T | 194 | Fr. | 336 | G->T | 261 | G->A | 154 | Stop at 180 |
| 267 | G->A | 255 | A->T | 124 | C->G | 304 | A->G | 164 | G->C |
| 325 | G->T | 339 | Ins | 284 | A->T | 222 | G->T | 75 | C->G |
| 71 | Ins | 35 | G->T | 144 | G->A | 291 | G->C | 163 | Stop at 165 |
| 120 | A->T | 213 | G->T | 227 | C->T | 147 | T->A/T->A | 238 | Stop at 244 |
| 151 | Ins | 261 | Stop at 263 | 208 | G->T | 216 | G->C | 8 | C->T |
| 307 | Fr. | 299 | T->C | 228 | G->A | 91 | Ins | 15 | A->C |
| 108 | Fr. | 204 | A->T | 196 | G->T | 311 | A->C | 61 | A->G |
| 257 | T->A | 47 | Fr. | 195 | C->G | 334 | Stop at 344 | 72 | C->T |
| | Stop at | | | | | | | | |
| 257 | 344 | 178 | C->A | 272 | T->G | 211 | T->C | 102 | ins |
| 138 | G->T | 257 | G->A | 53 | G->C | 197 | G->T | 104 | G->T |
| 155 | C->A | 341 | C->T | 290 | C->G | 202 | C->A | 106 | A->G |
| | | | | | A->T/A- | | | | |
| 167 | C->A | 290 | C->T | 292 | >T | 219 | C->A | 365 | C->T |
| 174 | A->G | 169 | T->C | 245 | Stop at 246 | 228 | G->C | 10 | C->T |
| 181 | G->T | 233 | C->T | 188 | Stop at 246 | 163 | A->C | 21 | C->T |
| | | | | | | | G->C/A- | | |
| 241 | T->A | 198 | G->A | 288 | Stop at 344 | 271 | >G | 361 | G->A |
| 305 | A->T | 200 | Fr. | 176 | Fr. | 238 | Fr. | 364 | C->T |
| 273 | C->A | 228 | C->G | 148 | Stop at 179 | 206 | T->A | 385 | T->C |
| 219 | C->T | 236 | C->A | 161 | Stop at 169 | 52 | del | 307 | A->G |
| | | | G->T/C- | | | | | | |
| 251 | C->G | 245 | >T | 211 | Stop at 246 | 94 | Stop at 122 | 161 | G->T/C->T |
| 233 | C->G | 249 | Ins | 244 | Stop at 246 | 236 | Stop at 236 | 241 | Stop at 263 |
| | Stop at | | | | | | | | |
| 215 | 246 | 251 | T->A | 247 | del | 107 | C->A | 327 | Stop at 335 |
| 216 | Ins | 258 | Fr. | 260 | Stop at 344 | 106 | Fr. | 157 | T->C |
| 344 | T->C | 278 | Ins | 216 | T->C | 69 | Fr. | 132 | Stop at 169 |
| 213 | G->C | 279 | Ins | 231 | A->T | 204 | Fr. | 221 | A->C |
| 82 | C->T | 296 | A->C | 208 | C->A | 305 | A->C/G->T | 184 | G->C |
| 151 | del | 255 | Stop at 343 | | C->G | 269 | Stop at 344 | 157 | Stop at 179 |
| 180 | G->A | 290 | Stop at 344 | 208 | C->G | 230 | Stop at 238 | 289 | Stop at 305 |
| 337 | G->A | 137 | Stop at 145 | 237 | G->C | 227 | Stop at 228 | 105 | G->C |
| 281 | A->T | 155 | Fr. | 243 | G->C | 363 | G->A | 215 | Stop at 221 |
| 133 | T->G | 206 | Ins | 159 | G->T | 253 | Fr. | 179 | Stop at 180 |
| 236 | del | 242 | Ins | 33 | Ins | 250 | Stop at 344 | 128 | Stop at 148 |
| 306 | G->C | 300 | Fr. | 192 | del | 259 | C->A | 256 | Stop at 263 |
| 227 | T->A | 191 | T->C | 312 | C->A | 167 | del | 131 | Stop at 169 |
| 138 | G->C | 191 | C->A | 321 | Stop at 344 | 173 | Stop at 246 | 143 | Stop at 167 |
| | Stop at | | | | C->G/C- | | | | |
| 178 | 246 | 246 | T->A | 283 | >G | 313 | Fr. | 158 | C->T/G->T |
| | C->T/A- | | | | | | | | |
| 213 | >G | 258 | A->T | 285 | G->T | 346 | Ins | 207 | A->T |
| 191 | Stop at | 143 | Ins | 283 | C->A | 293 | Ins | 245 | Stop at 262 |
| | 207 | | | | | | | | |
| 236 | A->G | 159 | Fr. | 216 | del | 224 | A->T | 258 | Stop at 291 |
| 196 | G->C | 165 | Stop at 178 | 318 | C->T | 158 | Stop at 167 | 266 | Stop at271 |
| | | | | | G->C/C- | | | | |
| 156 | G->A | 168 | Ins | 119 | >G/C->G | 154 | Stop at 167 | 284 | Stop at 344 |
| 339 | G->T | 169 | del | 344 | T->G | 283 | Stop at 304 | 285 | ins |
| 166 | C->G | 195 | T->G | 216 | Stop at 246 | 284 | C->A | 290 | ins |
| | Stop at | | | | | | | | |
| 184 | 246 | 191 | C->T | 240 | G->C | 176 | G->T/C->T | 294 | Stop at 344 |
| | Stop at | | | | | | | | |
| 279 | 344 | 152 | Ins | 306 | Stop at 344 | 47 | C->T/C->T | 308 | Stop at 344 |
| 140 | C->T | 168 | A->C | 210 | A->T | 151 | C->T/C->T | 49 | Stop at 50 |
| 282 | C->A | 209 | G->C | 198 | A->T | 88 | C->T/C->T | 254 | Stop at 260 |
| 162 | T->A | 209 | G->A | 273 | Fr. | 71 | C->A | 301 | Stop at 305 |
| 251 | C->T | 214 | T->G | 138 | del | 162 | T->C | 311 | Stop at 344 |
| | Stop at | | | | | | | | |
| 241 | 246 | 166 | Fr. | 171 | Stop at 173 | 180 | A->G | 320 | Stop at 344 |
| | G->A/G- | | | | | | | | |
| 248 | >A | 265 | Stop at 344 | 174 | Ins | 150 | Stop at 163 | 324 | Stop at 344 |
| | Stop at | | | | | | | | |
| 362 | 369 | 163 | C->G | 209 | A->C | 218 | Stop at 219 | 244 | Stop at 263 |
| 81 | C->T | 182 | T->A | 208 | Stop at 215 | 218 | ins | 265 | C->A |
| | G->A/G- | | | | | | | | |
| 224 | >A | 211 | Ins | 285 | Stop at 304 | 228 | C->A | 222 | Stop at 246 |
| 197 | T->G | 236 | T->A | 286 | G->C | 248 | Fr. | 296 | C->A |
| 301 | C->T | 267 | C->T | 130 | C->A | 282 | G->A/G->T | 205 | ins |
| 157 | G->C | 148 | G->A | 201 | Fr. | 61 | A->T | 77 | Fr. |
| 282 | G->A | 133 | Stop at 169 | 330 | Stop at 344 | 75 | T->C | 86 | G->T |
| 276 | C->A | 174 | Stop at 179 | 131 | Stop at 148 | 76 | G->A | 112 | C->T |
| | C->T/C- | | | | | | | | |
| 250 | >T | 239 | A->C | 236 | Stop at 239 | 295 | C->G | 320 | ins |
| 279 | G->T | 244 | Fr. | 236 | Ins | 340 | G->A | 125 | G->T |
| | C->T/C- | | | | | | | | |
| 219 | >T | 271 | G->T | 238 | del | 144 | A->G | 266 | Fr. |
| | C->T/C- | | | | | | | | |
| 152 | >T | 278 | Fr. | 313 | Stop at 334 | 126 | T->G | 135 | Stop at 148 |
| 157 | C->T | 171 | G->A | 240 | Stop at 262 | 234 | T->C/C->G | 316 | Stop at 336 |
| 158 | C->T | 229 | G->A | 266 | del | 165 | Fr. | 234 | T->A/C->A |
| | C->T/C- | | C->G/C- | | | | | | |
| 222 | >T | 161 | >A | 276 | Stop at 344 | 101 | A->T | 233 | C->T/A->C |
| 195 | C->T | 168 | C->A | 166 | Ins | 254 | Stop at 344 | 206 | T->A/G->T |
| | | | | | | | G->A/G- | | |
| 291 | G->T | 241 | T->C | 150 | Stop at 169 | 217 | >A | 226 | C->A |
| 192 | A->T | 154 | Ins | 222 | C->A | 169 | Fr. | 302 | G->C |
| | | | C->T/C- | | | | | | |
| 333 | G->A | 177 | >T | 218 | T->A | 167 | A->T | 260 | C->G |
| 369 | del | 202 | G->C | 228 | C->T | 148 | A->C | 220 | A->G/T->A |
| | | | C->T/C- | | | | | | |
| 203 | G->A | 250 | >G | 141 | C->A | 155 | Stop at 180 | 233 | Stop at 246 |
| 232 | C->G | 261 | A->T | 221 | G->C | 195 | del | 325 | G->C |
| 188 | G->A | 303 | G->C | 226 | C->T | 248 | del | 185 | A->T |
| 224 | Fr. | 182 | G->A | 215 | T->C | 275 | T->A | 186 | Stop at208 |
| 199 | A->G | 177 | C->A | 85 | C->T | 230 | Stop at 239 | 184 | Stop at 185 |
| 277 | T->C | 271 | G->C | 89 | C->T | 303 | Stop at 344 | 187 | Stop at 208 |
| 176 | T->C | 292 | A->T | 101 | A->G | 279 | del | 234 | C->T |
| 227 | T->C | 300 | C->T | 132 | A->T/A- | 153 | Fr. | 235 | Stop at 239 |
| | | | | | >G | | | | |
| 327 | T->C | 319 | A->T | 160 | G->A | 235 | A->C | 141 | Stop at 169 |
| 247 | C->T | 195 | Fr. | 46 | C->T | 82 | Stop at 145 | 264 | T->C |
| 135 | C->T | 269 | G->C | 122 | G->T | 196 | Fr. | 76 | A->T |
| 211 | C->T | 172 | T->G | 108 | G->A | 275 | ins | 96 | T->C |
| 149 | C->T | 35 | G->C | 103 | C->T | 82 | ins | 308 | C->A |
| | C->T/C- | | | | G->C/A- | | | | |
| 142 | >T | 90 | Fr. | 281 | >G/C->G | 273 | T->G | 96 | C->G |
| | C->T/C- | | | | | | | | |
| 138 | >T | 135 | C->A | 105 | C->T | 276 | Stop at 305 | 206 | G->A |
| | | | | | C->T/G- | | | | |
| 178 | C->T | 131 | A->T | 273 | >A | 254 | A->G | 241 | Ins |
| | C->T/C- | | | | | | | | |
| 241 | >T | 155 | del | 140 | Stop at 148 | 225 | T->G | 111 | T->G |
| 130 | C->T | 228 | Stop at239 | 270 | Stop at 337 | 246 | G->T | 238 | Ins |
| | C->T/C- | | | | | | | | |
| 127 | >T | 292 | Stop at 305 | 190 | Stop at 195 | 93 | G->A | 347 | C->G |
| 135 | G->A | 387 | del | 275 | Stop at 341 | 254 | C->A | 154 | G->T/C->T |
| 211 | A->G | 231 | A->G | 143 | Stop at 148 | 294 | Ins | 212 | T->G |
| 286 | Fr. | 268 | A->C | 107 | C->G | 139 | Fr. | 260 | C->A |
| 168 | Fr. | 179 | T->C | 194 | Stop at 206 | 356 | G->C | 123 | Stop at 148 |
| 175 | C->A | 232 | A->T | 271 | Stop at 343 | 182 | G->C | 146 | T->G |
| 381 | Fr. | 134 | T->G | 254 | del | 73 | Stop at 122 | 73 | T->A |
| 263 | Fr. | 274 | G->A | 255 | Stop at 262 | 76 | C->G | 165 | G->T |
| 292 | A->G | 243 | Stop at 246 | 322 | A->C | 150 | Stop at 165 | 313 | G->A |
| | | 131 | A->C | 323 | Stop at 340 | 214 | Stop at 220 | 130 | ins |
| | | | | | | | | 133 | A->T |
| | | | | | | | | 289 | Ins |

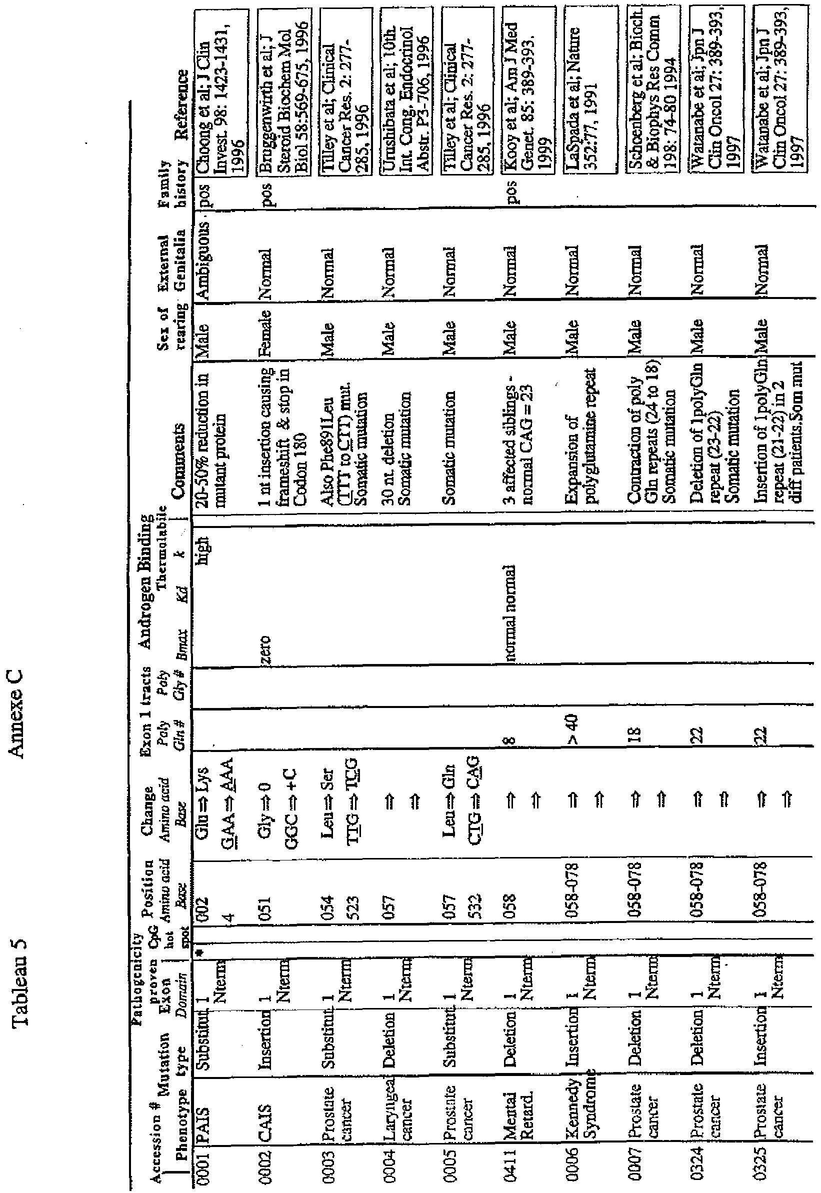

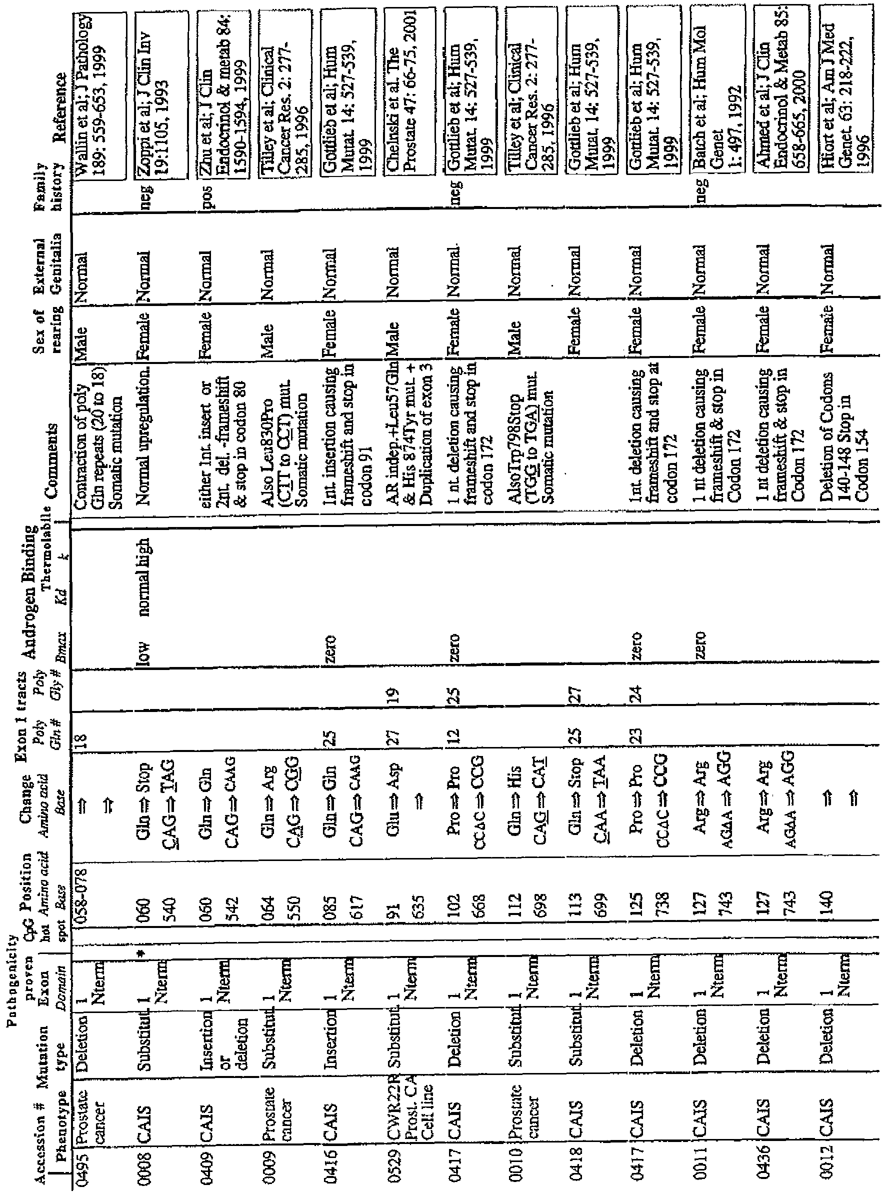

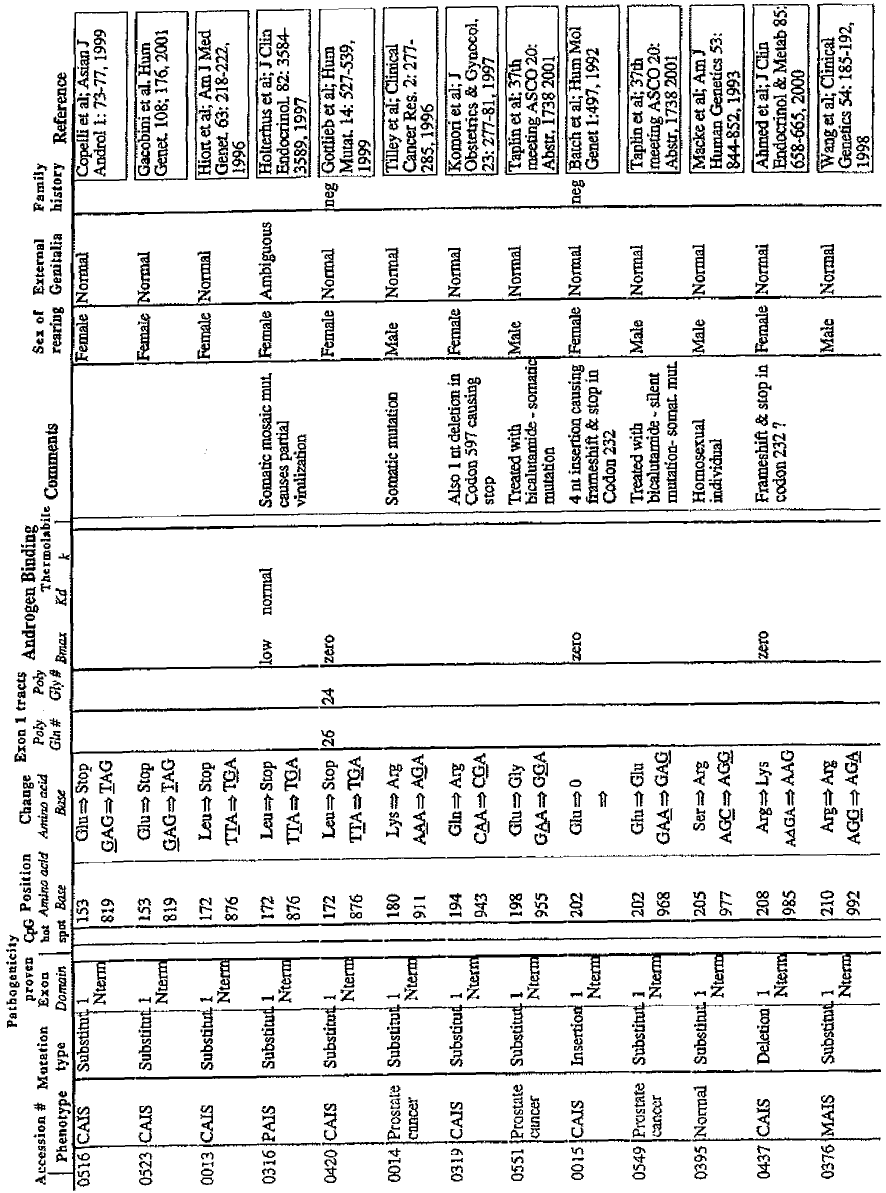

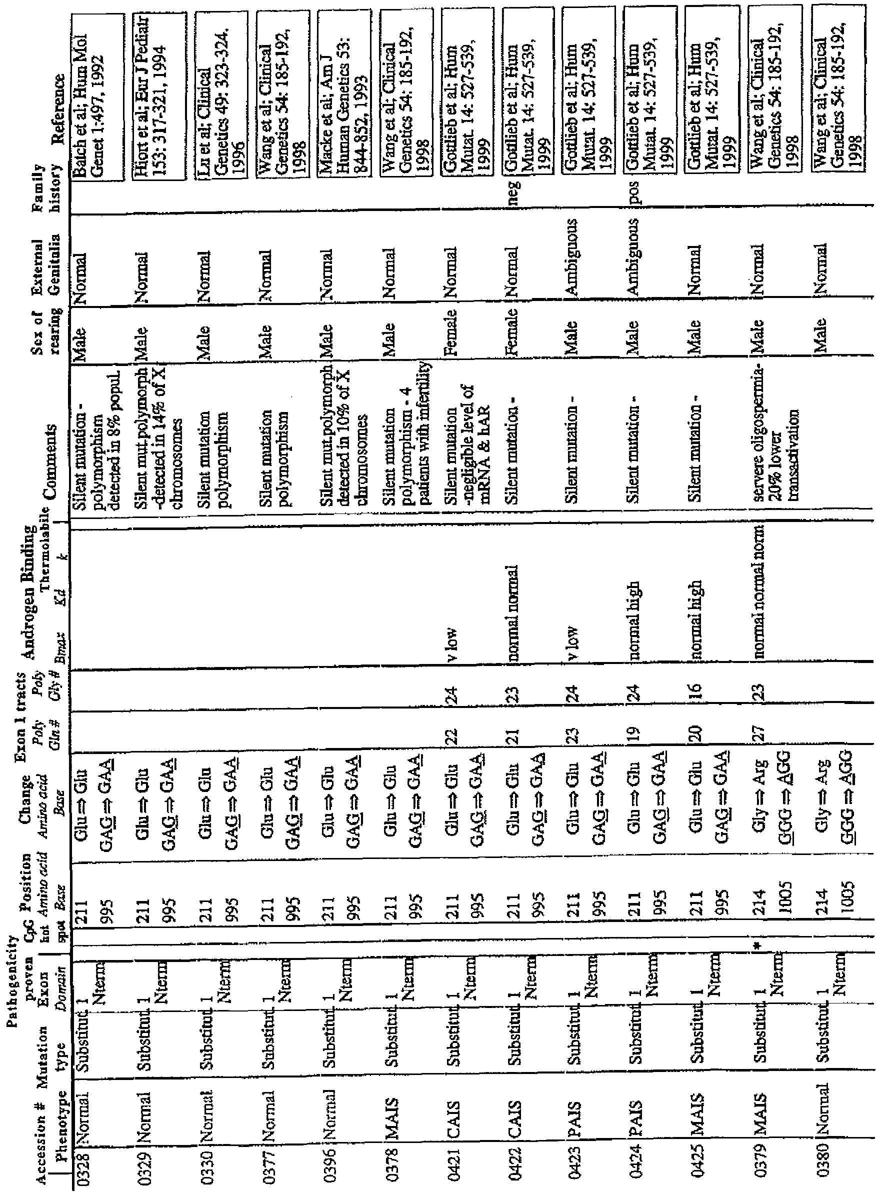

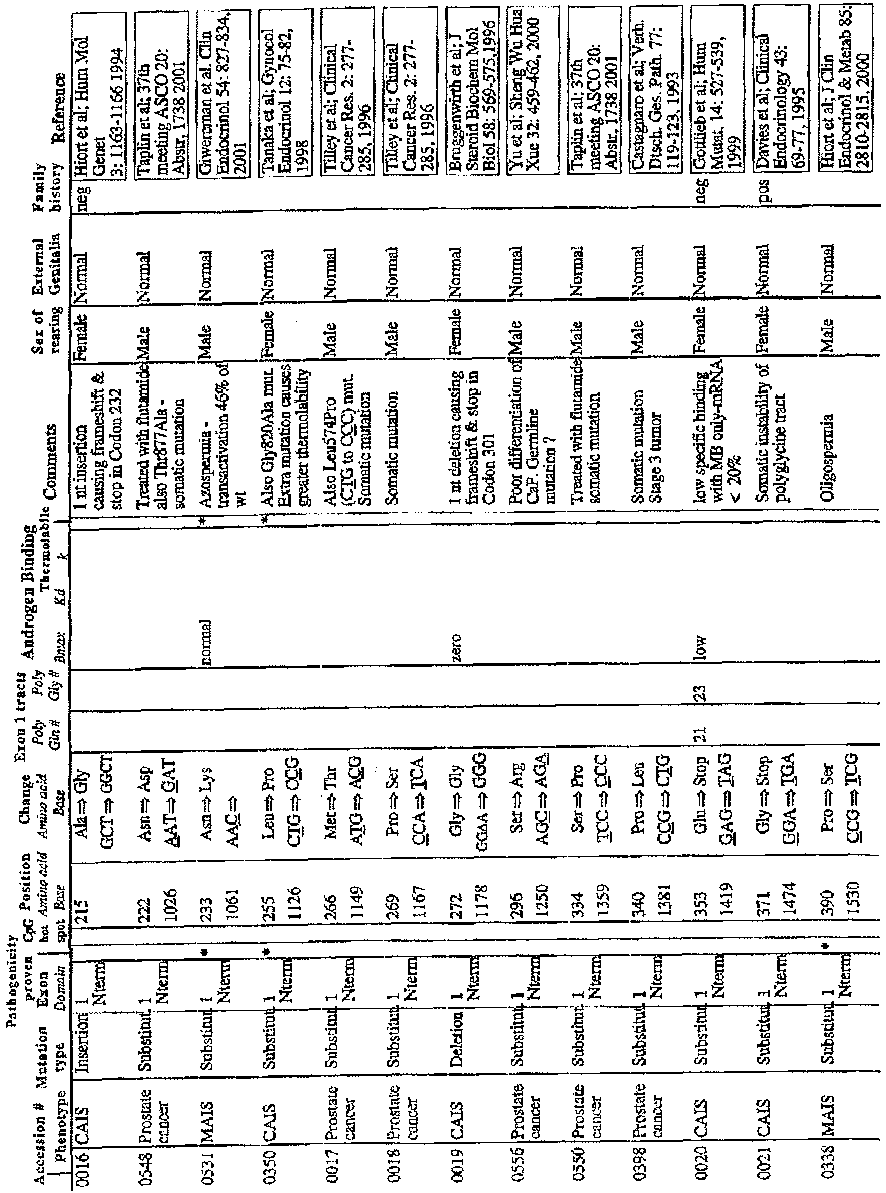

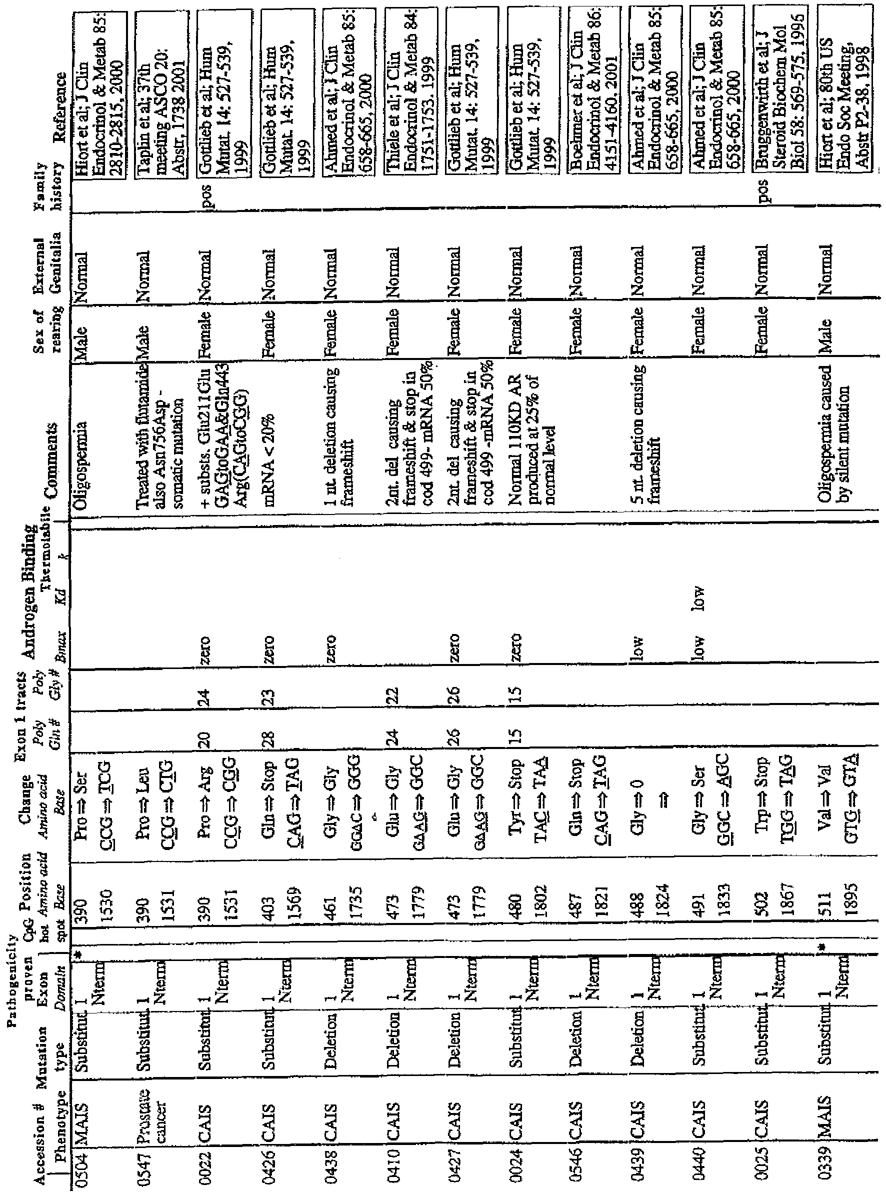

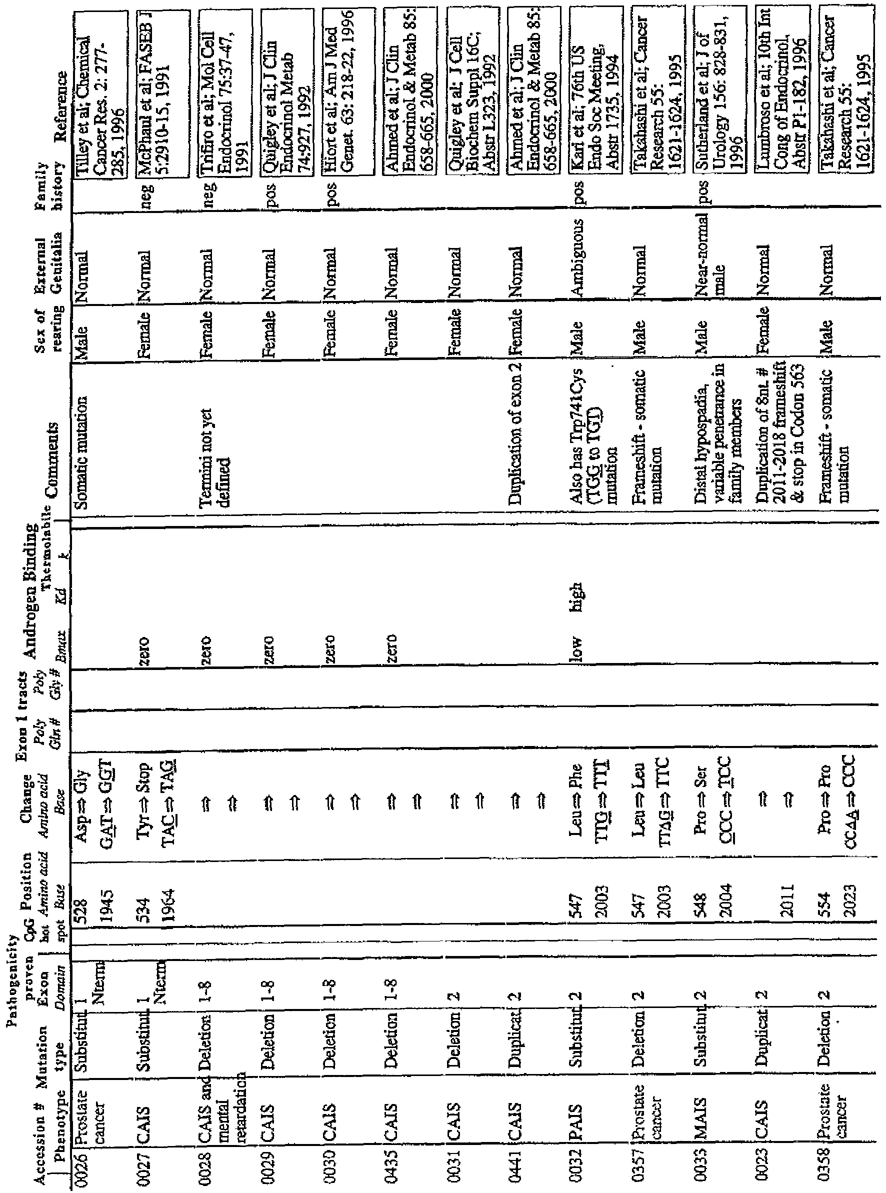

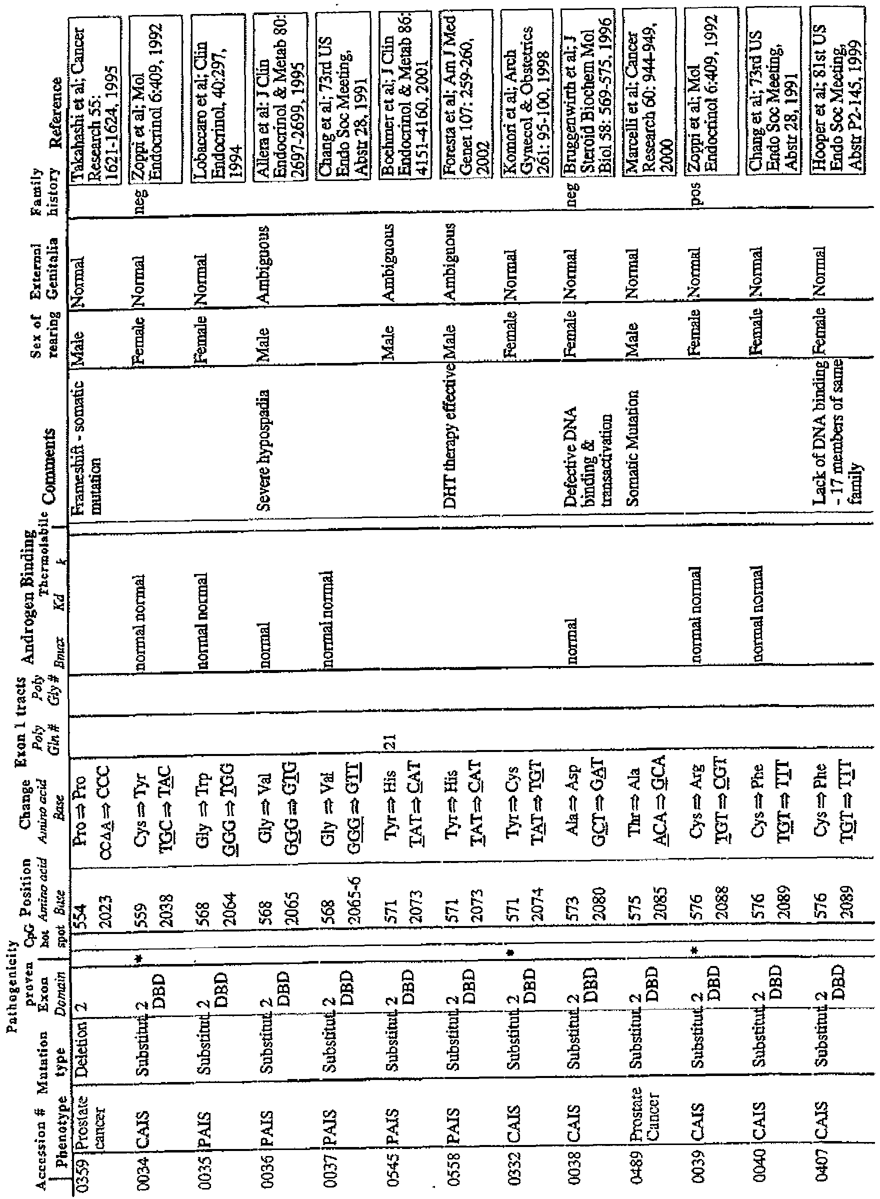

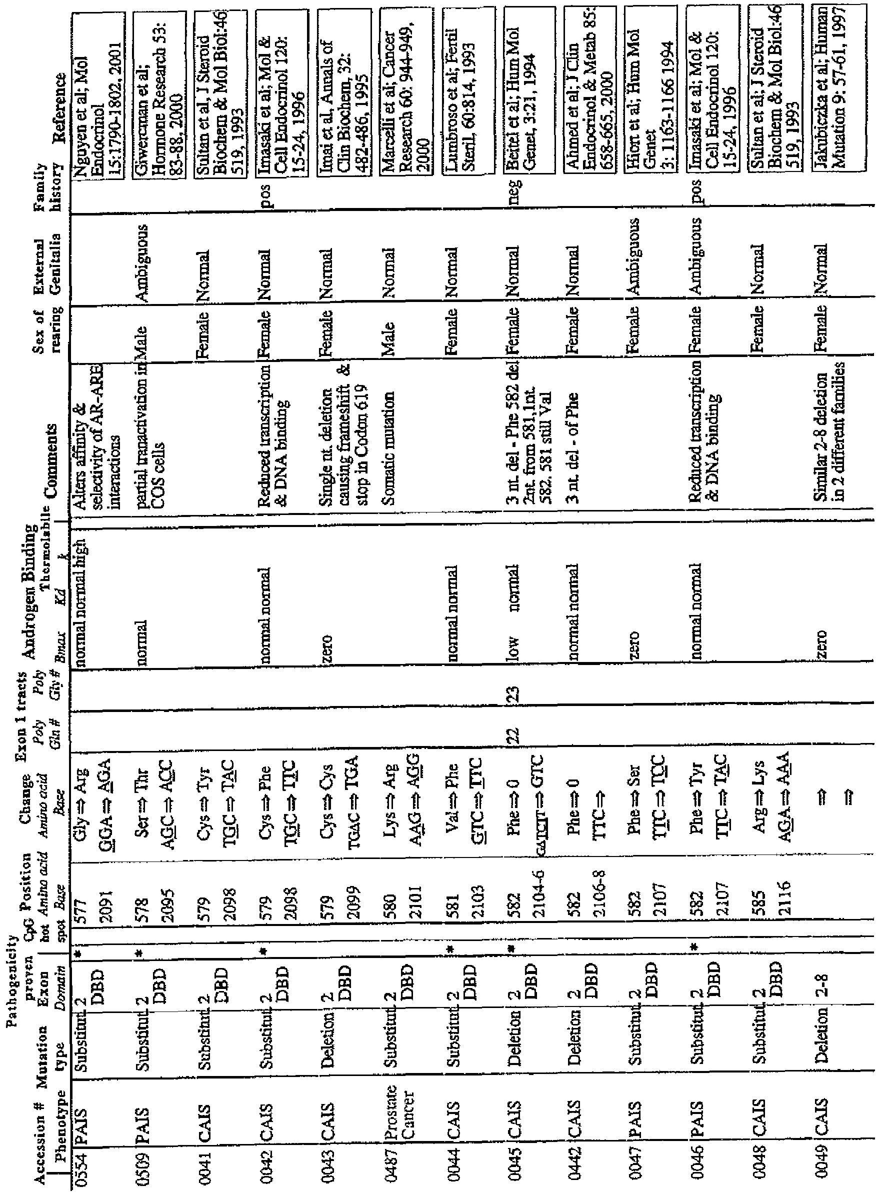

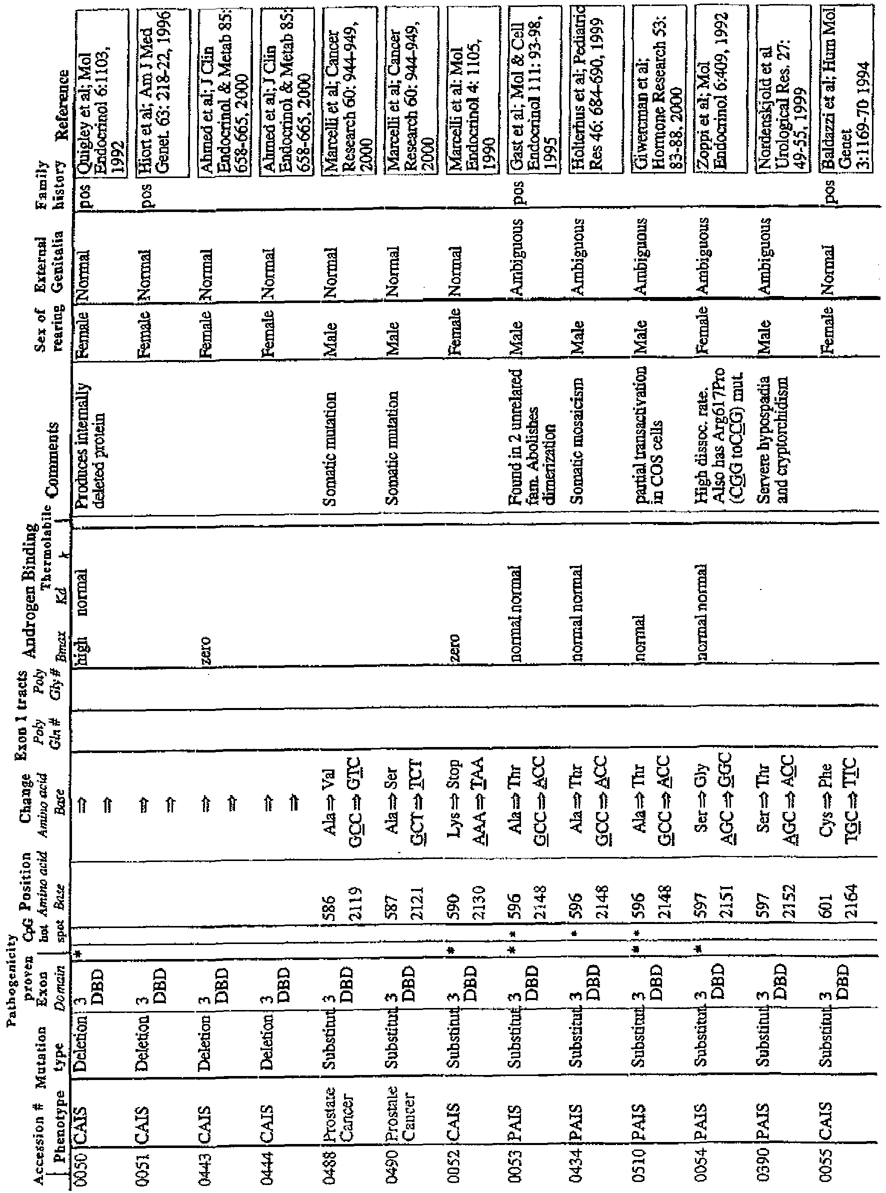

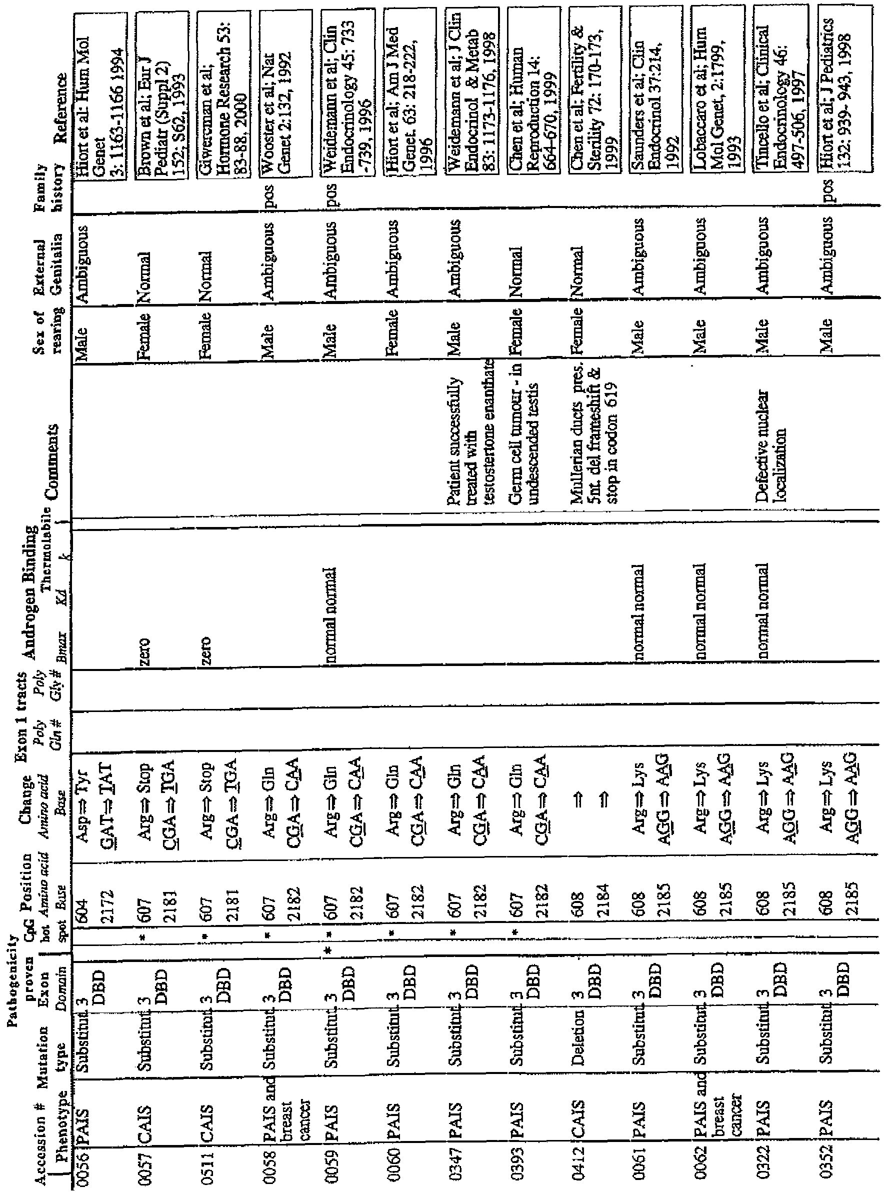

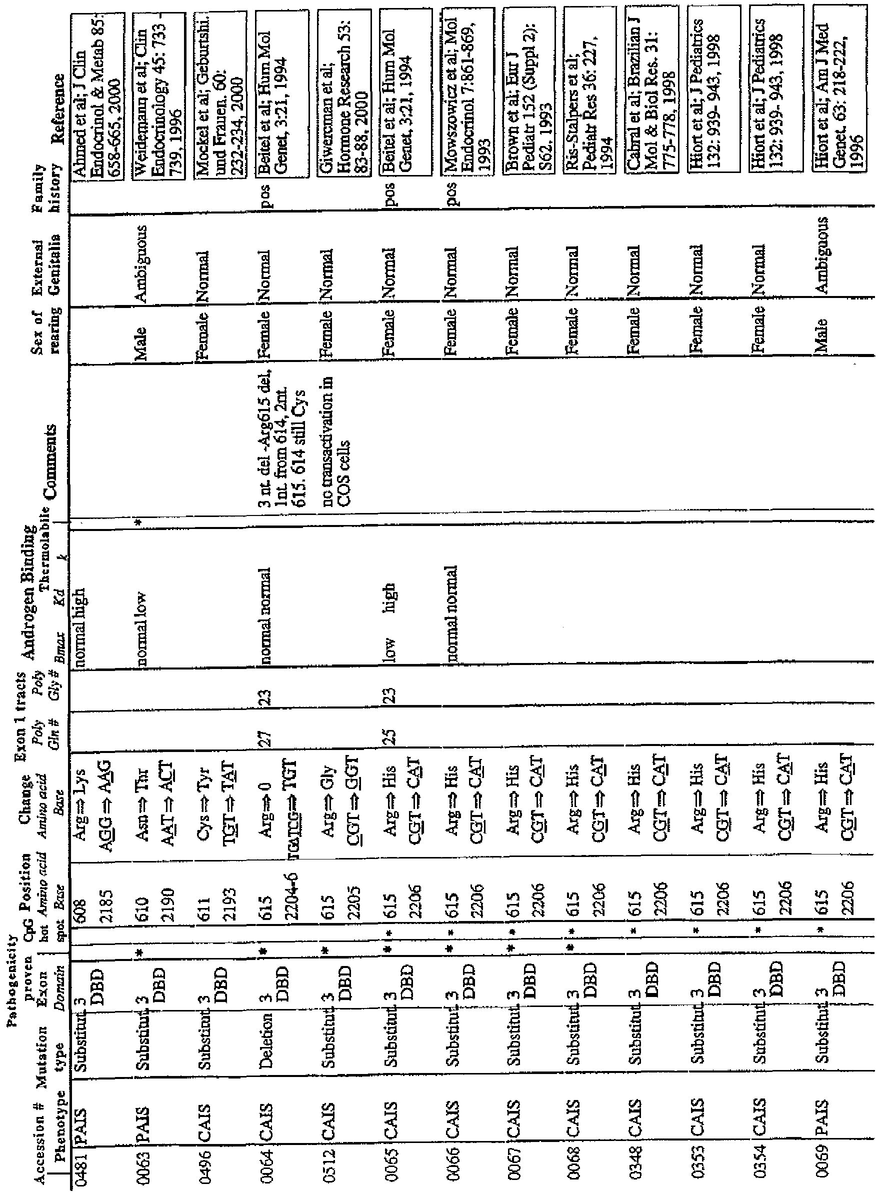

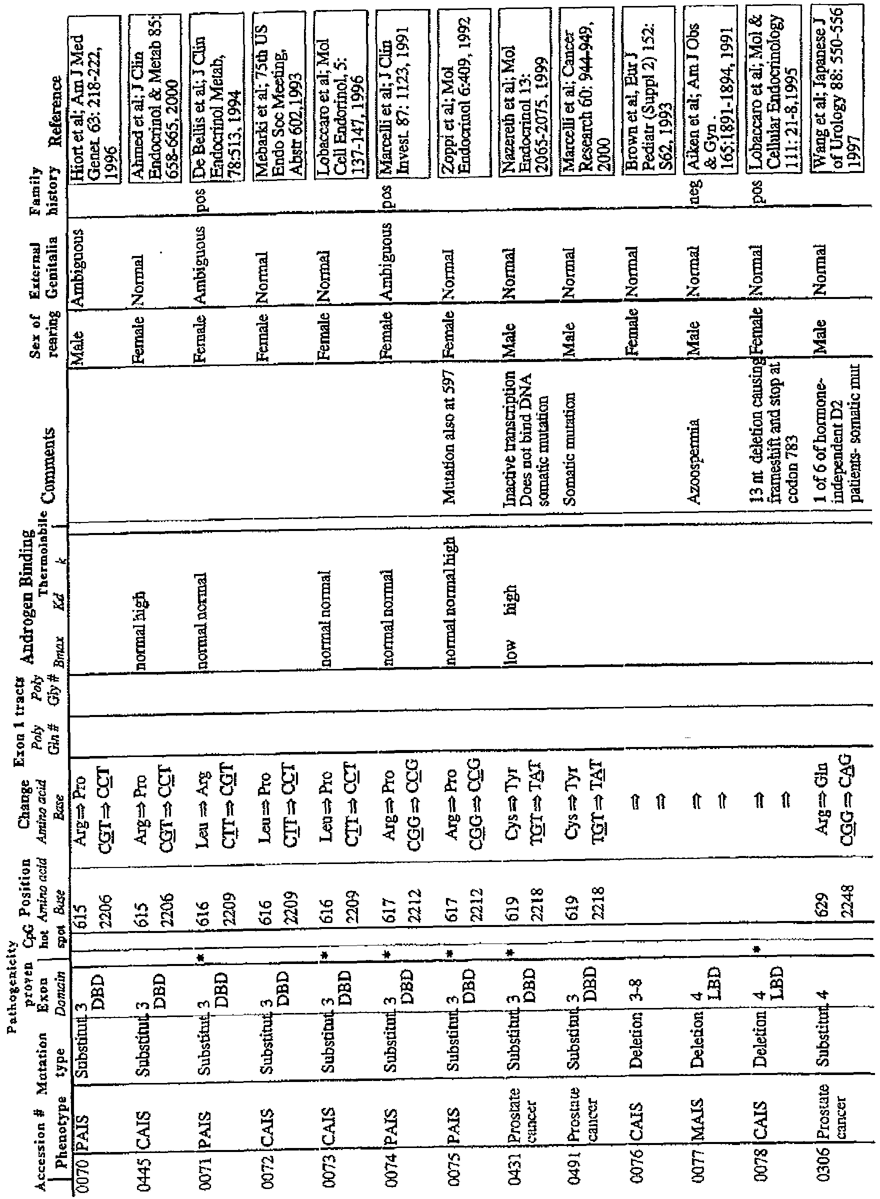

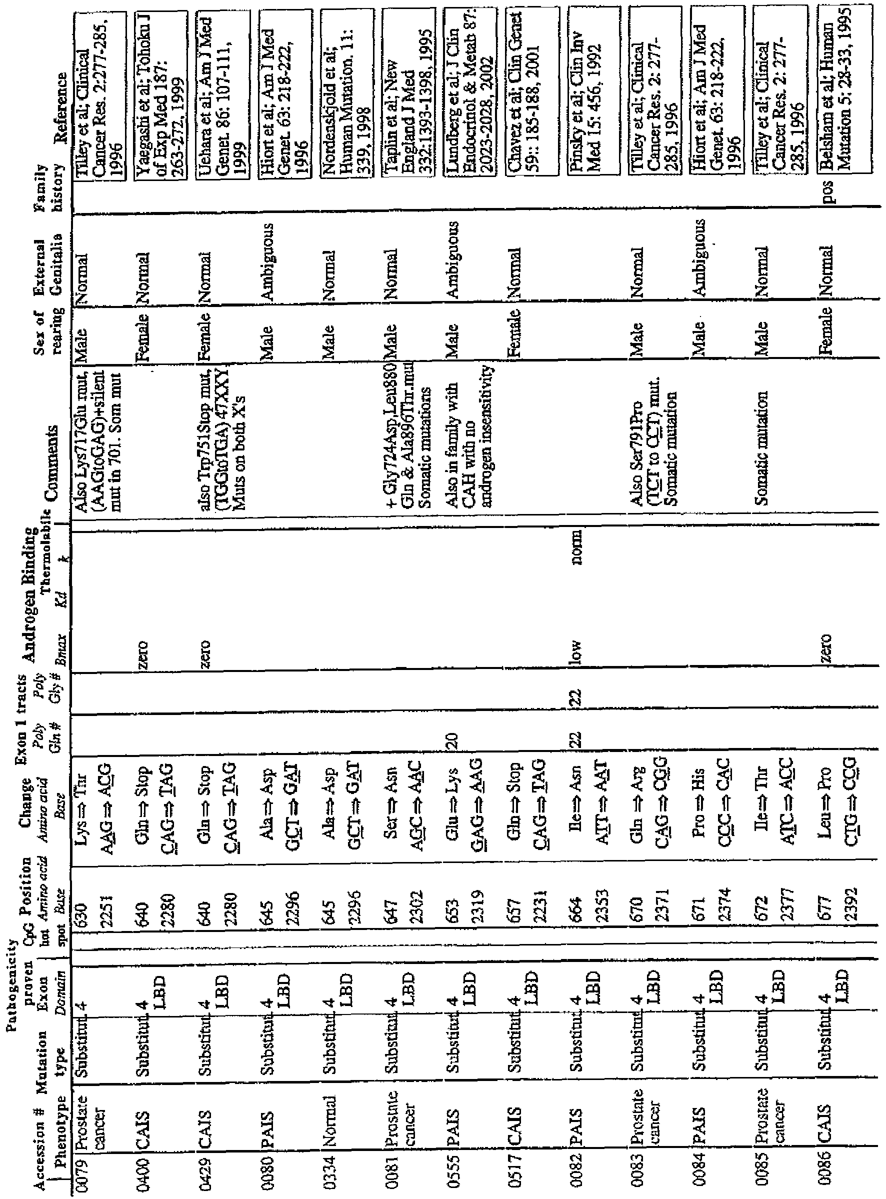

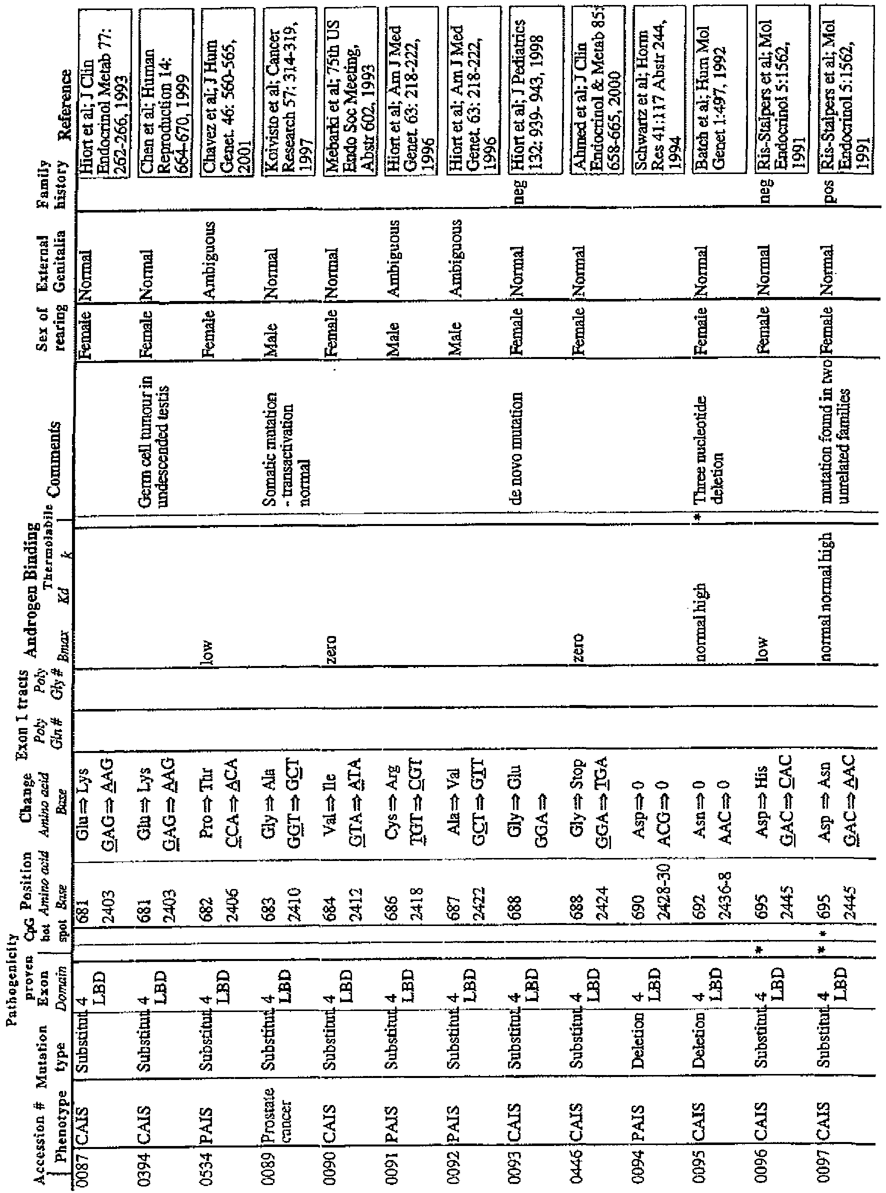

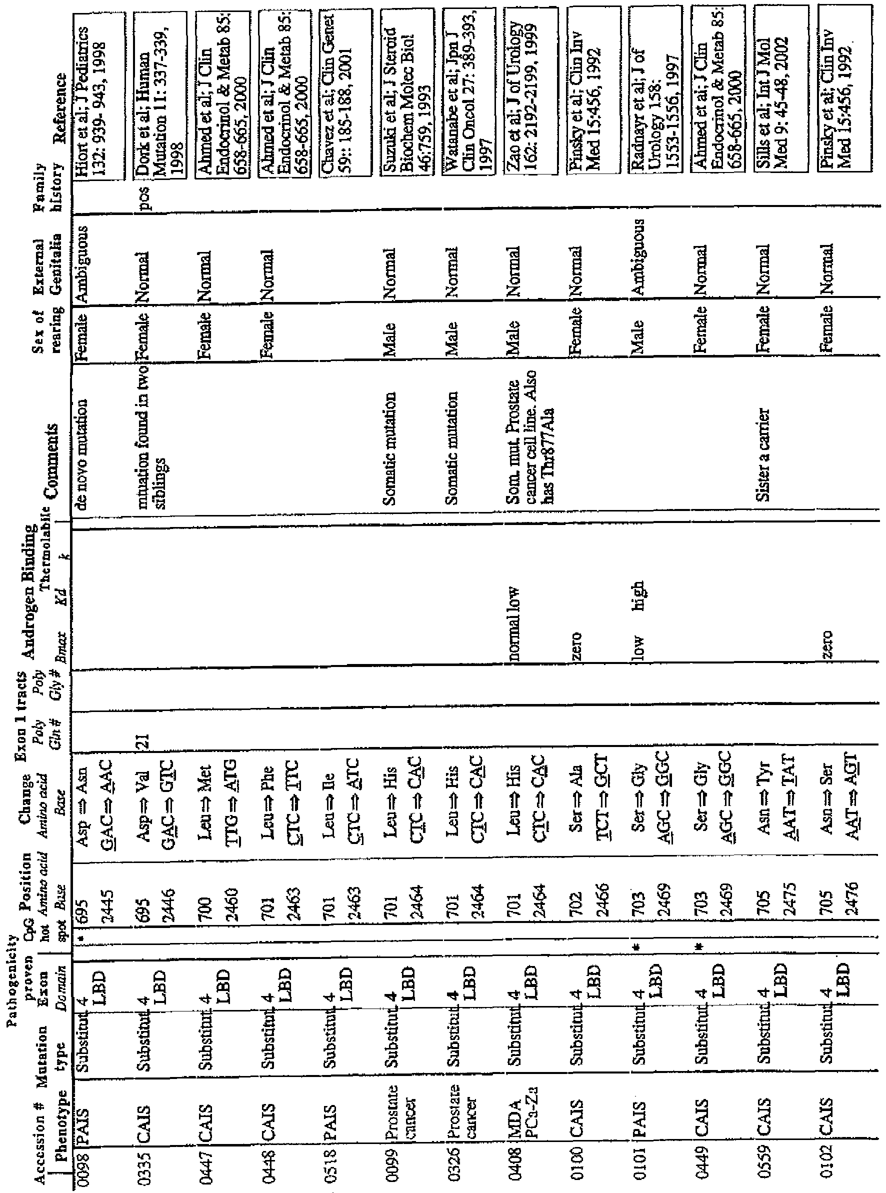

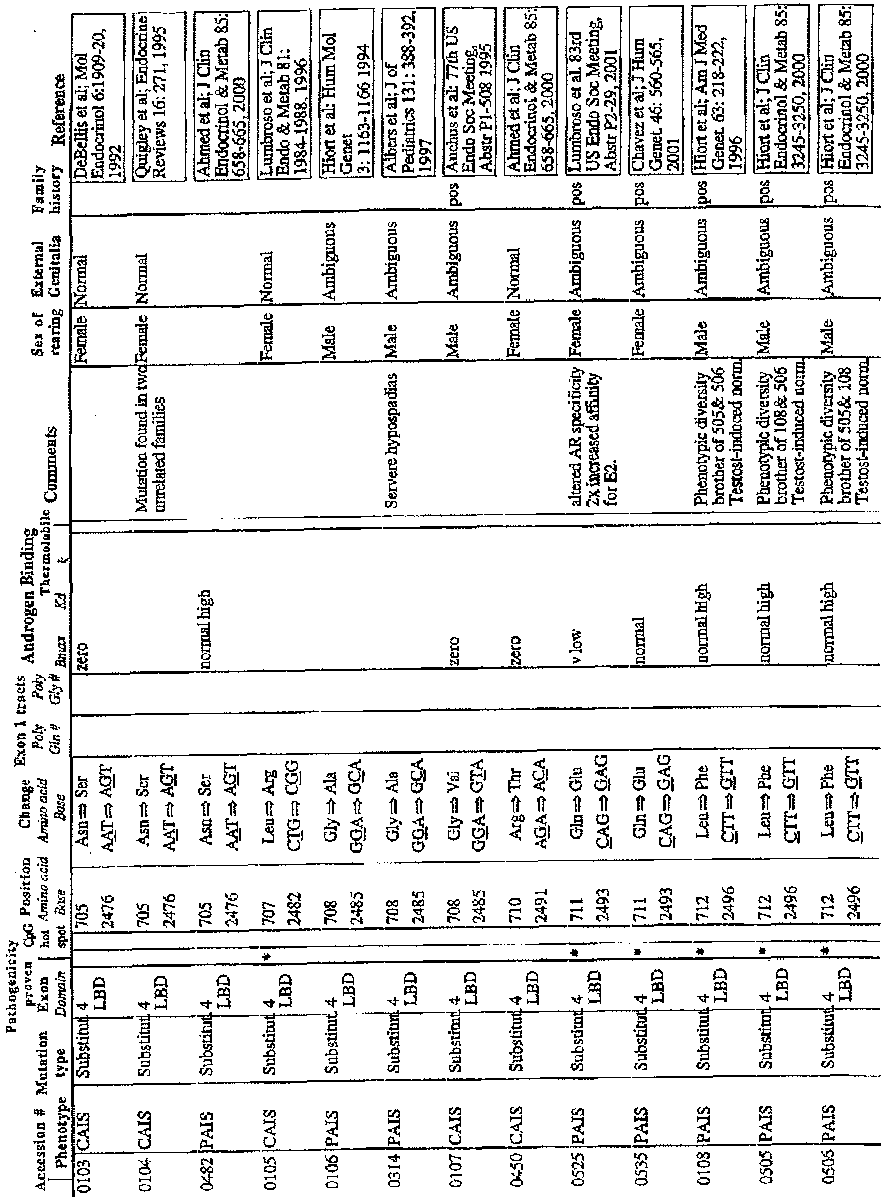

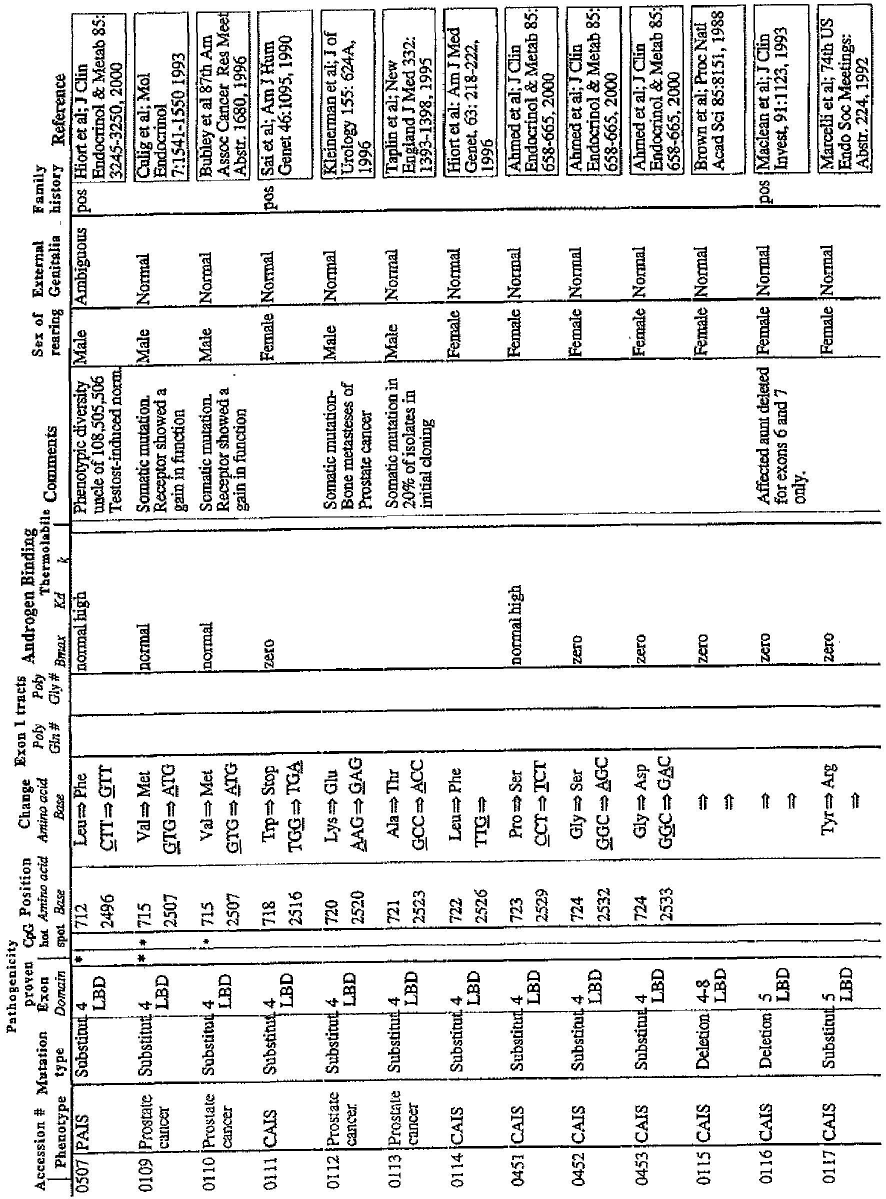

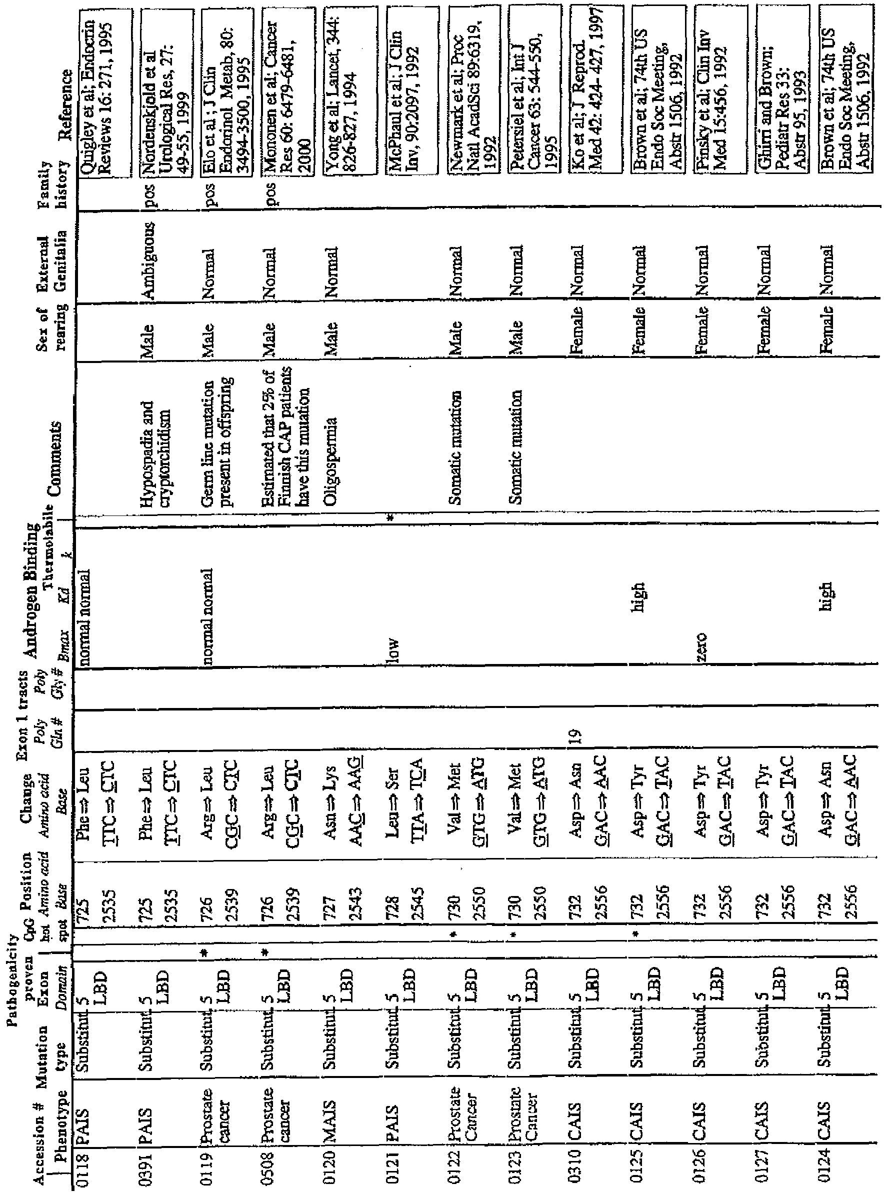

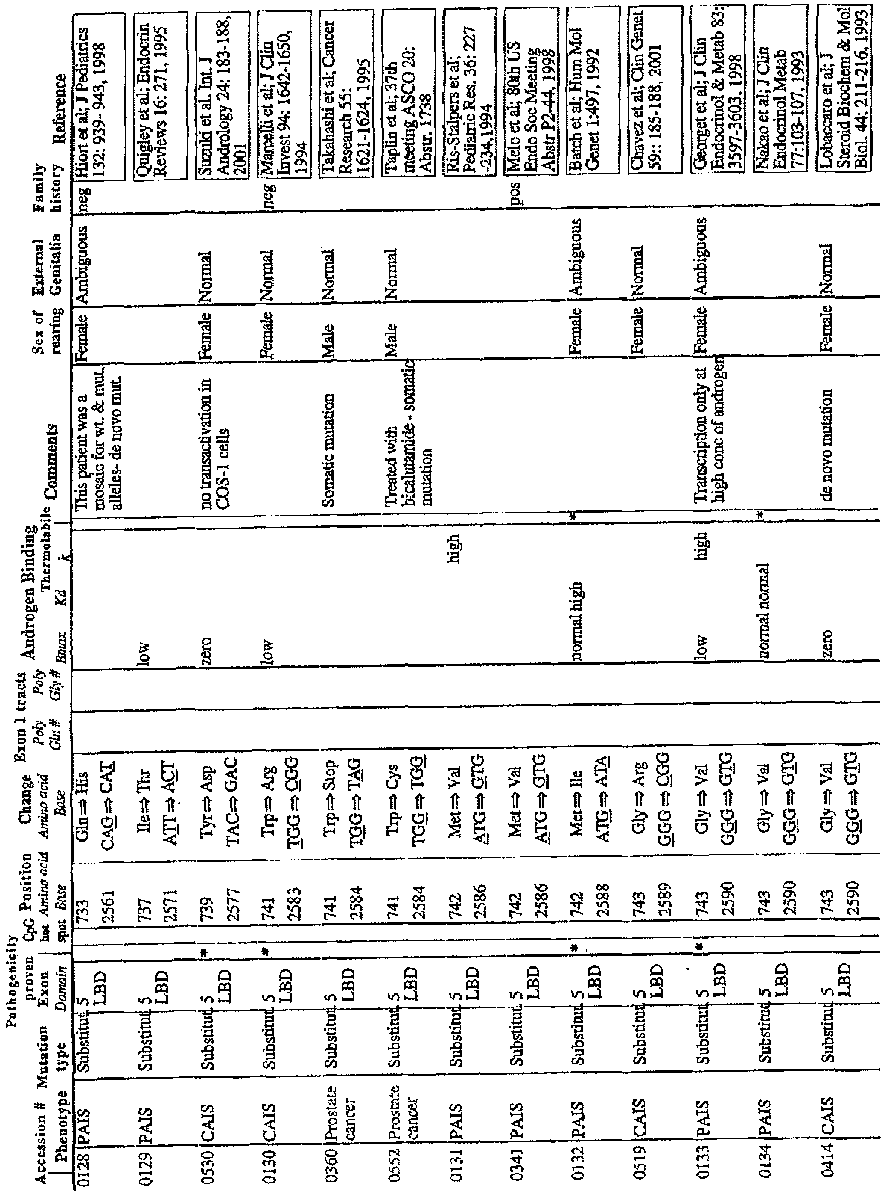

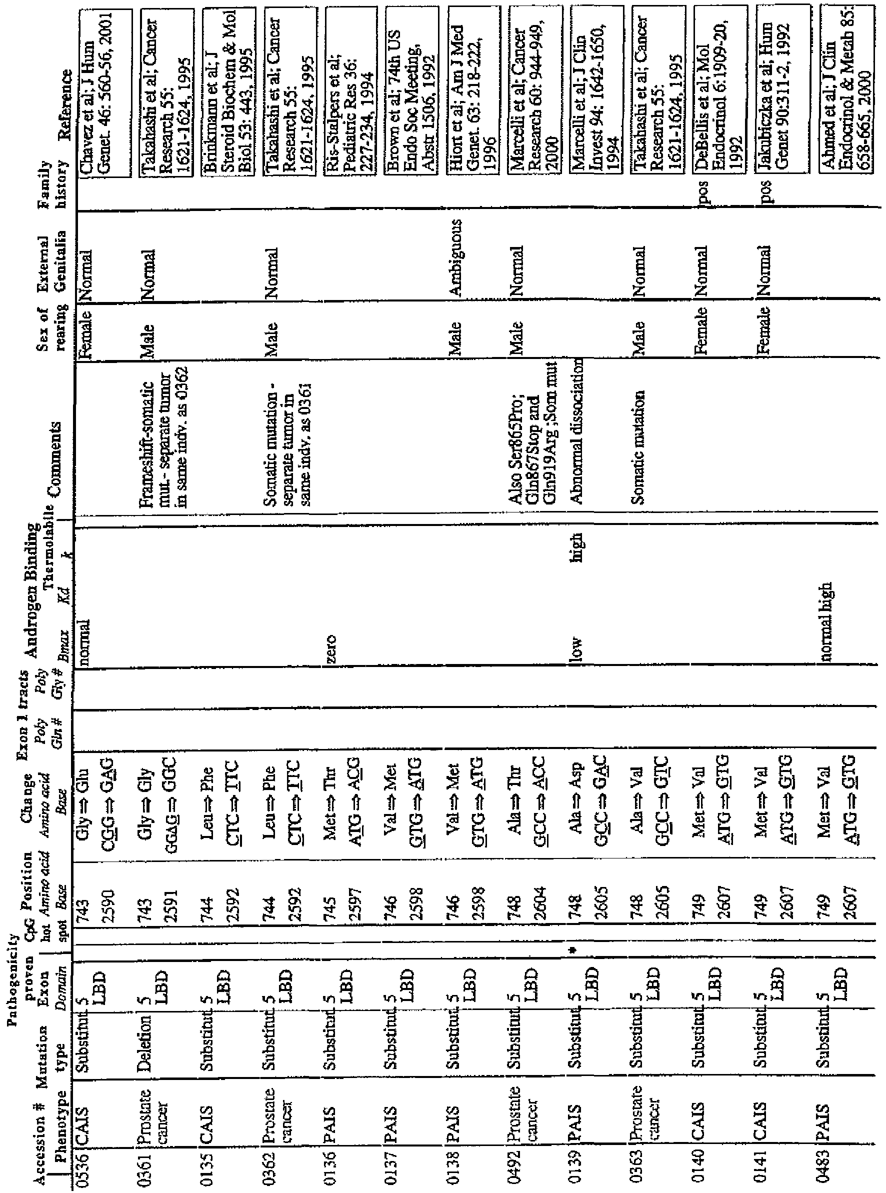

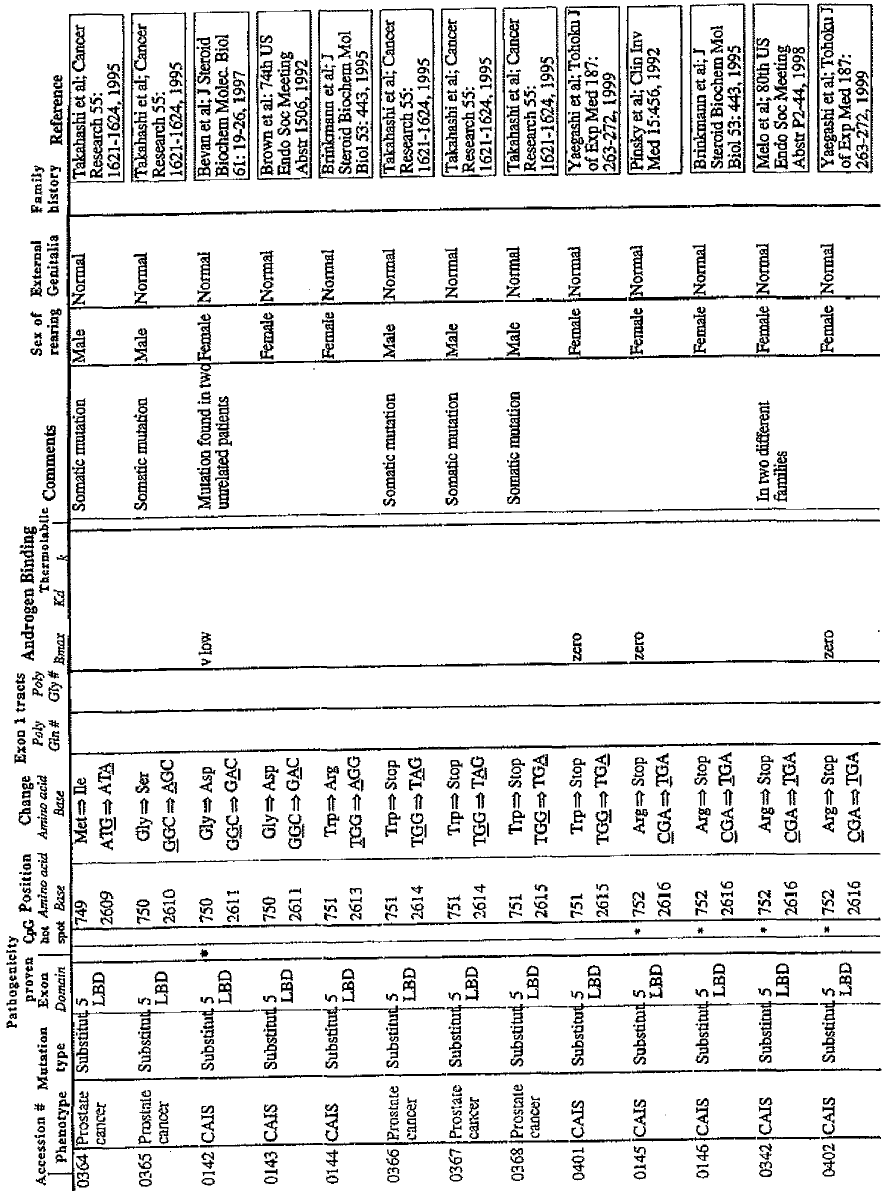

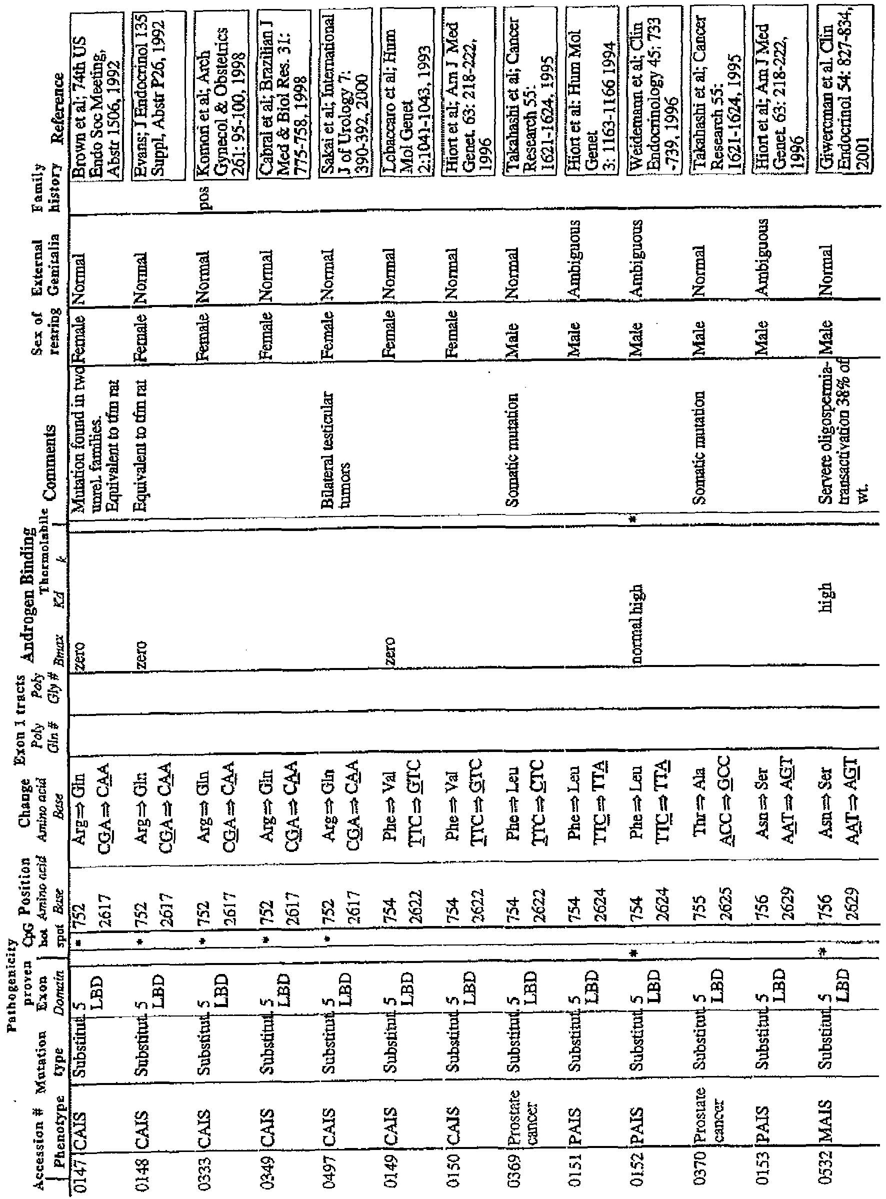

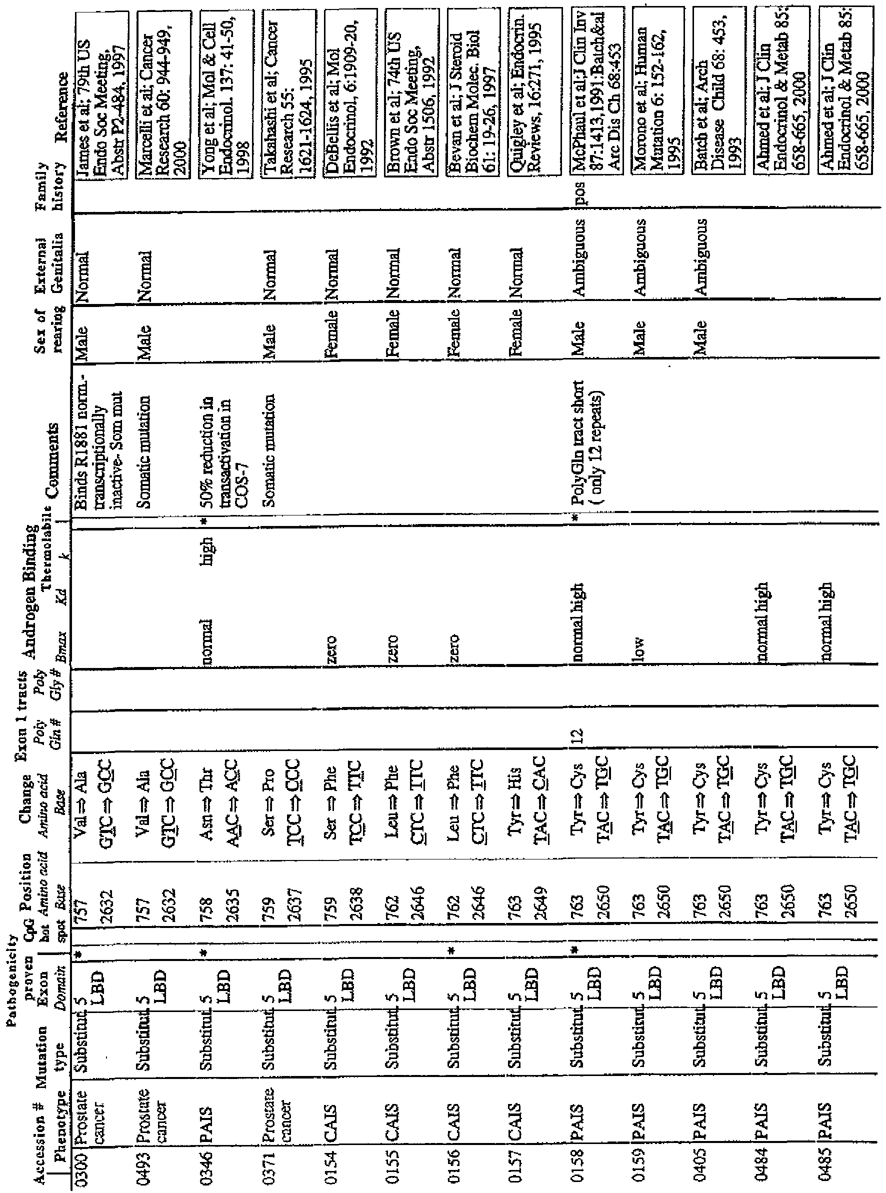

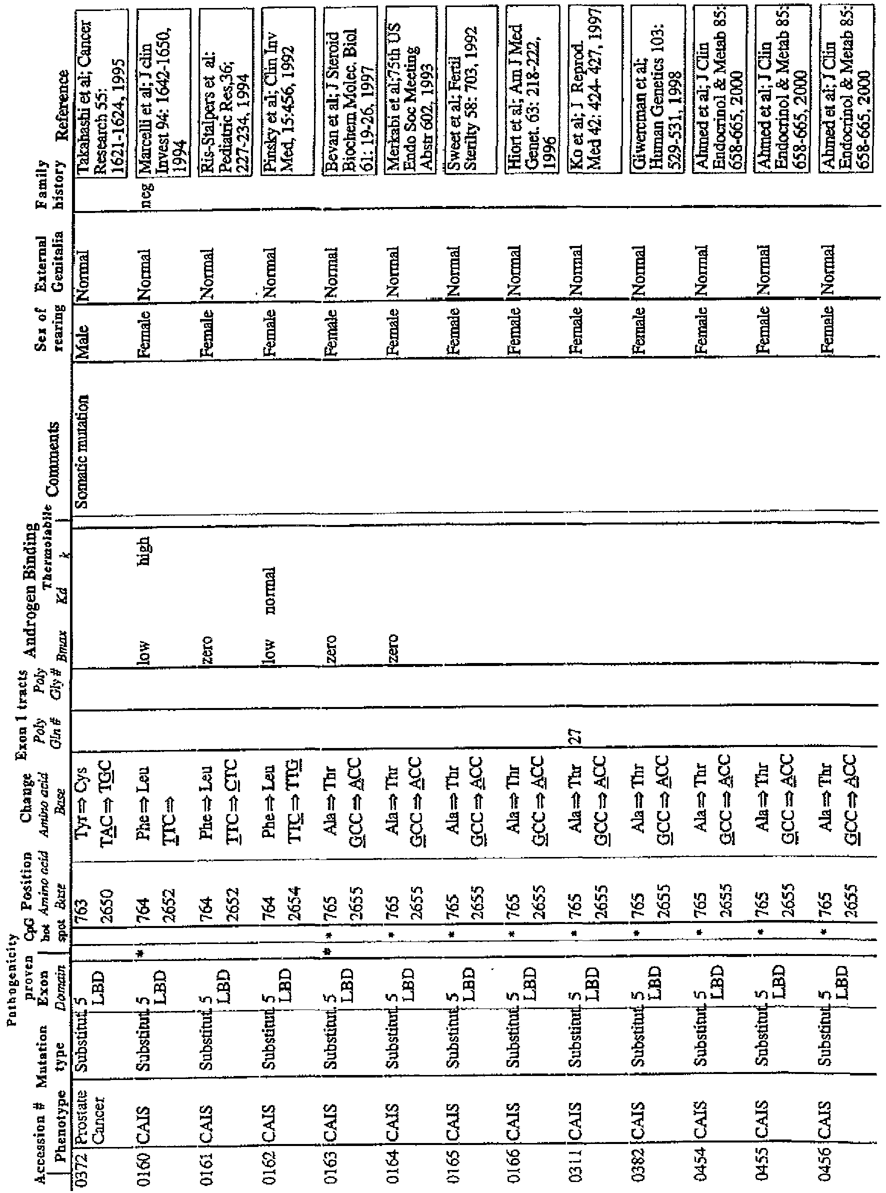

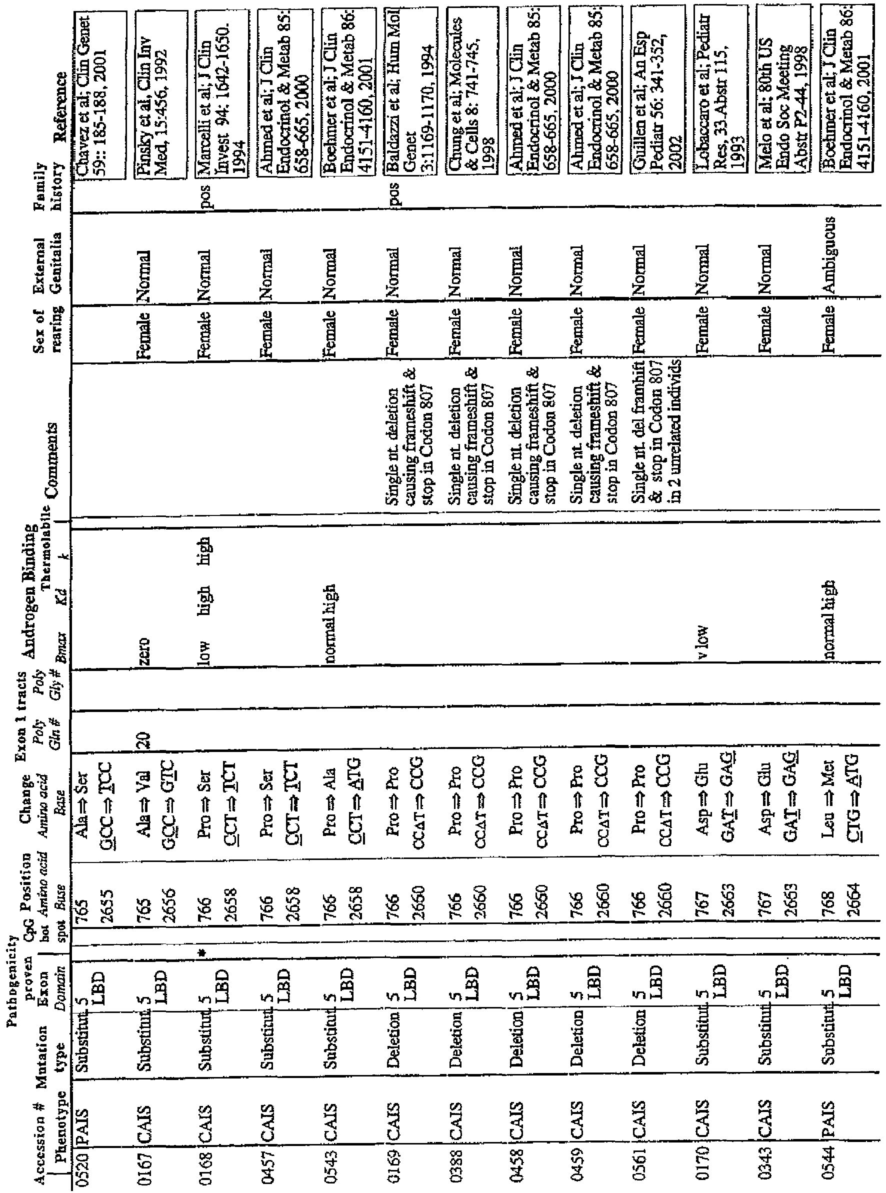

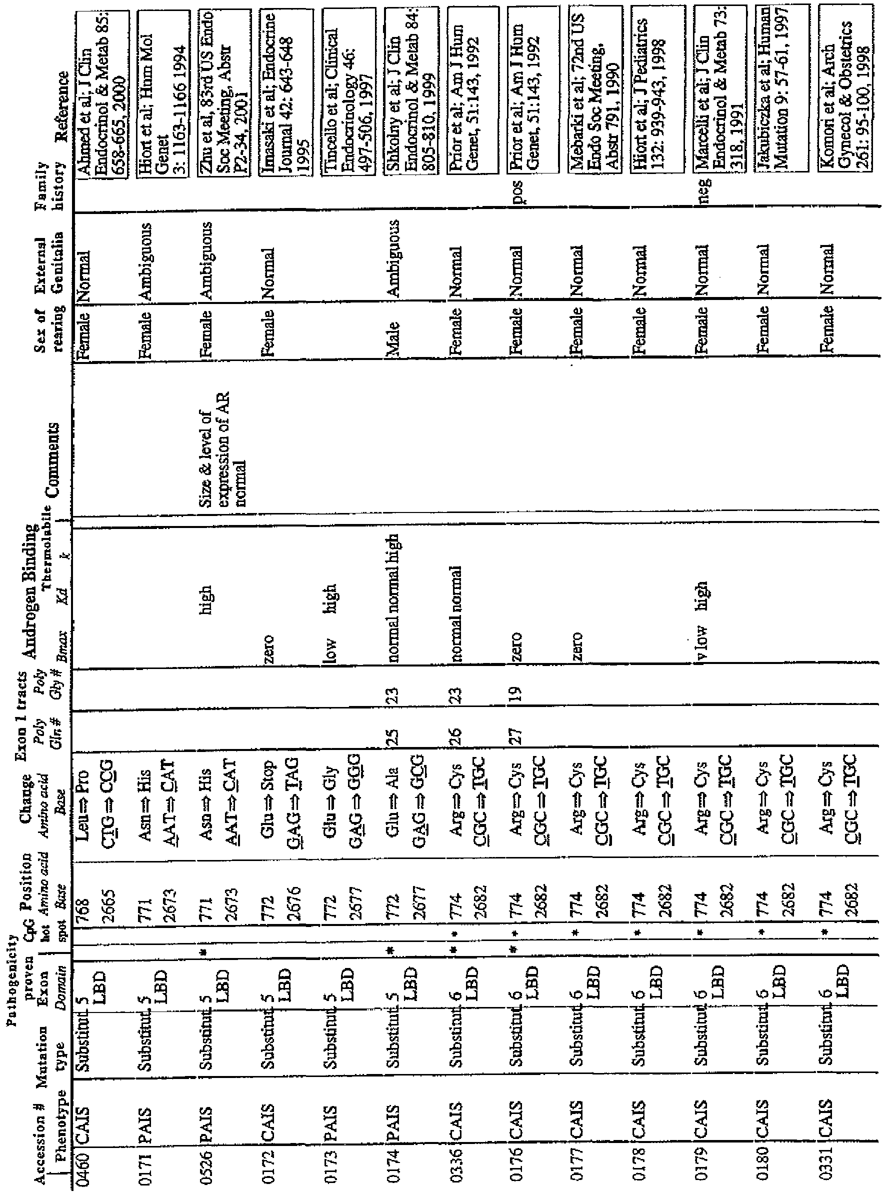

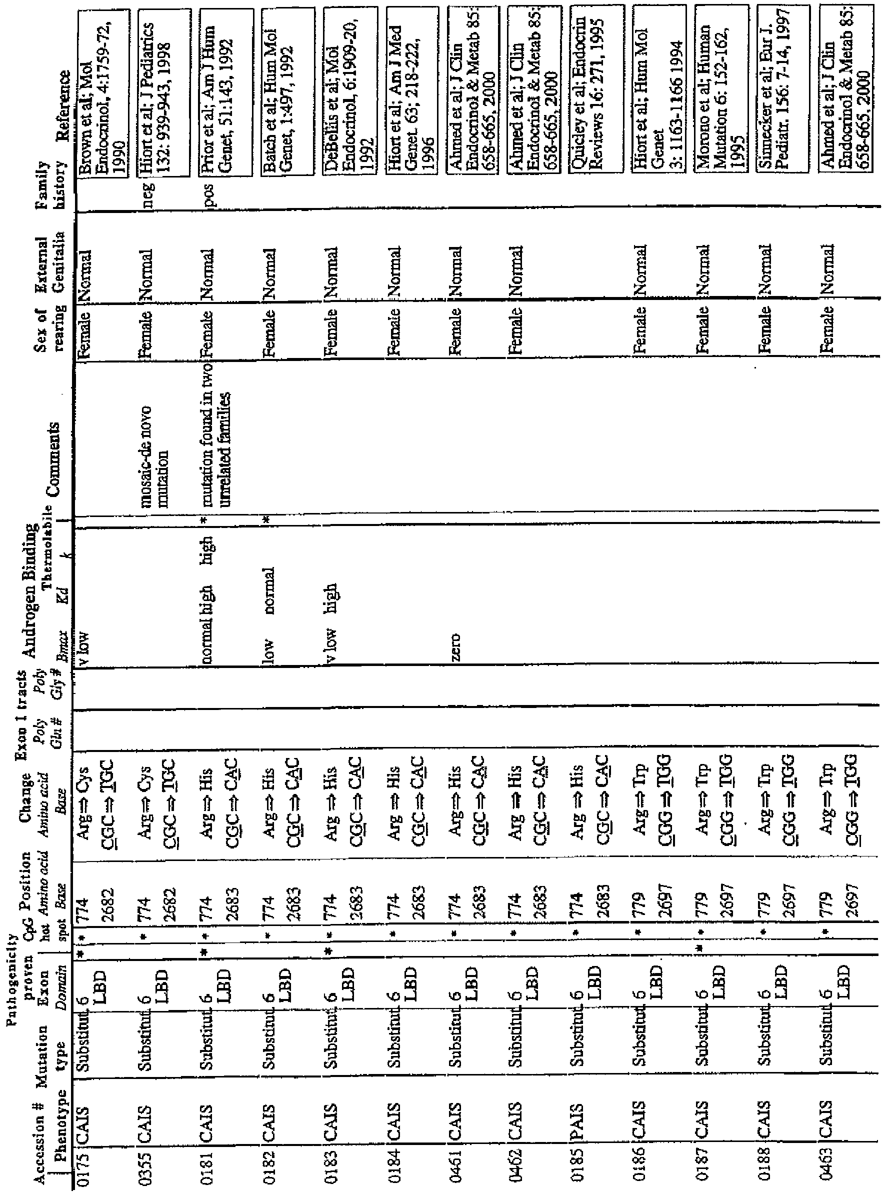

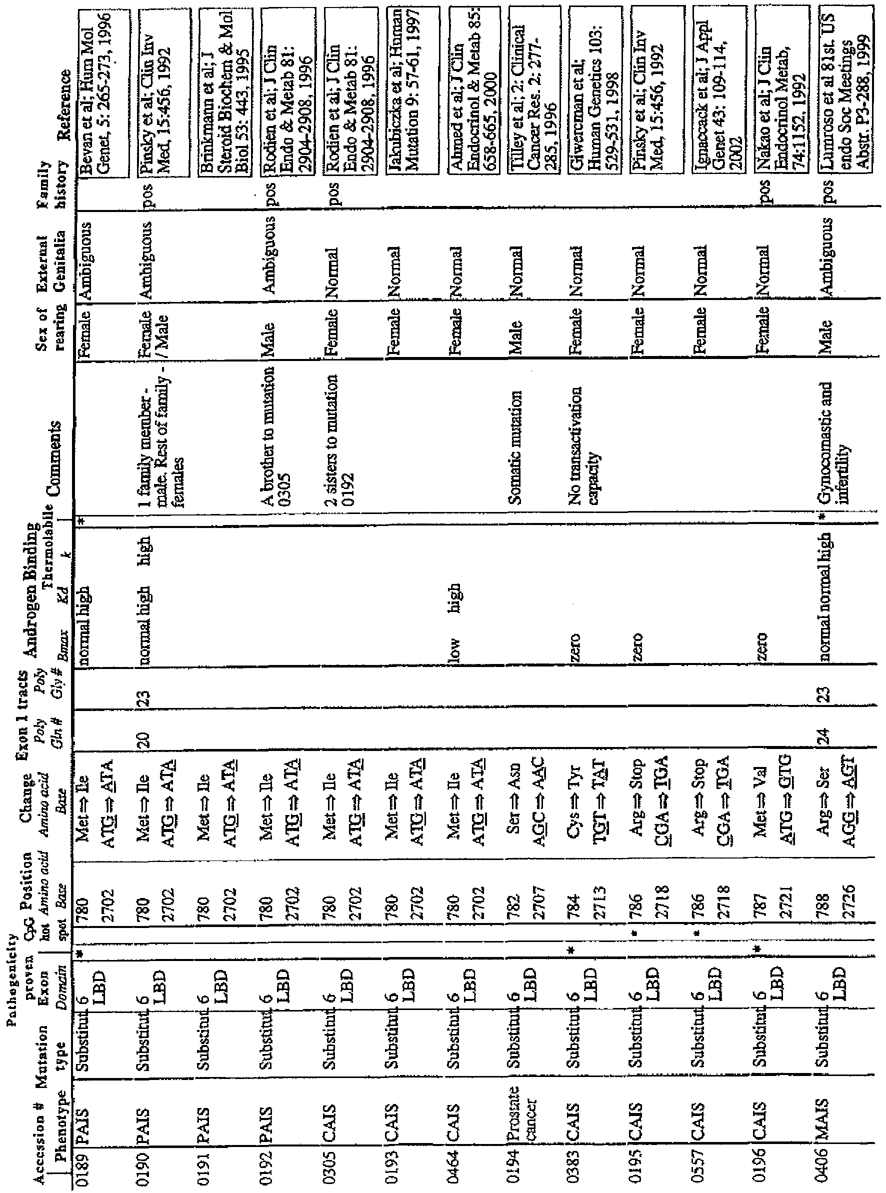

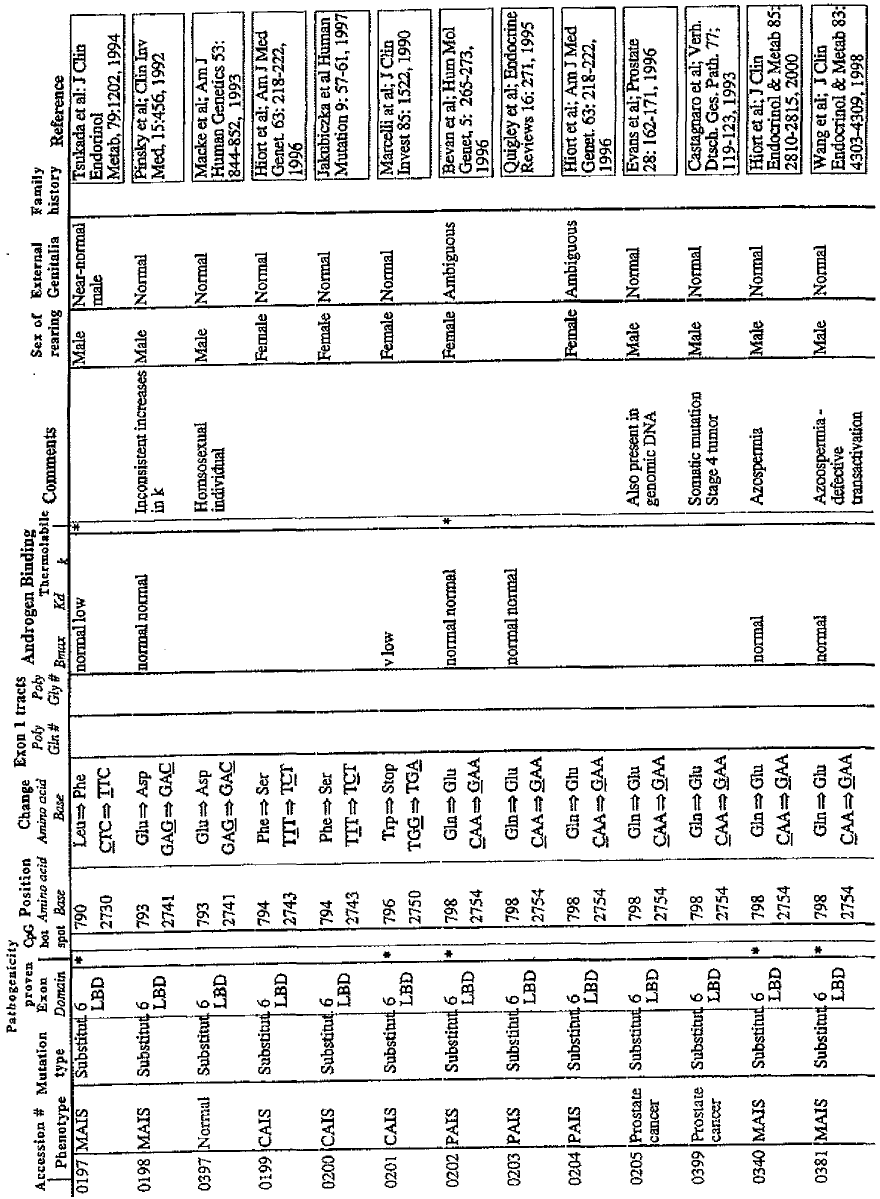

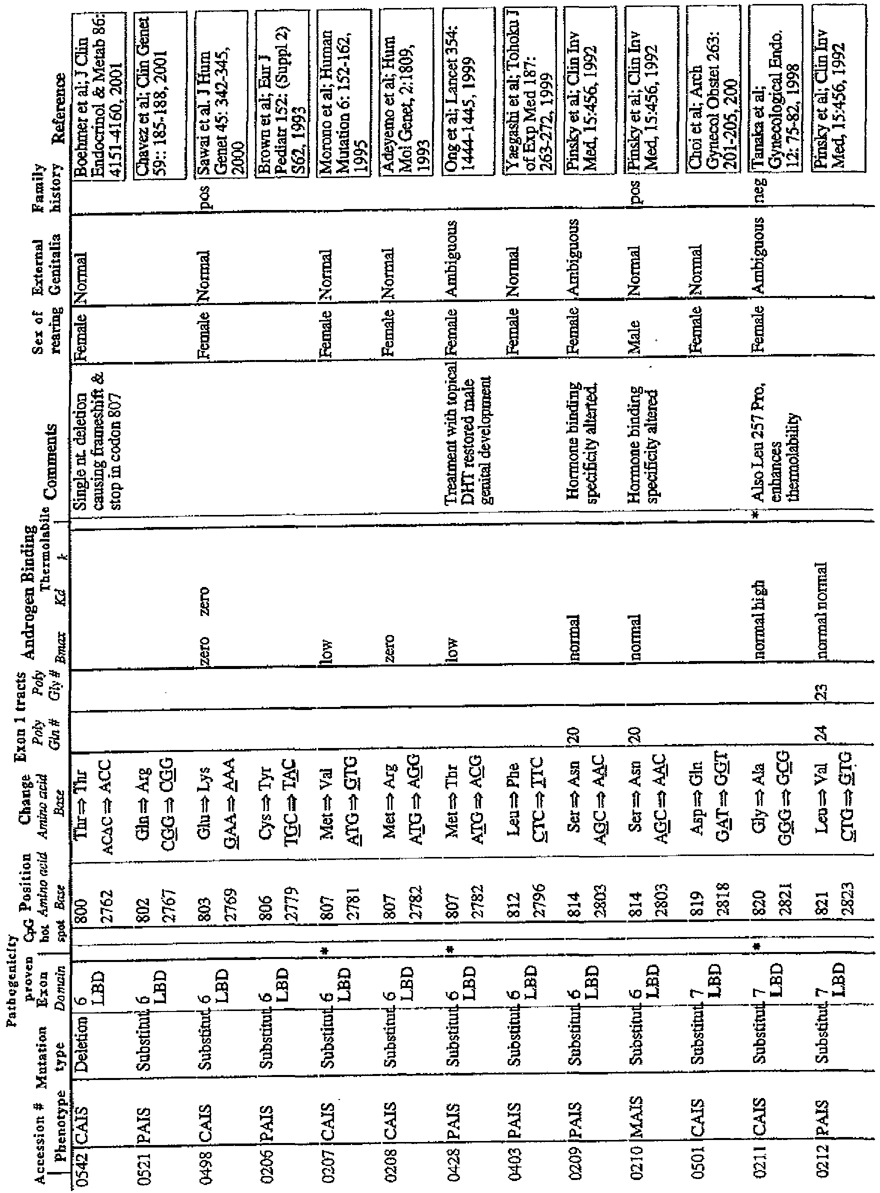

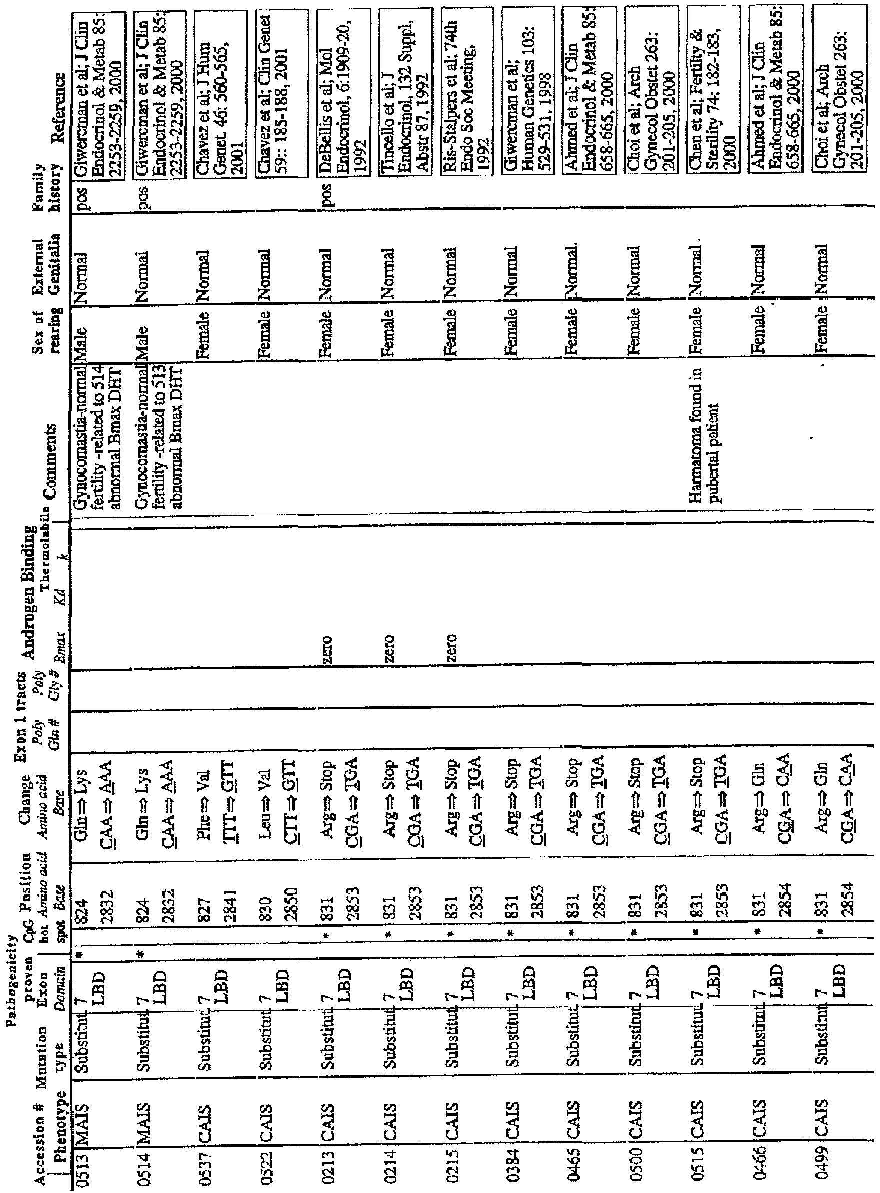

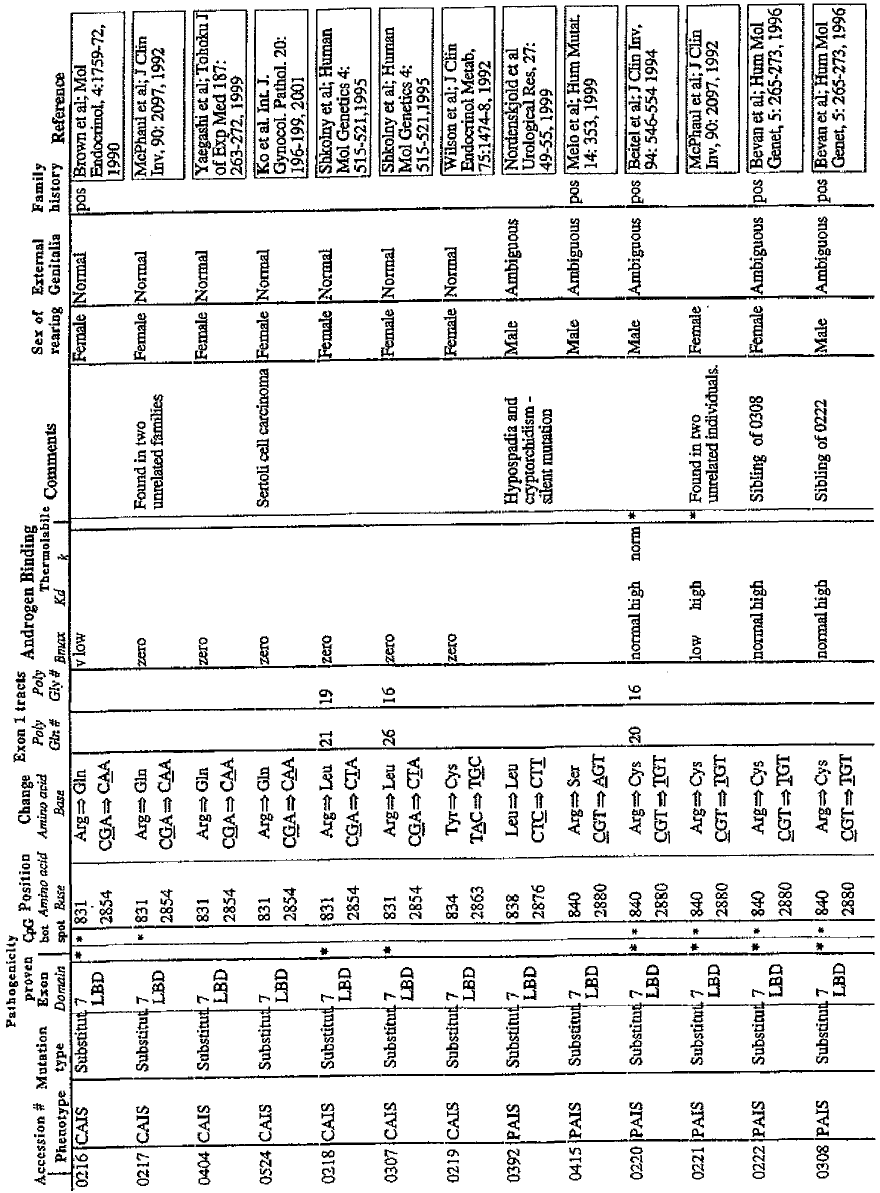

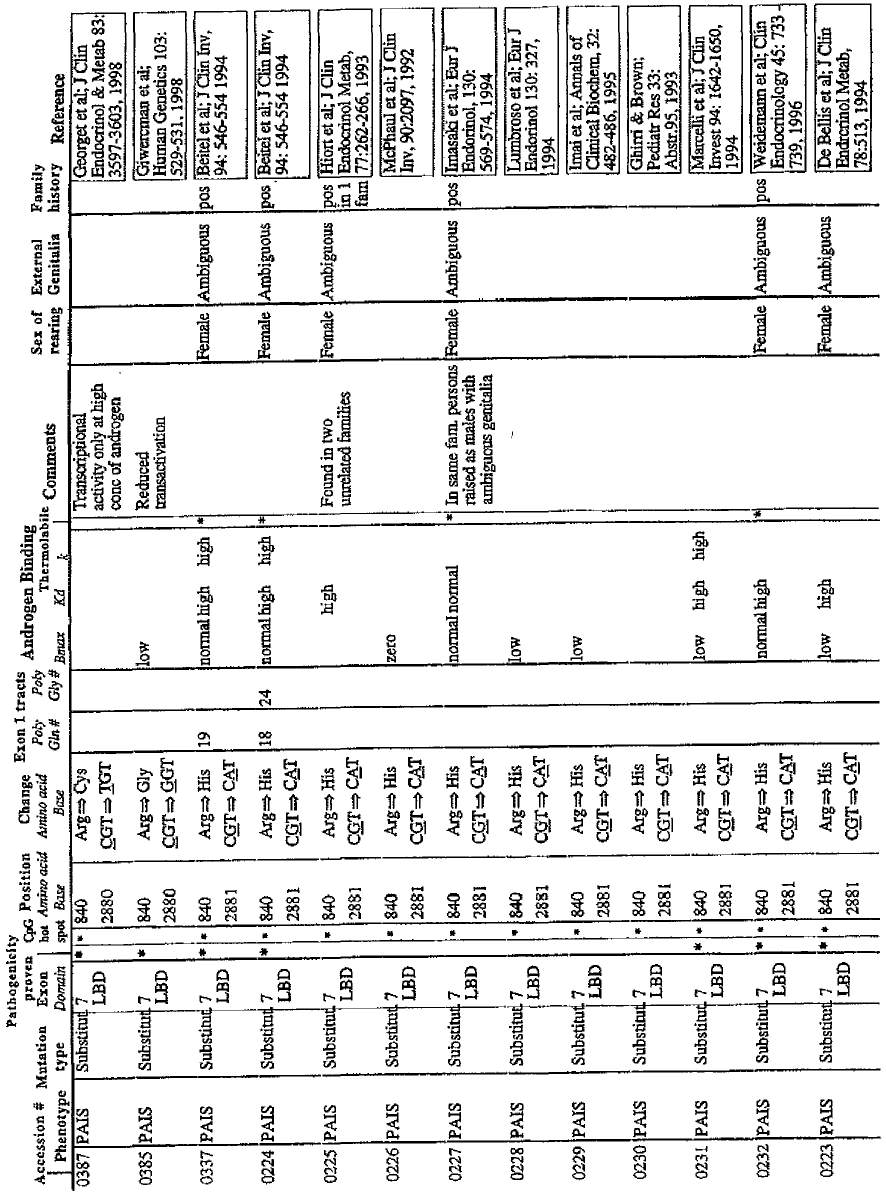

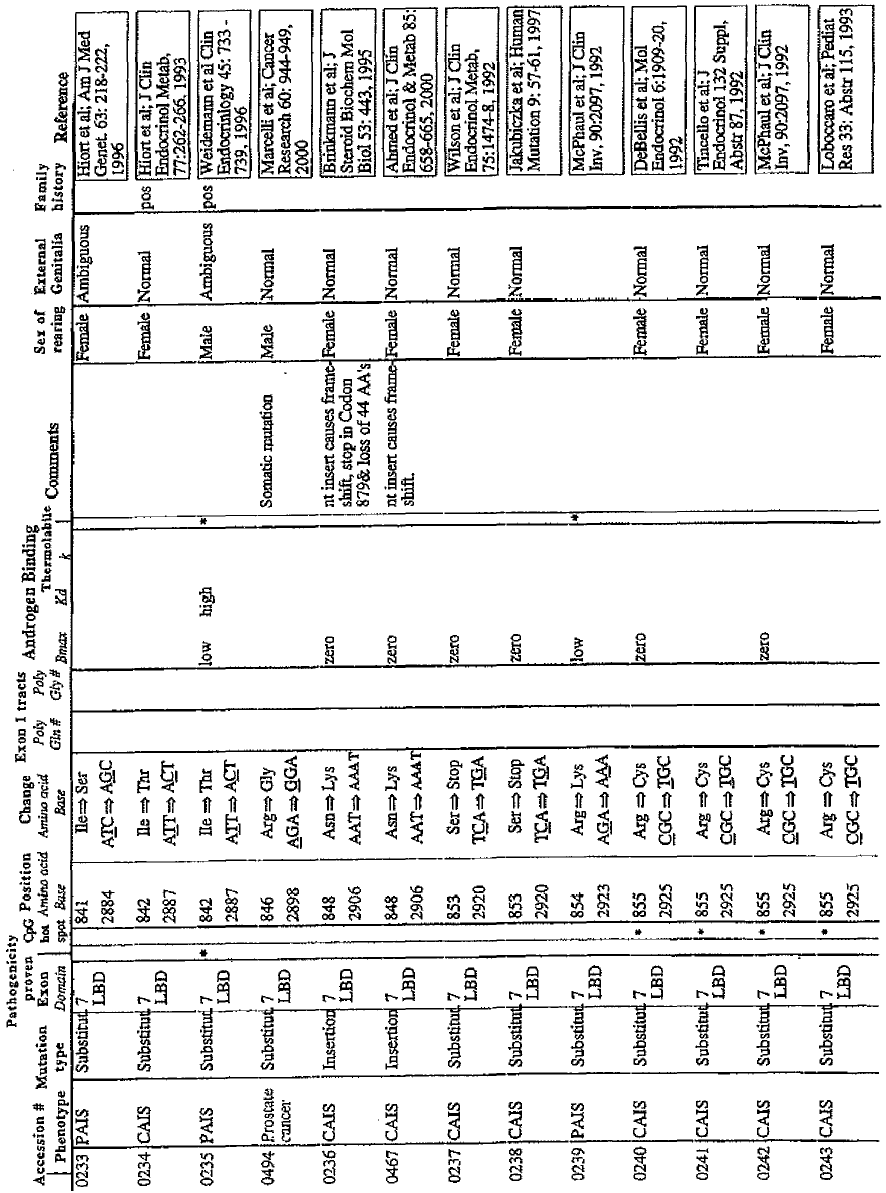

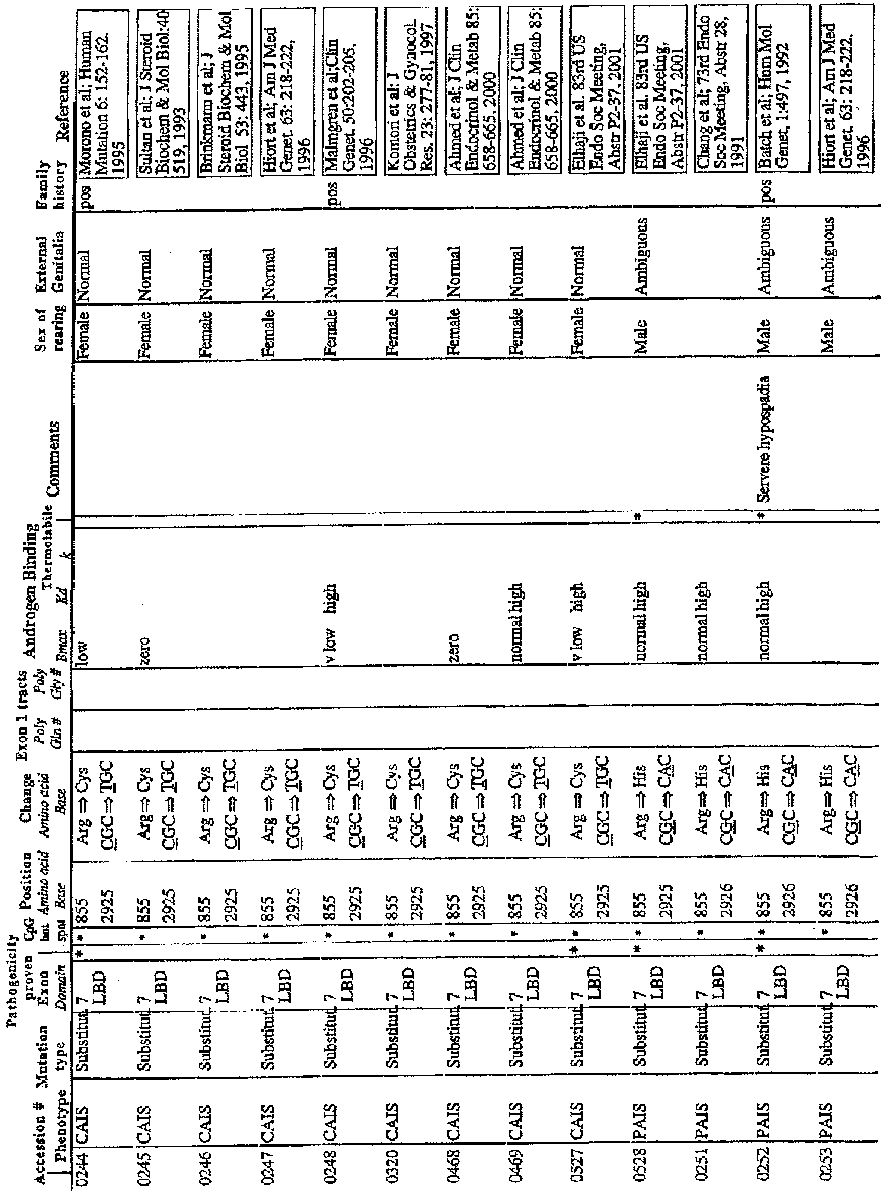

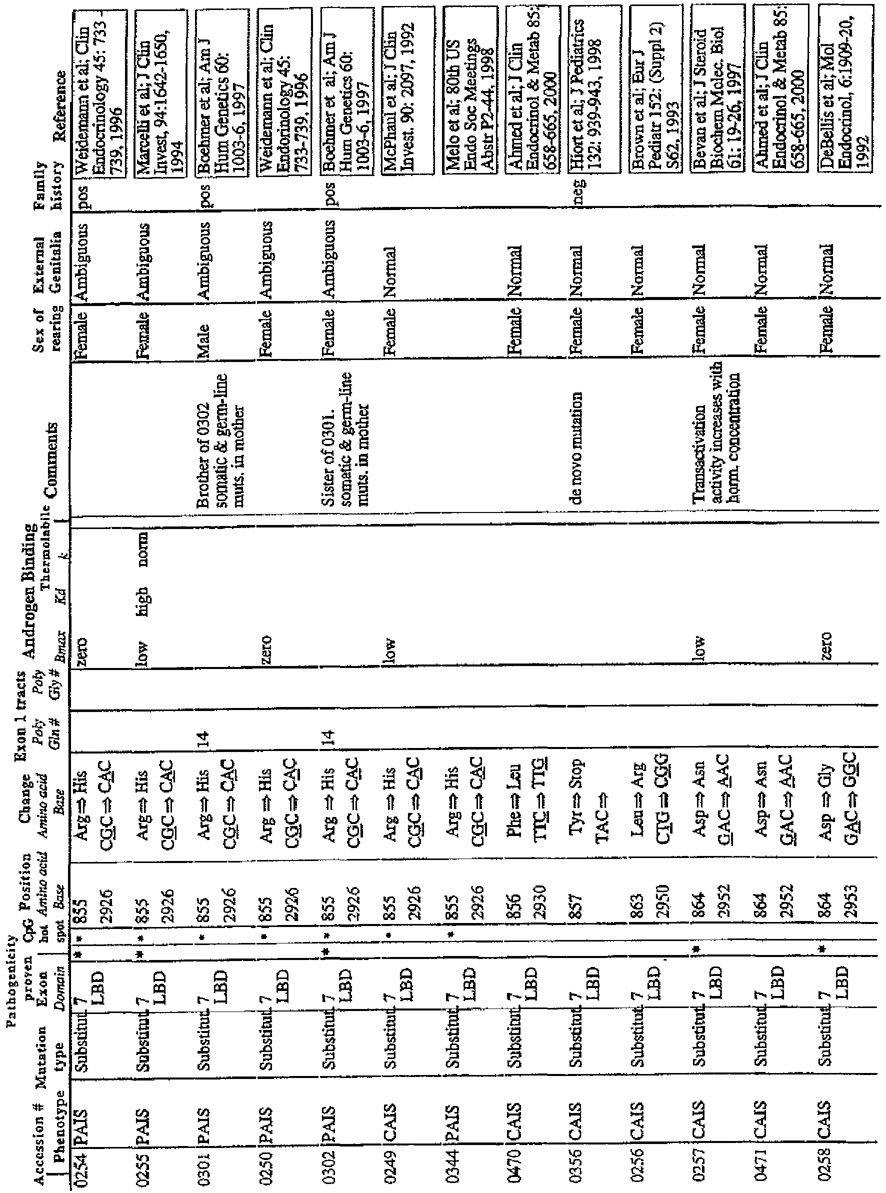

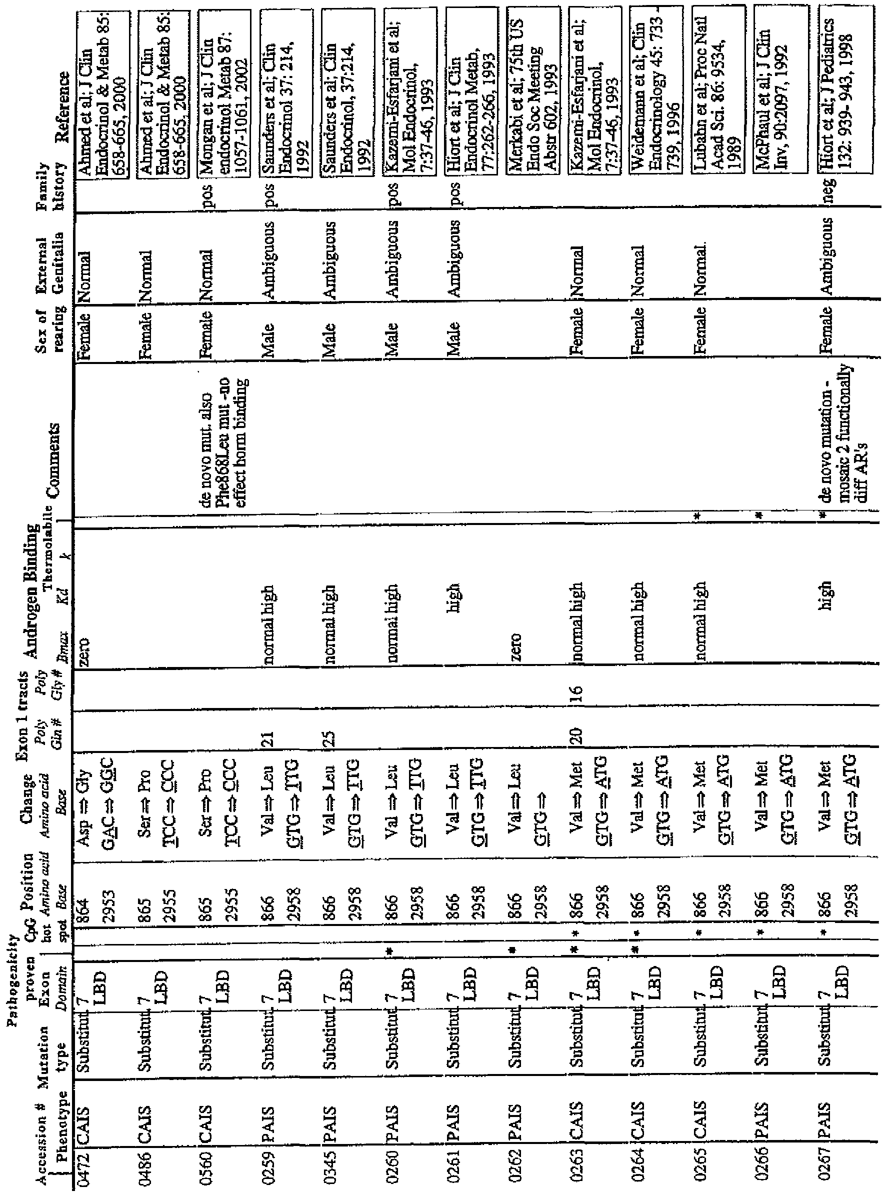

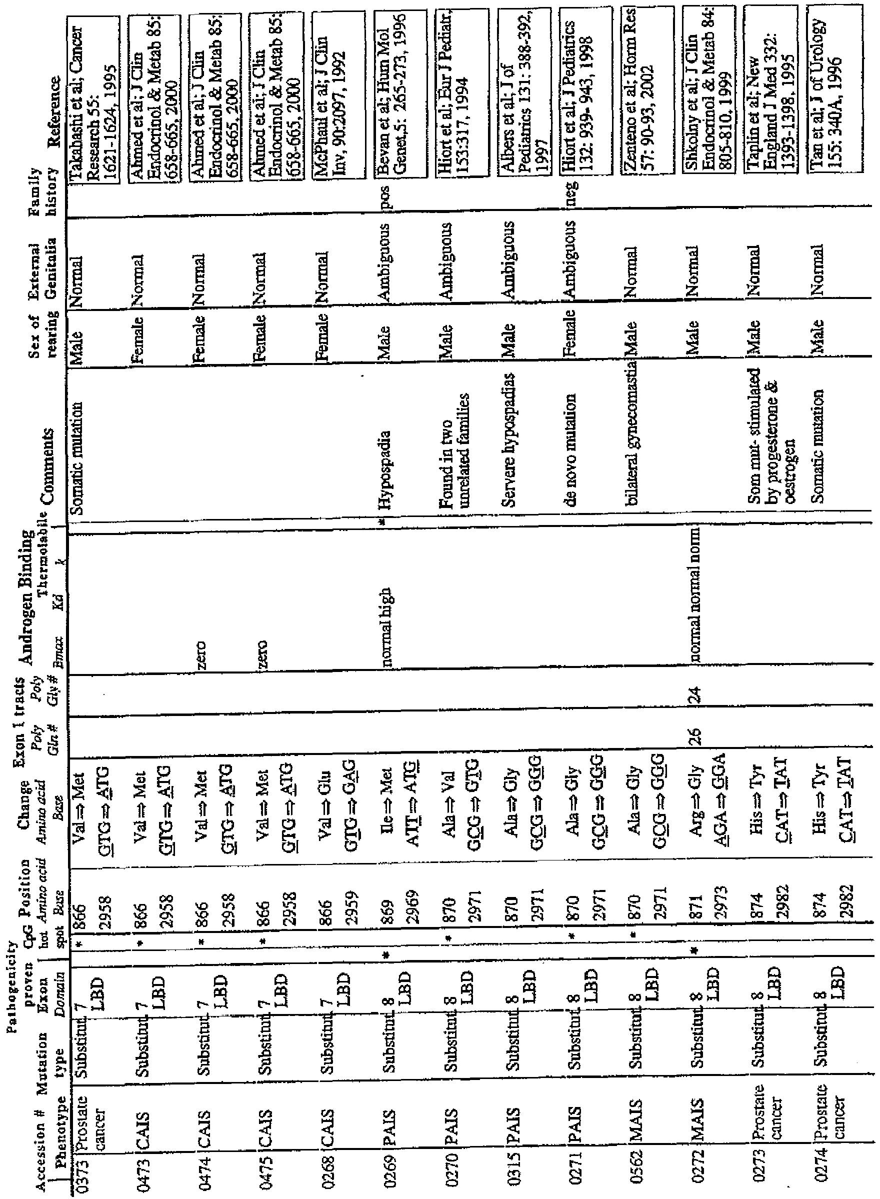

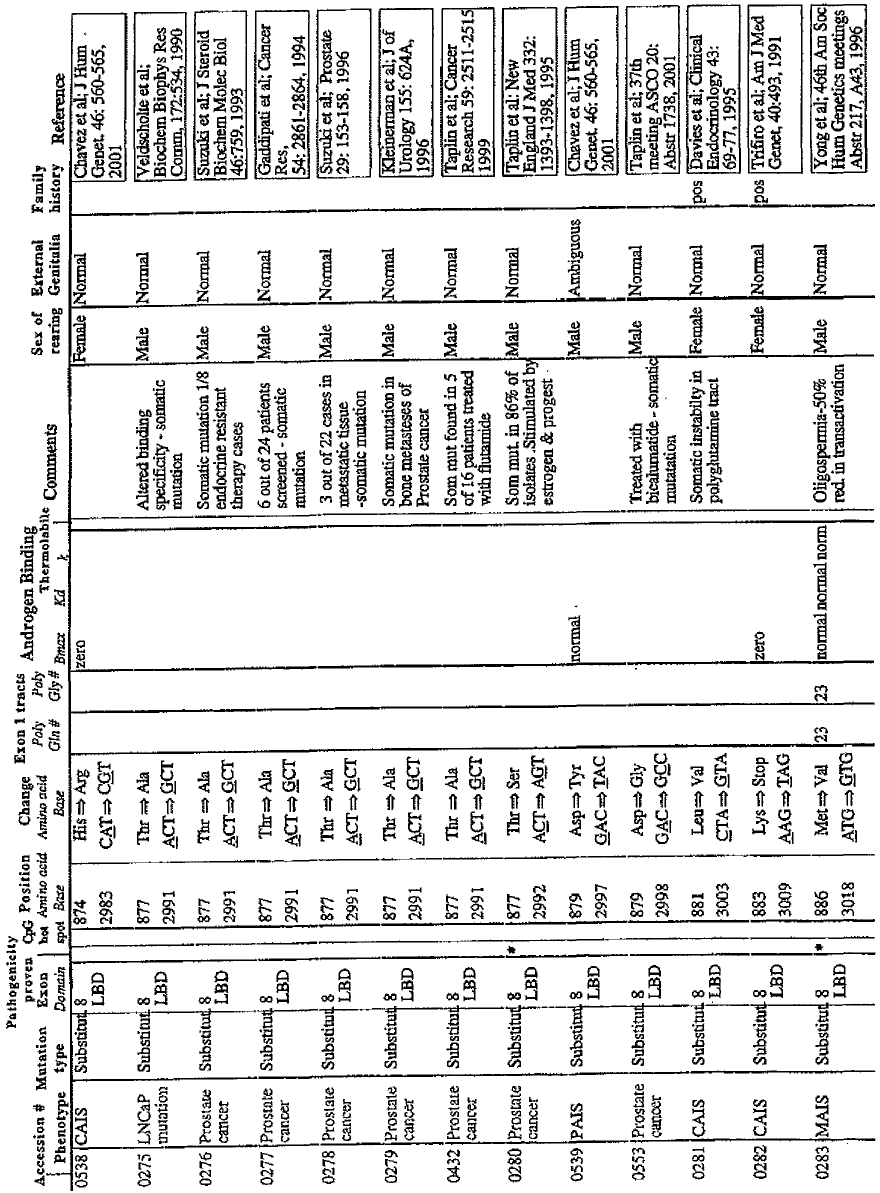

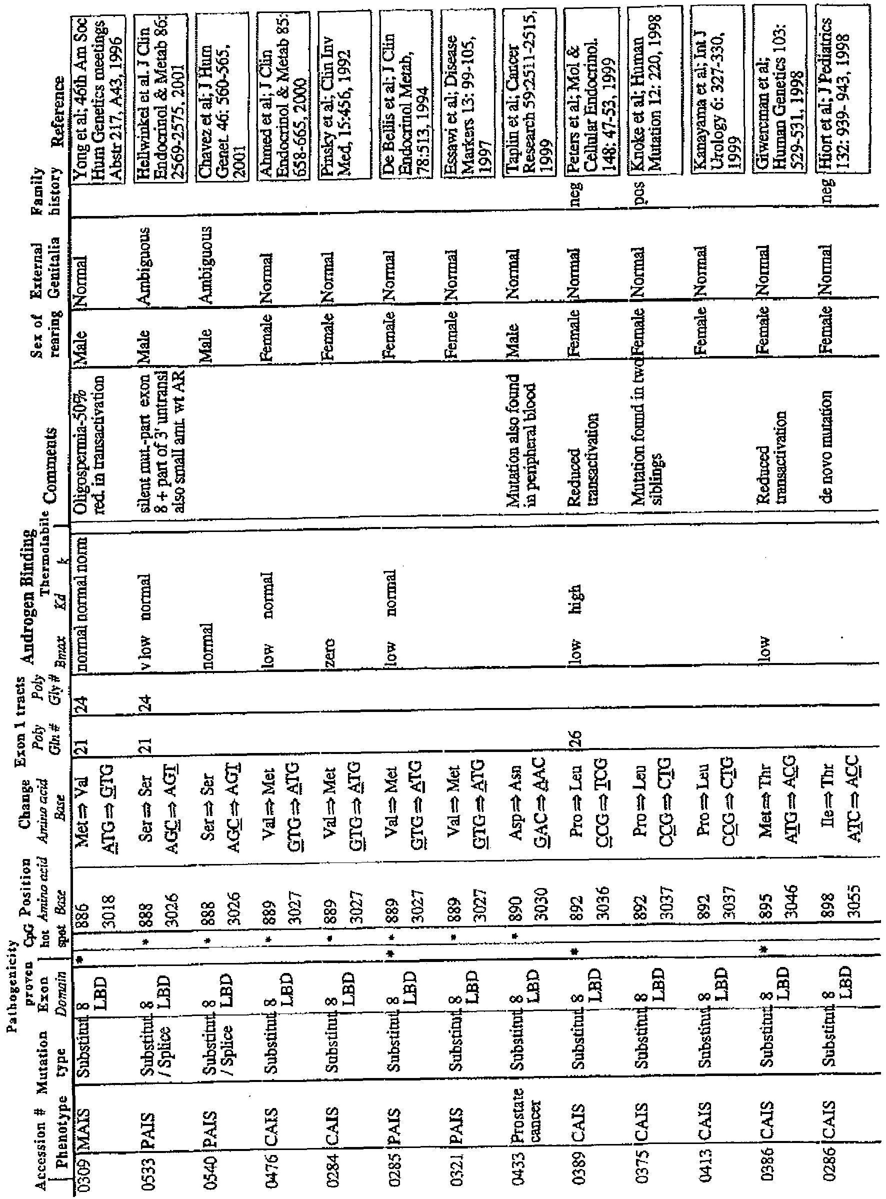

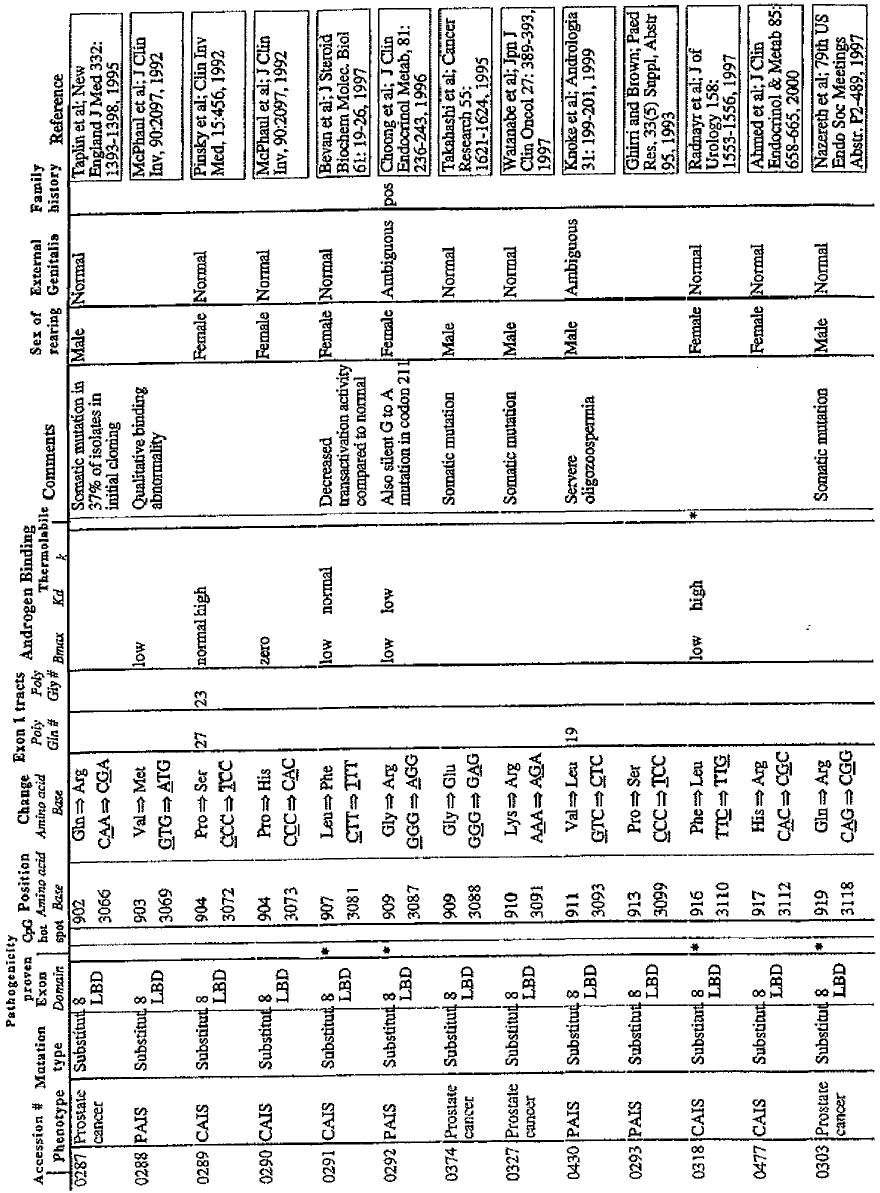

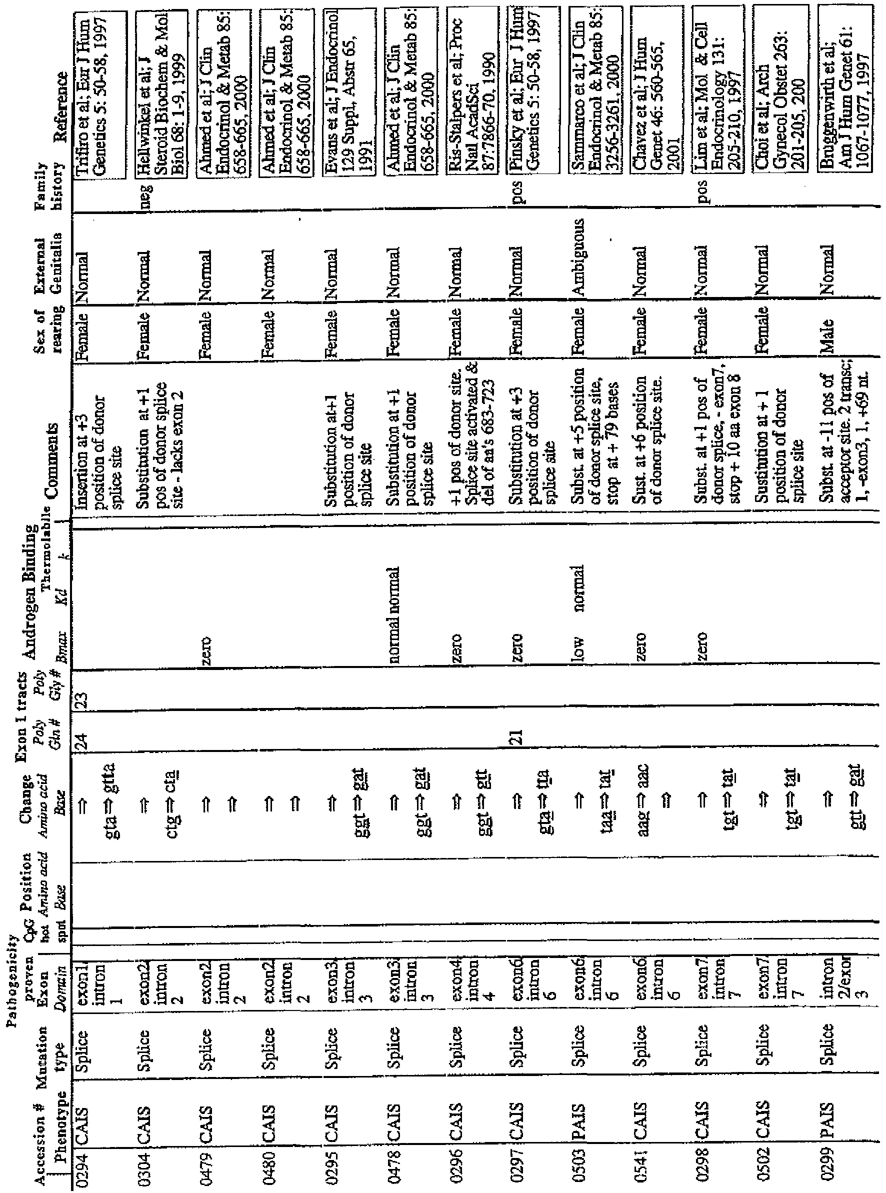

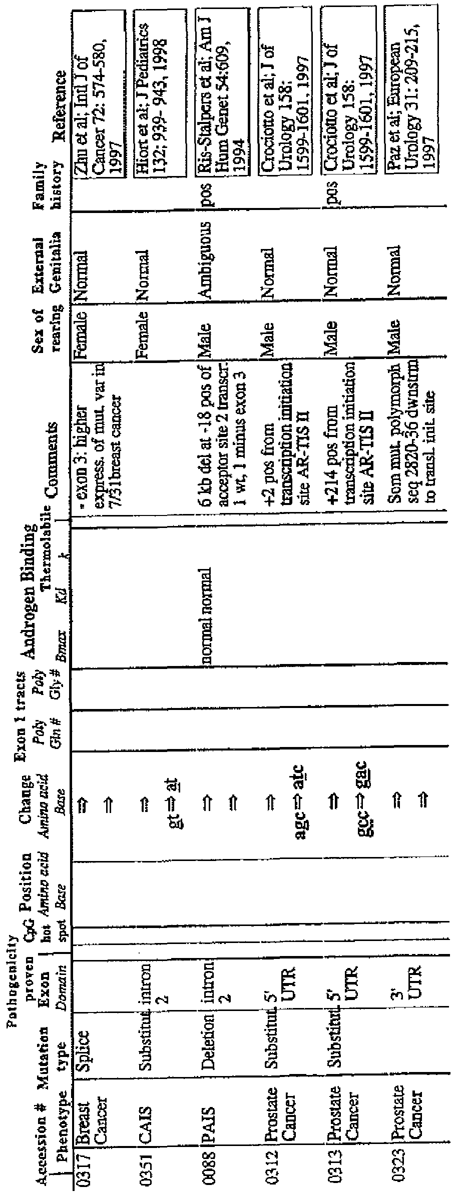

## Revendications

1. Un oligonucléotide double brin **caractérisé en ce qu'**il est constitué de deux séquences oligonucléotidiques complémentaires formant un hybride comprenant chacune à l'une de leurs extrémités 3' ou 5 ' un à cinq nucléotides non appariés formant des bouts simples brins débordant de l'hybride, l'une desdites séquences oligonucléotidiques étant substantiellement complémentaire d'une séquence cible appartenant à une molécule d'ARN messager du gène codant le récepteur aux androgènes muté ou non muté.

2. Un oligonucléotide selon la revendication 1, **caractérisé en ce que** le gène codant le récepteur aux androgènes non-muté est représenté par la SEQ ID NO : 77.

3. Un oligonucléotide selon la revendication 1, **caractérisé en ce que** le récepteur aux androgènes muté porte l'une au moins des mutations du tableau 5 de l'annexe C.

4. Un oligonucléotide selon la revendication 1, **caractérisé en ce qu'**il est représenté par les séquences SEQ ID NO : 19 et SEQ ID NO : 20.

5. Composition notamment pharmaceutique pour être utilisée dans la recherche de la fonction de gène ou à des fins thérapeutiques ou diagnostiques, **caractérisée en ce qu'**elle comprend à titre d'agent actif au moins un oligonucléotide selon l'une quelconque des revendications 1 à 4, et un excipient pharmaceutiquement acceptable.

6. Utilisation d'un oligonucléotide selon l'une quelconque des revendications 1 à 4 pour la préparation d'une composition pharmaceutique utile pour la prévention ou le traitement des carcinomes.

7. Utilisation d'un oligonucléotide selon l'une quelconque des revendications 1 à 4 pour la préparation d'une composition pharmaceutique utile pour la prévention ou le traitement d'une maladie androgéno-dépendante.

8. Utilisation d'un oligonucléotide selon la revendication 7, **caractérisée en ce que** ladite maladie androgéno-dépendante est le cancer de la prostate.
